# EUROPEAN PATENT SPECIFICATION

(11) **EP 0 573 603 B1**
(45) Date of publication and mention of the grant of the patent: **25.06.2003**
(21) Application number: 92908481.2
(22) Date of filing: 28.02.1992
(51) Int. Cl.: C07K 14/47

(54) **INHIBITORS OF HUMAN NEUTROPHIL ELASTASE AND HUMAN CATHEPSIN G**
HEMMSTOFFE FÜR MENSCHLICHE NEUTROPHILE ELASTASE UND MENSCHLICHES KATHEPSIN G
INHIBITEURS DE L'ELASTASE NEUTROPHILE ET DE LA CATHEPSINE G CHEZ L'HOMME

(30) Priority: 01.03.1991 US 664989; 17.06.1991 US 715834
(43) Date of publication of application: 15.12.1993
(60) Divisional application: 03000399.0
(73) Proprietor: Dyax Corp., Cambridge, MA 02139 (US)
(72) Inventor: LEY, Arthur, Charles, Newton, MA 02165 (US); LADNER, Robert, Charles, Ijamsville, MD 21754 (US); GUTERMAN, Sonia, Kosow, Belmont, MA 02178 (US); ROBERTS, Bruce, Lindsay, Milford, MA 01757 (US); MARKLAND, William, Milford, MA 01757 (US); KENT, Rachel, Baribault, Boxborough, MA 01719 (US)
(74) Representative: Wichmann, Hendrik, Dr.
(86) International application number: US9201501
(87) International publication number: WO92015605

(56) References cited:
- EP-A- 0 195 212
- EP-A- 0 307 592
- EP-A- 0 401 508
- WO-A-90/02809
- BIOL. CHEM. HOPPE-SEYLER, VOL. 371, SUPPL. May 1990, BERLIN, FRG pages 43 - 52 T. BRINKMANN & H. TSCHESCHE 'Design of an Aprotinin Variant with Inhibitory Activity against Chymotrypsin and Cathepsin G by Recombinant DNA Technology' cited in the application

## Description

### Field of the invention

The present invention relates to novel protease inhibitors and, in particular to small engineered proteins that inhibit human neutrophil elastase (hNE) and to proteins that inhibit human cathepsin G (hCG).

### Description of the Background Art

*Neutrophil Elastase and Cathepsin G.* The active sites of serine proteases are highly similar. Trypsin, chymotrypsin, neutrophil elastase, cathepsin G and many other proteases share strong sequence homology. The so-called catalytic triad comprises (in standard chymotrypsinogen numbering) aspartic acid-102, histidine-57, and serine-195. Residues close to the catalytic triad determine the substrate specificity of the particular enzyme (Cf. CREI84, p366-7). The structure and function of the digestive enzymes trypsin, pancreatic elastase, and chymotrypsin has been more throughly studied than have the neutrophil enzymes. X-ray structures of hNE complexed with a substrate have been solved and the similarity of the active site of hNE to that of trypsin is very high. The specificity of hNE is higher than trypsin and lower than Factor Xₐ.

Serine proteases are ubiquitous in living organisms and play vital roles in processes such as: digestion, blood clotting, fibrinolysis, immune response, fertilization, and post-translational processing of peptide hormones. Although the roles these enzymes play is vital, uncontrolled or inappropriate proteolytic activity can be very damaging. Several serine proteases are directly involved in serious disease states.

Human Neutrophil Elastase (hNE, or HLE; EC 3.4.21.11) is a 29 Kd serum protease with a wide spectrum of activity against extracellular matrix components (CAMP82, CAMP88, MCWH89, and references therein). The enzyme is one of the major neutral proteases of the azurophil granules of polymorphonuclear leucocytes and is involved in the elimination of pathogens and in connective tissue restructuring (TRAV88). In cases of hereditary reduction of the circulating alpha-1-anti-protease inhibitor (α₁-PI), the principal physiological inhibitor of hNE (HEID86), or the inactivation of α₁-PI by oxidation ("smoker's emphysema"), extensive destruction of lung tissue may result from uncontrolled elastolytic activity of hNE (CANT89, BEIT86, HUBB86, HUBB89a,b, HUTC87, SOMM90, WEWE87). Several human respiratory disorders, including cystic fibrosis and emphysema, are characterized by an increased neutrophil burden on the epithelial surface of the lungs (SNID91, MCEL91, GOLD86) and hNE release by neutrophils is implicated in the progress of these disorders (MCEL91, WEIS89). hNE is implicated as an essential ingredient in the pernicious cycle of: 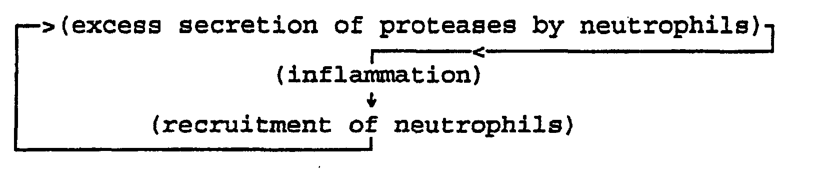 observed in cystic fibrosis (CF) (NADE90). Inappropriate hNE activity is very harmful and to stop the progression of emphysema or to alleviate the symptoms of CF, an inhibitor of very high affinity is needed. The inhibitor must be very specific to hNE lest it inhibit other vital serine proteases or esterases. Nadel (NADE90) has suggested that onset of excess secretion is initiated by 10⁻¹⁰ M hNE; thus, the inhibitor must reduce the concentration of free hNE to well below this level. Thus, hNE is an enzyme for which an excellent inhibitor is needed.

There are reports that suggest that Proteinase 3 (also known as p29 or PR-3) is as important or even more important than hNE; see NILE89, ARNA90, KAOR88, CAMP90, and GUPT90. Cathepsin G is another protease produced by neutrophils that may cause disease when present in excess; see FERR90, PETE89, SALV87, and SOMM90. Cathepsin G is less stable than hNE and thus harder to study *in vitro*. Powers and Harper (POWE86) indicate that cathepsin G is involved in inflammation, emphysema, adult respiratory distress syndrome, and rheumatoid arthritis.

*Proteinaceous Serine Protease Inhibitors*. A large number of proteins act as serine protease inhibitors by serving as a highly specific, limited proteolysis substrate for their target enzymes. In many cases, the reactive site peptide bond ("scissile bond") is encompassed in at least one disulfide loop, which insures that during conversion of virgin to modified inhibitor the two peptide chains cannot dissociate.

A special nomenclature has evolved for describing the active site of the inhibitor. Starting at the residue on the amino side of the scissile bond, and moving away from the bond, residues are named P1, P2, P3, etc. (SCHE67). Residues that follow the scissile bond are called P1', P2', P3', etc. It has been found that the main chain of protein inhibitors having very different overall structure are highly similar in the region between P3 and P3' with especially high similarity for P2, P₁ and P1' (LASK80 and works cited therein). It is generally accepted that each serine protease has sites S1, S2, etc. that receive the side groups of residues P1, P2, etc. of the substrate or inhibitor and sites S1', S2', etc. that receive the side groups of P1', P2', etc. of the substrate or inhibitor (SCHE67). It is the interactions between the S sites and the P side groups that give the protease specificity with respect to substrates and the inhibitors specificity with respect to proteases.

The serine protease inhibitors have been grouped into families according to both sequence similarity and the topological relationship of their active site and disulfide loops. The families include the bovine pancreatic trypsin inhibitor (Kunitz), pancreatic secretory trypsin inhibitor (Kazal), the Bowman-Birk inhibitor, and soybean trypsin inhibitor (Kunitz) families. Some inhibitors have several reactive sites on a single polypeptide chains, and these distinct domains may have different sequences, specificities, and even topologies.

One of the more unusual characteristics of these inhibitors is their ability to retain some form of inhibitory activity even after replacement of the P1 residue. It has further been found that substituting amino acids in the P₅ to P₅' region, and more particularly the P3 to P3' region, can greatly influence the specificity of an inhibitor. LASK80 suggested that among the BPTI (Kunitz) family, inhibitors with P1 Lys and Arg tend to inhibit trypsin, those with P1=Tyr, Phe, Trp, Leu and Met tend to inhibit chymotrypsin, and those with P1=Ala or Ser are likely to inhibit elastase. Among the Kazal inhibitors, they continue, inhibitors with P1 = Leu or Met are strong inhibitors of elastase, and in the Bowman-Kirk family elastase is inhibited with P1 Ala, but not with P1 Leu.

We will next discuss a number of proteinaceous anti-elastase and anti-cathepsin G inhibitors of particular interest. Known HNE and cathepsin G inhibitors include the Kunitz family inhibitor UTI (GEBH86), the eglin/barley family inhibitor eglin (SCHN86b), and the serpin family inhibitors alpha1-antichymotrypsin and alpha1-antitrypsin (BARR86).

*α*_{*1*}*-Proteinase Inhibitor (α1-antitrypsin)*. A logical approach to treatment of diseases attributable to excessive hNE levels is treatment with the endogenous irreversible inhibitor, α₁-PI. STON90 reports on studies of the efficacy of α1-PI in protecting hamster lung from damage by hNE. They conclude that α1-PI is only about 16% as effective *in vivo* as one might have estimated from in vitro measurements. Nevertheless, α1-PI shows a therapeutic effect. A preliminary study of aerosol administration of α₁-PI to cystic fibrosis patients indicates that such treatment can be effective both in prevention of respiratory tissue damage and in augmentation of host antimicrobial defenses (MCEL91).

However, there are practical problems with its routine use as a pulmonary anti-elastolytic agent. These include the relatively large size of the molecule (394 residues, 51 Kd), the lack of intramolecular stabilizing disulfide bridges, and specific post translational modifications of the protein by glycosylation at three sites. HEIM91 reports inhibition of PMN leukocyte-mediated endothelial cell detachment by protease inhibitors. They compared secretory leukocyte protease inhibitor (SLPI), α1-protease inhibitor (α1-PI), and a chloromethylketone inhibitor (CMK). While SLPI and CMK inhibited the hNE mediated cell detachment, α1-PI did not; the author suggest that, because of its size, α1-PI can not penetrate to the site at which hNE acts. As the inhibitors disclosed in the present invention are smaller than SLPI, we expect them to move freely throughout the extracellular space.

Moreover, both cleaved α₁-PI and the α₁-PI/hNE complex (BAND88a,b) may be neutrophil chemoattractants. This could be a serious disadvantage if one wishes to interupt the cycle by which excessive numbers of neutrophils migrate to the lung, release hNE, the hNE reacts with α₁-PI generating a signal for more neutrophils to migrate to the lungs. Hence a small, stable, nontoxic, and potent inhibitor of hNE would be of great therapeutic value.

*Human Pancreatic Secretory Trypsin Inhibitor*. This is a Kazal family inhibitor. The inhibitors of this family are stored in zymogen granules and secreted with the zymogens in pancreatic juice. In general, the natural Kazal inhibitors are specific for trypsin. However, there are exceptions, such as certain domains of ovomucoids and ovoinhibitors, that inhibit chymotrypsin, subtilisin and elastase.

While the wild type hPSTI is completely inactive toward hNE, Collins *et al*. (COLL90) report designed variants of human pancreatic secretory trypsin inhibitor (hPSTI) having high affinity for hNE. Three of the reported variants have Kᵢ for hNE below 10 pM: PSTI-5D36 at 7.3 pM, PSTI-4A40 at 7 pM, and PSTI-4F21 at 5.2 pM.

*Squash Seed Inhibitor.* The squash seed inhibitors are yet another family of serine protease inhibitors. Those reported so far have lysine or arginine at the P1 residue, inhibit trypsin, and are completely inactive toward hNE. McWherter *et al.* (1989) synthesized several homologues of the squash-seed inhibitor, CMTI-III. CMTI-III has a Kᵢ for trypsin of ≈1.5·10⁻¹² M. McWherter *et al*. (MCWH89) suggested substitution of "moderately bulky hydrophobic groups" at P1 to confer HLE (same as hNE) specificity. For cathepsin G, they expected bulky (especially aromatic) side groups to be strongly preferred. They found that PHE, LEU, MET, and ALA were functional by their criteria; they did not test TRP, TYR, or HIS. (Note that ALA has the second smallest side group available.) They found that a wider set of substituted residues (VAL, ILE, LEU, ALA, PHE, MET, and GLY) gave detectable binding to HLE. In particular, CMTI-III(VAL₅) has Kᵢ = 9 nM relative to hNE.

"*Kunitz*" *Domain Proteinase Inhibitors.* Bovine pancreatic trypsin inibitor (BPTI, a.k.a. aprotonin) is a 58 a.a. serine proteinase inhibitor of the BPTI (Kunitz) domain (KuDom) family. Under the tradename TRASYLOL, it is used for countering the effects of trypsin released during pancreatitis. Not only is its 58 amino acid sequence known, the 3D structure of BPTI has been determined at high resolution by X-ray diffraction (HUBE77, MARQ83, WLOD84, WLOD87a, WLOD87b), neutron diffraction (WLOD84), and by NMR (WAGN87). One of the X-ray structures is deposited in the Brookhaven Protein Data Bank as "6PTI" [sic]. The 3D structure of various BPTI homologues (EIGE90, HYNE90) are also known. At least sixty homologues have been reported; the sequences of 39 homologues are given in Table 13, and the amino acid types appearing at each position are compiled in Table 15. The known human homologues include domains of Lipoprotein Associated Coagulation Inhibitor (LACI) (WUNT88, GIRA89), Inter-α-Trypsin Inhibitor (ALBR83a, ALBR83b, DIAR90, ENGH89, TRIB86, GEBH86, GEBH90, KAUM86, ODOM90, SALI90), and the Alzheimer beta-Amyloid Precursor Protein. Circularized BPTI and circularly permuted BPTI have binding properties similar to BPTI (GOLD83). Some proteins homologous to BPTI have more or fewer residues at either terminus.

In BPTI, the P1 residue is at position 15. Tschesche *et al*. (TSCH87) reported on the binding of several BPTI P1 derivatives to various proteases:

### Dissociation constants for BPTI P1 derivatives, Molar.

| Residue #15 P1 | Trypsin (bovine pancreas) | Chymotrypsin (bovine pancreas) | Elastase (porcine pancreas) | Elastase (human leukocytes) |
|---|---|---|---|---|
| lysine | 6.0·10⁻¹⁴ | 9.0·10⁻⁹ | - | 3.5·10⁻⁶ (WT) |
| glycine | - | - | + | 7.0·10⁻⁹ |
| alanine | + | - | 2.8·10⁻⁸ | 2.5·10⁻⁹ |
| valine | - | - | 5.7·10⁻⁸ | 1.1·10⁻¹⁰ |
| leucine | - | - | 1.9·10⁻⁸ | 2.9·10⁻⁹ |

From the report of Tschesche *et al*. we infer that molecular pairs marked "+" have K_{d}s ≥ 3.5·10⁻⁶ M and that molecular pairs marked "-" have K_{d}s >> 3.5·10⁻⁶ M. It is apparent that wild-type BPTI has only modest affinity for hNE, however, mutants of BPTI with higher affinity are known. While not shown in the Table, BPTI does not significantly bind hCG. However, Brinkmann and Tschesche (BRIN90) made a triple mutant of BPTI (viz. K15F, R17F, M52E) that has a Kᵢ with respect to hCG of 5.0 x 10⁻⁷ M.

Works concerning BPTI and its homologues include: STAT87, SCHW87, GOLD83, CHAZ83, CREI74, CREI77a, CREI77b, CREI80, SIEK87, SINH90, RUEH73, HUBE74, HUBE75, HUBE77KIDOB8, PONT88, KID090, AUER87, AUER90, SCOT87b, AUER88, AUER89, BECK88b, WACH79, WACH80, BECK89a, DUFT85, FIOR88, GIRA89, GOLD84, GOLD88, HOCH84, RITO83, NORR89a, NORR89b, OLTE89, SWAI88, and WAGN79.

Inter-α-trypsin inhibitor (ITI) is a large (Mᵣ *ca* 240,000) circulating protease inhibitor found in the plasma of many mammalian species (for reviews see ODOM90, SALI90, GEBH90, GEBH86). Its affinity constant for hNE is 60-150 nM; for Cathepsin G it is 20-6000 nM. The intact inhibitor is a glycoprotein and is currently believed to consist of three glycosylated subunits that interact through a strong glycosaminoglycan linkage (ODOM90, SALI90, ENGH89, SELL87). The anti-trypsin activity of ITI is located on the smallest subunit (ITI light chain, unglycosylated Mᵣ ca 15,000) which is identical in amino acid sequence to an acid stable inhibitor found in urine (UTI) and serum (STI) (GEBH86, GEBH90). The mature light chain consists of a 21 residue N-terminal sequence, glycosylated at SER₁₀, followed by two tandem KuDoms the first of which is glycosylated at ASN₄₅ (ODOM90). In the human protein, the second KuDom (ITI-D2 or HI-8t) has been shown to inhibit trypsin, chymotrypsin, and plasmin (ALBR83a, ALBR83b, SELL87, SWAI88). The first domain (ITI-D1 or HI-8e, comprising residues 22-76 of the UTI sequence shown in Fig. 1 of GEBH86) lacks these activities (ALBR83a,n, SWAI88) but has been reported to inhibit leukocyte elastase (10⁻⁶> Kᵢ > 10⁻⁹) (-ALBR83a,b, ODOM90) and cathepsin G (SWAI88, ODOM90). The affinity is, however, too weak to be directly useful.

Sinha *et al.* (SINH91) report converting the KuDom of Alzheimer's β-amyloid precursor protein into an hNE inhibitor having Kᵢ = 800 pM when valine was substituted for arginine at the P1 site (residue 13). They made a second protein having three mutations (*viz*. R13V(P1), A14S(P1'), M15I(P2')). The changes at P1' and P2' correspond to the amino acids found in the active site of α₁-PI. This protein is completely inactive with respect to hNE. They state, "Caution should therefore be used in extrapolating site-specific mutagenesis results among mechanistically unrelated inhibitors. In addition, unpredictable results can be obtained even within the KuDom family, as our experience with chymotrypsin and kallikrein illustrate."

*Nonproteinaceous Elastase Inhibitors.* The compounds ICI 200,355 (SOMM91) and ICI 200,880 show strong preference for HNE over other proteases such as trypsin. These compounds are analogues of peptides in which the amide nitrogen of the scissile bond has been replaced by a CF₃ group. Each of these compounds have an isopropyl group (as does valine) at the P1 position and a prolyl residue at P2. Neither compound has any extension toward P1'. Imperiali and Abeles (IMPE86) describe protease inhibitors consisting of acetyl peptidyl methyl ketones in which the terminal methyl group bears zero to three fluorine atoms; there is no P1' residue in any of their compounds. PEET90 (and works cited therein) report synthesis of peptidyl fluoromethyl ketones and peptidyl α-keto esters and the inhibitory properties of these compounds relative to porcine pancreatic elastase (PPB), HME, rat cathepsin G, and human cathepsin G; these compounds do not extend to P1'. Mehdi et al. (MEHD90) report inhibition of HNE and human cathepsin G by methyl esters of peptidyl α-keto carboxylic acids; none of these compounds contain P1' residues. Angelastro et al. (ANGE90) report protease inhibitors having diketo groups; none of these compounds extend beyond P1.

Govhardan and Abeles (GOVH90) describe compounds in which the amide -NH- has been replaced by -CF₂-CH₂- followed by an α-amino-linked amino-acid methyl ester, thus providing a P1' residue.

Imperiali and Abeles (IMPR87) describe inhibitors of chymotrypsin extending to P3'. Works cited by these authors indicate that the inhibitory constant, Kᵢ, can be lowered by specifically matching the S1', S2', S3', ... binding sites on the protease. These authors do not discuss inhibition of HNE. Furthermore, their inhibitors are not derived from high affinity protein protease inhibitors; rather the side groups at P1', P2', and P3' are determined by trial and error. In addition, between P1 and P1', they insert -CO-CF₂-CH₂- in place of -CO-NH- so that the distal part of the chain is displaced. We prefer to replace -CO-NH- with -CO-CF₂- or -CO-CFH- so that the remainder of the residues can take up conformations highly similar to those found in EpiNE proteins.

Another class of protease inhibitors are those in which the carbonyl carbon of the scissile peptide is replaced by boron. These compounds inhibit serine proteases, but are not very specific.

Another class of elastase inhibitors are the chloromethylketones as described by Robert et al. (US 4,665,053). These compounds have a chlorine atom adjacent to a keto group. The active-site serine of the protease acts as a nucleophile, displacing chloride and yielding a covalent enzyme-inhibitor adduct that is irreversibly inactive. An-Zhi et al. (FEBS Lett, **234** (2) 367-373 (1988)) describe the X-ray crystal structure of HNE with a peptidyl chloromethyl ketone. Tsuda et al. (Chem Pharm Bull, **35**(9)3576-84 (1987)) describe synthesis of peptide chloromethyl ketones and their activity against proteases, including HNE. Ganu and Shaw (Thrombosis Research, **45**:1-6 (1987)) describe improved peptidyl chloromethyl ketone plasmin inhibitors. Because the chloromethyl ketones form irreversible adducts, they are less desirable as drugs. Other classes of inhibitors that form irreversible complexes include a) peptide enol lactones (J Biol Chem **266**(1)13-21 (1991) and Biochemistry **29**:4305-11 (1990)), isocoumarins (Krantz et al., US 4,657,893, Powers et al., US 4,845,242, and Kobuko et al., US 4,980287), and peptidyl (a-aminoalkyl)phosphonate diphenyl esters (Biochemistry **30**:485-93 (1991)).

A class of compounds, related to the chloromethyl ketones, that bind reversibly to proteases with some degree of specificity comprises peptidyl methyl ketones. Peters and Fittkau (Biomed Biochim Acta **49**(4)173-178 (1990) and works cited therein) report that peptidyl methyl ketones bind serine- and cysteine-proteases reversibly and that the binding depends on the sequence of the peptidyl group. If the peptidyl methyl ketones are viewed as peptide analogues in which the carbonyl group of an amino acid is replaced by a methyl group, Peters and Fittkau discuss only compounds that are extended toward the amino terminus. Thus, they supply P1, P2, etc., but not P1', P2', etc.

*Miscellaneous Information* on *Elastase Inhibition*. PADR91 reports that elastin (the definitive substrate of all elastases) greatly reduces the efficacy of a variety of reversible and irreversible hNE inhibitors when compared to the efficacy determined with small, soluble artificial substrates. They found that both classes of inhibitors have from 20-fold to more than 100-fold less efficacy. They suggest that elastin reduces the on rate, but say they have no explanation for this phenomenon. One possibility is that the synthetic inhibitors (all rather hydrophobic) bind to elastin (which is also hydrophobic). They tested one reversible protein inhibitor, mucus protease inhibitor, which has Kᵢ = 30 nM without elastin or 900 nM with elastin. If our inhibitors suffer a 30-fold loss of efficacy, they can still reduce free hNE to below 10⁻¹⁰ M.

No admission is made that any cited reference is prior art or pertinent prior art, and the dates given are those appearing on the reference and may not be identical to the actual publication date. All references cited in this specification are hereby incorporated by reference.

### SUMMARY OF THE INVENTION

The present invention relates to mutants of Kunitz Domain serine protease inhibitors, such as BPTI and ITI-D1, with substantially enhanced affinity for elastase.

Thus, the invention relates to a non-naturally occurring protein which inhibits human neutrophil elastase, and which is a protein comprising at least the core sequence of a non-naturally occurring Kunitz domain, a Kunitz domain being characterized by cysteines at positions corresponding to bovine pancreatic trypsin inhibitor (BPTI) positions 5, 30, 51, and 55, glycine at a position corresponding to BPTI position 12, Asn at a position corresponding to BPTI 43 and Phe at a position corresponding to BPTI 33, with the proviso that when the positions corresponding to BPTI 14 and 38 are cysteine, the position corresponding to BPTI 37 is glycine; the core sequence being the residues corresponding to BPTI positions 5-55; where, in said non-naturally occurring Kunitz domain, the residue corresponding to BPTI position 18 is Phe, and where the residues corresponding to BPTI positions 39-42 are all uncharged amino acids.

These muteins have an affinity for elastase estimated to be at least an order of magnitude higher than that of the wild-type domain and, in some instances, at least three orders of magnitude (1000-fold) higher.

For some of the proteins, kinetic inhibitory data show that the binding affinity is in the range 1.0 x 10⁻¹² M to 3.0 x 10⁻¹² M. Other proteins are displayed on fusion phage and the affinity for hNE or hCG is estimated by the pH elution profile from immobilized active protease (hNE or hCG). A number of the proteins have been produced in useful quantities as secreted proteins in yeast.

The present invention also relates to linear and cyclic oligopeptide analogues of aprotonin and related polypeptides that specifically bind human neutrophil elastase and/or cathepsin G. It relates in particular to analogues of the novel elastase-binding polypeptides (EpiNe) and cathepsin G-binding polypeptides disclosed herein.

These analogues differ from aprotonin and its kindred inhibitors in several respects. First, they are smaller molecules, preferably less than 1,500 daltons molecular weight, and including only the P₅-P₅' residues (or analogues thereof) or a subset thereof. Secondly, the scissile peptide (-CO-NH-) bond between the P₁ and P₁' residues is replaced by a substantially nonhydrolyzable bond that substantially maintains the distance between the alpha carbons of those two residues.

### BRIEF DESCRIPTION OF THE DRAWINGS

Figure 1 illustrates fractionation of the Mini PEPI library on hNE beads. The abscissae shows pH of buffer. The ordinants show amount of phage (as fraction of input phage) obtained at given pH. Ordinants scaled by 10³.

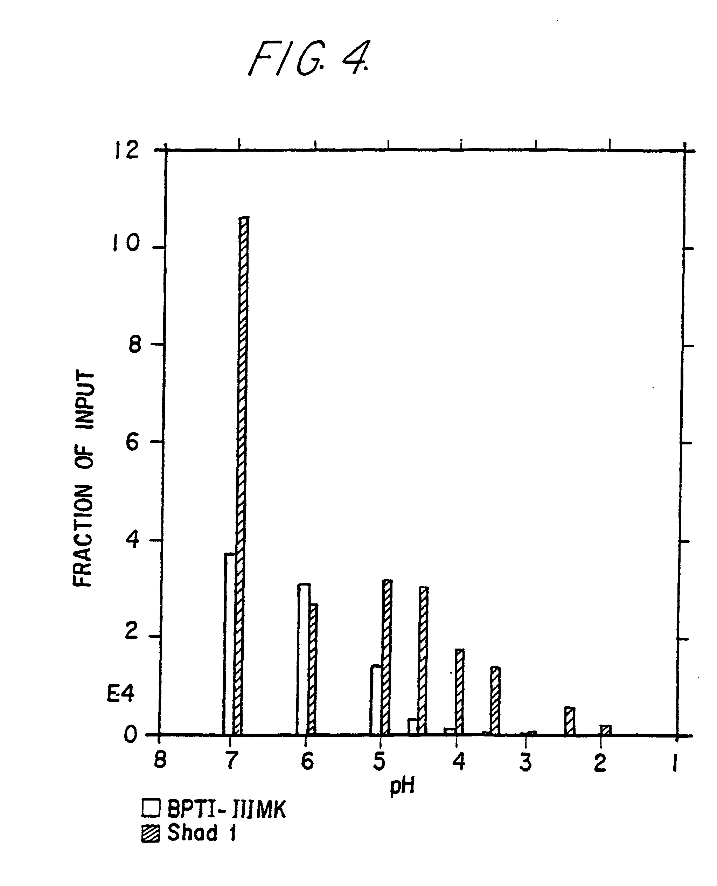

Figure 2 illustrates fractionation of the MYMUT PEPI library on hNE beads. The abscissae shows pH of buffer. The ordinants show amount of phage (as fraction of input phage) obtained at given pH. Ordinants scaled by 10³.
Figure 3 shows the elution profiles for EpiNE clones 1, 3, and 7. Each profile is scaled so that the peak is 1.0 to emphasize the shape of the curve.
Figure 4 shows pH profile for the binding of BPTI-III MK and EpiNE1 on cathepsin G beads. The abscissae shows pH of buffer. The ordinants show amount of phage (as fraction of input phage) obtained at given pH. Ordinants scaled by 10³.

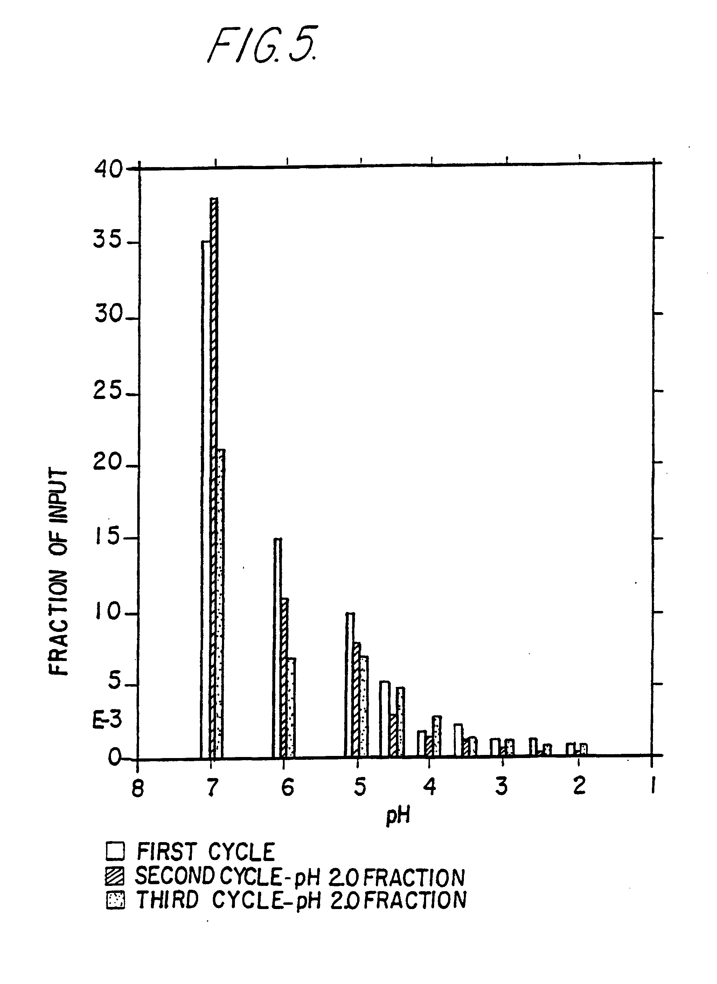

Figure 5 shows pH profile for the fractionation of the MYMUT Library on cathepsin G beads. The abscissae shows pH of buffer. The ordinants show amount of phage (as fraction of input phage) obtained at given pH. Ordinants scaled by 10³.

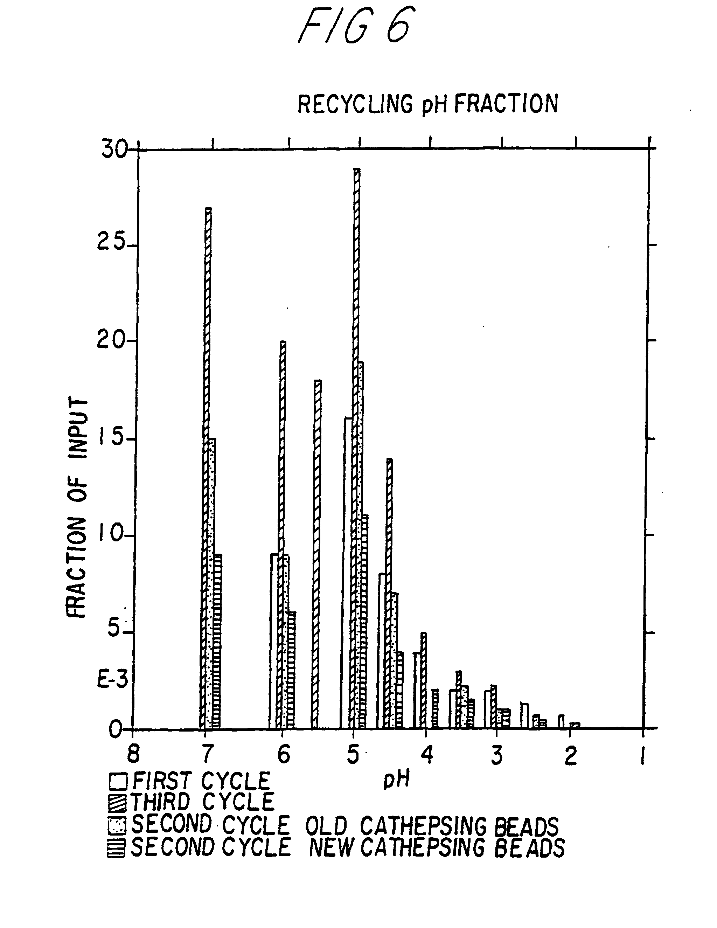

Figure 6 shows a second fractionation of MYMUT library over cathepsin G.
Figure 7 shows elution profiles on immobilized cathepsin G for phage selected for binding to cathepsin G.
Figure 8 shows the form of one group of preferred HNE inhibitors, hereinafter Class I inhibitors. Carbons marked 7, 8, 9, and 10 are chiral centers.
Figure 9 shows the form of a second group of preferred HNE inhibitors, hereinafter Class II inhibitors. Carbons marked 7, 8, 9, and 10 are chiral centers.
Figure 10 shows 2-carboxymethyl-6-aminomethyl anthraquinone as a linker. Other relatively rigid molecules of similar dimension can be used.
Figure 11 shows compounds I through XVIII involved in preparing analogues of the VAL-ALA dipeptide having -NH- replaced by -CF₂-, -CH₂-, or -CHF- for Class I and Class II inhibitors.
Figure 12 shows the form of a third group of preferred HNE inhibitors, hereinafter Class III inhibitors. Carbons marked 8, 9 and 10 are chiral centers.
Figure 13 shows compounds XXXI through XXXV that are involved in synthesizing the boron-containing dipeptide analogue used in Class I and Class II inhibitors.
Figure 14 shows compounds XLI through XLIV that are involved in synthesis of a portion of the molecule shown in Figure 5.

### DETAILED DESCRIPTION OF THE PREFERRED EMBODIMENTS

### Small proteins With High Affinity for Elastase or Cathepsin G

The present invention relates to muteins of BPTI, ITI-D1 and other Kunitz domain-type inhibitors which have a high affinity for elastase and cathepsin G. Some of the described inhibitors are derived from BPTI and some from ITI-D1. However, hybrids of the identified muteins and other Kunitz domain-type inhibitors could be constructed.

For the purpose of simultaneously assessing the affinity of a large number of different BPTI and ITI-D1 muteins, DNA sequences encoding the BPTI or ITI-DI was incorporated into the genome of the bacteriophage M13. The KuDom is displayed on the surface of M13 as an amino-terminal fusion with the gene III coat protein. Alterations in the KuDom amino acid sequence were introduced. Each pure population of phage displaying a particular KuDom was characterized with regard to its interactions with immobilized hNE or hCG. Based on comparison to the pH elution profiles of phage displaying other KuDoms of known affinities for the particular protease, mutant KuDoms having high affinity for the target proteases were identified. Subsequently, the sequences of these mutant KuDoms were determined (typically by sequencing the corresponding DNA sequence).

Certain aprotonin-like protease inhibitors were shown to have a high affinity for HNE (≈10¹²/M). These 58 amino acid polypeptides were biologically selected from a library of aprotinin mutants produced through synthetic diversity. Positions P1, P1', P2', P3', and P4' were varied. At P1, only VAL and ILE were selected, although LEU, PHE, and MET were allowed by the synthetic conditions. At P1', ALA and GLY were allowed and both were found in proteins having high affinity. (While not explored in the library, many Kazal family inhibitors of serine proteases have glutamic or aspartic acid at P1'.) All selected proteins contained either PHE or MET at P2'; LEU, ILE, and VAL, which are amino acids with branched aliphatic side groups, were in the library but apparently hinder binding to HNE. Surprisingly, position P3' of all proteins selected for high affinity for HNE have phenylalanine. No one had suggested that P3' was a crucial position for determining specificity relative to HNE. At P4', SER, PRO, THR, LYS, and GLN were allowed; all of these except THR were observed. PRO and SER are found in the derivatives having the highest affinity.

As previously noted, BPTI is a protein of 58 amino acids. The sequence of BPTI is given in entry 1 of Table 13. The invention is not limited to 58-amino-acid proteins, as homologues having more or fewer amino acids are expected to be active.

Wild-type BPTI is not a good inhibitor of hNE. BPTI with a single K15L mutation exhibits a moderate affinity for HNE (K_{d} = 2.9·10⁻⁹ M) (BECK88b). However, the amino terminal Kunitz domain (BI-8e) of the light chain of bovine inter-α-trypsin inhibitor has been generated by proteolysis and shown to be a potent inhibitor of HNE (K_{d} = 4.4·10⁻¹¹ M) (ALBR83).

It has been proposed that the P1 residue is the primary determinant of the specificity and potency of BPTI-like molecules (SINH91, BECK88b, LASK80 and works cited therein). Although both BI-8e and BPTI(K15L) feature LEU at their respective P1 positions, there is a 66 fold difference in the affinities of these molecules for HNE. We therefore hypothesized that other structural features must contribute to the affinity of BPTI-like molecules for HNE.

A comparison of the structures of BI-8e and BPTI (K15L) reveals the presence of three positively charged residues at positions 39, 41, and 42 of BPTI which are absent in BI-8e. These hydrophilic and highly charged residues of BPTI are displayed on a loop which underlies the loop containing the P1 residue and is connected to it *via* a disulfide bridge. Residues within the underlying loop (in particular residue 39) participate in the interaction of BPTI with the surface of trypsin (BLOW72) and may contribute significantly to the tenacious binding of BPTI to trypsin. These hydrophilic residues might, however, hamper the docking of BPTI variants with HNE. Supporting this hypothesis, BI-8e displays a high affinity for HNE and contains no charged residues in residues 39-42. Hence, residues 39 through 42 of wild type BPTI were replaced with the corresponding residues (MGNG) of the human homologue of BI-8e. As we anticipated, a BPTI(K15L) derivative containing the MGNG 39-42 substitution exhibited a higher affinity for HNE than did the single substitution mutant BPTI(K15L). Mutants of BPTI with Met at position 39 are known, but positions 40-42 were not mutated simultaneously.

Tables 207 and 208 present the sequences of additional novel BPTI mutants with high affinity for hNE. We believe these mutants to have an affinity for hNE which is about an order of magnitude higher than that of BPTI (K15V, R17L). All of these mutants contain, besides the active site mutations shown in the Tables, the MGNG mutation at positions 39-42.

Similarly, Table 209 presents the sequences of novel BPTI mutants with high affinity for cathepsin G. The P1 residue in the Epic mutants is predominantly MET, with one example of PHE, while in BPTI P1 is LYS and in the EpiNE variants P1 is either VAL or ILE. In the EpiC mutants, P1' (residue 16) is predominantly ALA with one example of GLY and P2' (residue 17) is PHE, ILE, or LEU. Interestingly, residues 16 and 17 appear to pair off by complementary size, at least in this small sample. The small GLY residue pairs with the bulky PHE while the relatively larger ALA residue pairs with the less bulky LEU and ILE. Alternatively, the pairing could be according to flexibility at P1'; glycine at P1' might allow the side group of phenylalanine to reach a pocket that is not accessible when P1' is alanine. When P1' is alanine, leucine or isoleucine appear to be the best choice.

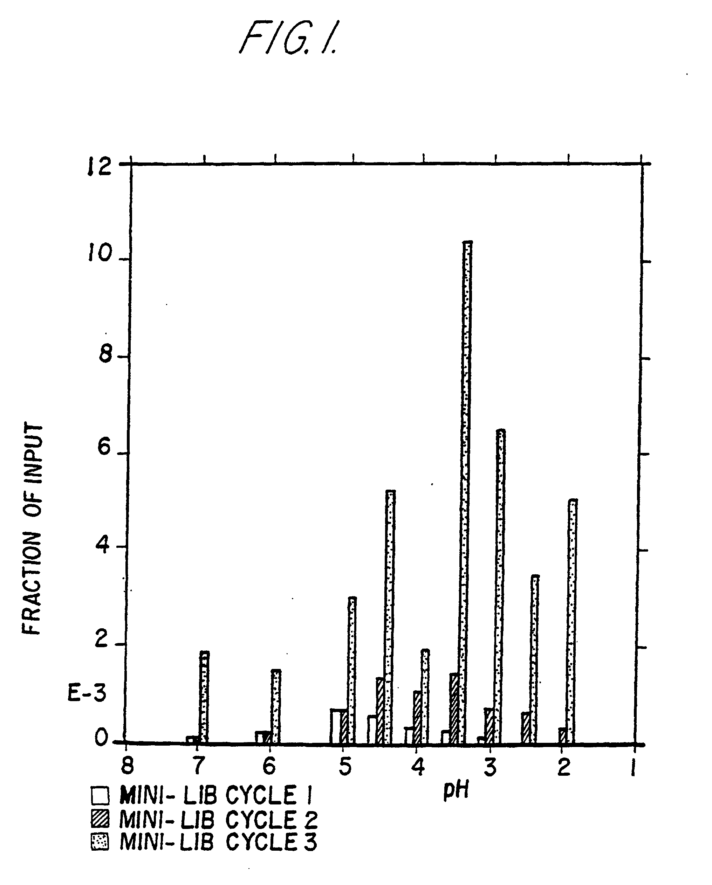

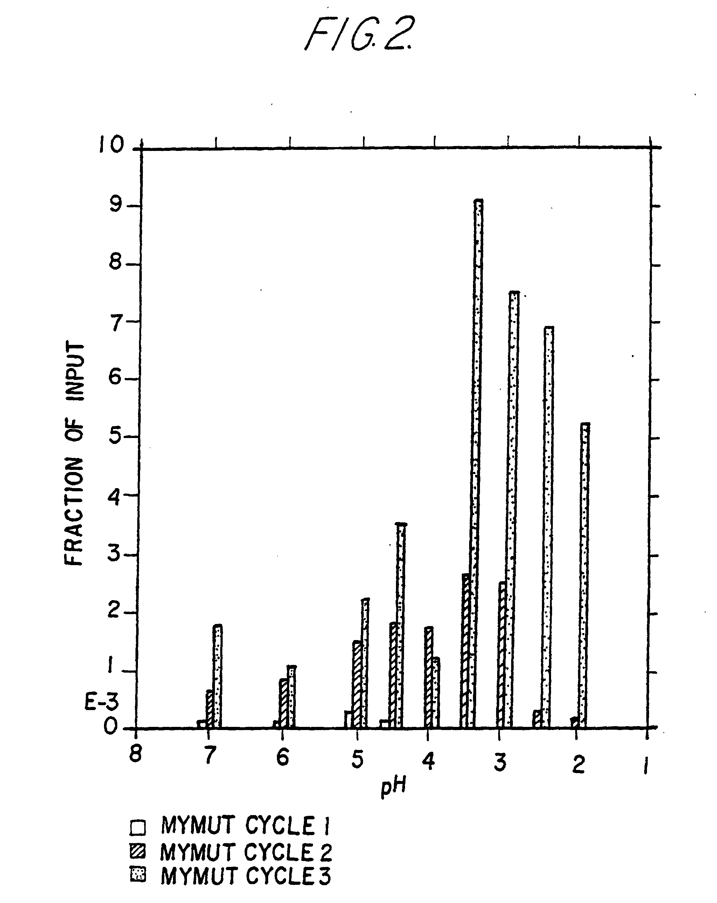

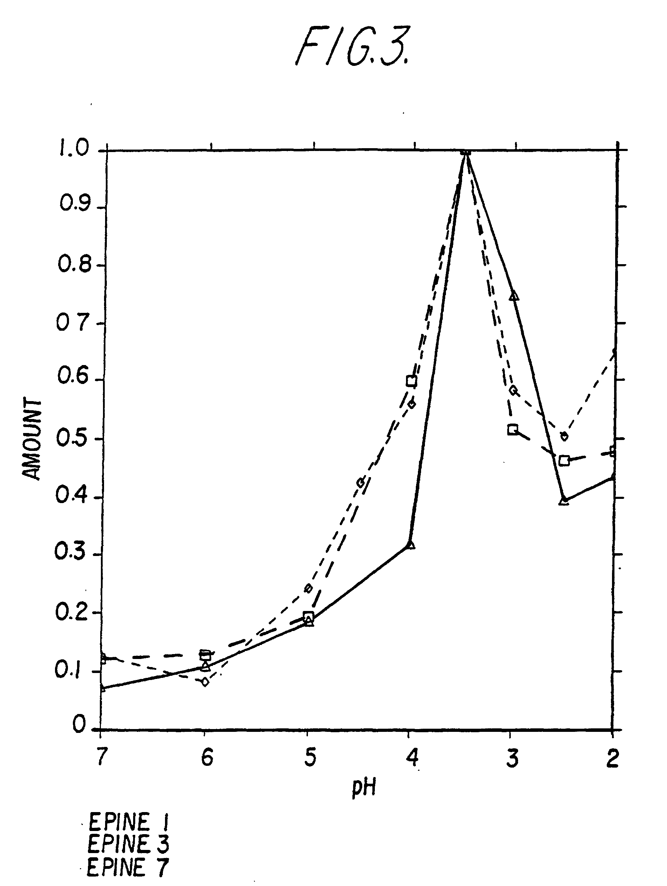

Although BPTI has been used in humans with very few adverse effects, a KuDom having much higher similarity to a human KuDom poses much less risk of causing an immune response. Thus, we transferred the active site changes found in EpiNE7 into the first KuDom of inter-α-trypsin inhibitor (Example IV). For the purpose of this application, the numbering of the nucleic acid sequence for the ITI light chain gene is that of TRAB86 and that of the amino acid sequence is the one shown for UTI in FIg. 1 of GEBHB6. The necessary coding sequence for ITI-DI is the 168 bases between positions 750 and 917 in the cDNA sequence presented in TRAB86. The amino acid sequence of human ITI-D1 is 56 amino acids long, extending from Lys-22 to Arg-77 of the complete ITI light chain sequence. The P1 site of ITI-DI is Met-36. Tables 220-221 present certain ITI mutants; note that the residues are numbered according to the homologus Kunitz domain of BPTI, i.e., with the P1 residue numbered 15. It should be noted that it is probably acceptable to truncate the amino-terminal of ITI-D1, at least up to the first residue homologous with BPTI.

The EpiNE7-inspired mutation (BPTI 15-19 region) of ITI-D1 significantly enhanced its affinity for hNE. We also discovered that mutation of a different part of the molecule (BPTI 1-4 region) provided a similar increase in affinity. When these two mutational patterns were combined, a synergistic increase in affinity was observed. Further mutations in nearby amino acids (BPTI 26, 31, 34) led to additional improvements in affinity.

The elastase-binding muteins of ITI-DI envisioned herein preferably differ from the wild-type domain at one or more of the following positions (numbered per BPTI) : 1, 2, 4, 15, 16, 18, 19, 31 and 34. More preferably, they exhibit one or more of the following mutations: Lys1 -> Arg; Glu2 -> Pro; Ser4 -> Phe*; Met15 -> Val*, Ile; Gly16 -> Ala; Thr18 -> Phe*; Ser19 -> Pro; Thr26 -> ALa; Glu31 -> Gln; Gln34 -> Val*. Introduction of one or more of the starred mutations is especially desirable, and, in one preferred embodiment, at least all of the starred mutations are present.

It will be recognized by those of ordinary skill in the art that the identified HNE and HCG inhibitors, may be modified in such a manner that the change will not greatly diminish the affinity, specificity, or stability of the inhibitor. Proposed changes can be assessed on several bases. First we ask whether a particular amino acid can fit into the KuDom framework at a given location; a change that disrupts the framework is very likely to impair binding and lower specificity. The likelihood that an amino acid can fit into the KuDom framework can be judged in several ways: 1) does the amino acid appear there in any known KuDom? 2) Do structural models of KuDoms indicate compatibility between the structure and the proposed substitution? and 3) do dynamic computational models suggest that the proposed mutant protein will be stable? The sequence variability of naturally-occurring KuDoms gives us proof that certain amino acids are acceptable at certain locations; lack of examples does not prove that the amino acid won't fit.

If a proposed change is deemed to be structurally acceptable, we then ask what effect it is likely to have on binding to the target and to other substances. Generally, a mutant protein having a changed residue in the interface between KuDom and target will need to be tested, usually via binding studies of a phage that displays the mutant protein. Most changes in the binding interface reduce binding, but some do increase affinity. Changes at residues far-removed from the binding interface usually do not reduce binding if the protein is not destabilized.

Table 61 shows the variability of 39 naturally-occurring Kunitz domains. All these proteins have 51 residues in the region C₅ through C₅₅; the total number of residues varies due to the proteins having more or fewer residues at the termini. Table 62 list the names of the proteins that are included in Table 61. Table 64 cites works where these sequences are recorded. Table 63 shows a histogram of how many loci show a particular variability *vs*. the variability. "Core" refers to residues from 5 to 55 that show greater sequence and structural similarity than do residues outside the core.

At ten positions a single amino-acid type is observed in all 42 cases, these are C₅, G₁₂, C₁₄, C₃₀, F₃₃, G₃₇, C₃₈, N₄₃, C₅₁, and C₅₅. Although there are reports that each of these positions may be substituted without complete loss of structure, only G₁₂, C₁₄, G₃₇, and C₃₈ are close enough to the binding interface to offer any incentive to make changes. G₁₂ is in a conformation that only glycine can attain; this residue is best left as is. Marks *et al.* (MARK87) replaced both C₁₄ and C₃₈ with either two alanines or two threonines. The C₁₄/C₃₈ cystine bridge that Marks *et al*. removed is the one very close to the scissile bond in BPTI; surprisingly, both mutant molecules functioned as trypsin inhibitors. Both BPTI(C14A,C38A) and BPTI(C14T,C38T) are stable and inhibit trypsin. Altering these residues might give rise to a useful inhibitor that retains a useful stability, and the phage-display of a variegated population is the best way to obtain and test mutants that embody alterations at either 14 or 38. Only if the C₁₄/C₃₈ disulfide is removed, would the strict conservation of G₃₇ be removed.

At seven positions (*viz.* 23, 35, 36, 40, 41, 45, and 47) only two amino-acid types have been found. At position 23 only Y and F are observed; the para position of the phenyl ring is solvent accessible and far from the binding site. Changes here are likely to exert subtle influences on binding and are not a high priority for variegation. Similarly, 35 has only the aromatic residues Y and W; phenylalanine would probably function well here. At 36, glycine predominates while serine is also seen. Other amino acids, especially {N, D, A, R}, should be allowed and would likely affect binding properties. Position 40 has only G or A; structural models suggest that other amino acids would be tolerated, particularly those in the set {S, D, N, E, K, R, L, M, Q, and T}. Position 40 is close enough to the binding site that alteration here might affect binding. At 41, only N, and K have been seen, but any amino acid, other than proline, should be allowed. The side group is exposed, so hydrophilic side groups are preferred, especially {D, S, T, E, R, Q, and A}. This residue is far enough from the binding site that changes here are not expected to have big effects on binding. At 45, F is highly preferred, but Y is observed once. As one edge of the phenyl ring is exposed, substitution of other aromatics (W or H) is likely to make molecules of similar structure, though it is difficult to predict how the stability will be affected. Aliphatics such as leucine or methionine (not having branched C_{β}s) might also work here. At 47, only S and T have been seen, but other amino acids, especially {N, D, G, and A}, should give stable proteins.

At one position (44), only three amino-acid types have been observed. Here, asparagine predominates and may form internal hydrogen bonds. Other amino acids should be allowed, excepting perhaps proline.

At the remaining 40 positions, four or more amino acids have been observed; at 28 positions, eight or more amino-acid types are seen. Position 25 exhibits 13 different types and 5 positions (1, 6, 17, 26, and 34) exhibit 12 types. Proline (the most rigid amino acid) has been observed at fourteen positions: 1, 2, 8, 9, 11, 13, 19, 25, 32, 34, 39, 49, 57, and 58. The φ,ψ angles of BPTI (CREI84, Table 6-3, p. 222) indicate that proline should be allowed at positions 1, 2, 3, 7, 8, 9, 11, 13, 16, 19, 23, 25, 26, 32, 35, 36, 40, 42, 43, 48, 49, 50, 52, 53, 54, 56, and 58. Proline occurs at four positions (34, 39, 57, and 58) where the BPTI φ, ψ angles indicate that it should be unacceptable. We conclude that the main chain rearranges locally in these cases.

Based on these data and excluding the six cysteines, we judge that the KuDom structure will allow those substitutions shown in Table 65. The class indicates whether the substitutions: A) are very likely to give a stable protein having substantially the same binding to hNE, hCG, or some other serine protease as the parental sequence, B) are likely to give similar binding as the parent, or C) are likely to give a proteins retaining the KuDom structure, but which are likely to affect the binding. Mutants in class C must be tested for affinity, which is relatively easy using a display-phage system, such as the one set forth in WO/02809. The affinity of hNE and hCG inhibitors is most sensitive to substitutions at positions 15, 16, 17, 18, 34, 39, 19, 13, 11, 20, 36 of BPTI, if the inhibitor is a mutant of ITI-D1, these positions must be converted to their ITI-D1 equivalents by aligning the cysteines in BPTI and ITI-D1.

Certain of our hNE inhibitors will be useful as PR-3 inhibitors. We have modeled the interaction of our inhibitors with hNE by reference to the BPTI-trypsin complex. First we listed the residues of trypsin that touch BPTI. Next we considered the corresponding sets of residues from hNE and PR-3. These sets differ at eleven residues. Only one of the differences occurs in the S1 specificity pocket, viz. V₁₉₀ in hNE vs. I₁₉₀ in PR3. We therefore believe that our hNE inhibitors are likely to be PR-3 inhibitors as well. In particular, the inhibitors having valine at P1 are likely to inhibit PR3. PR3 has an extra methyl in this region so the inhibitors having one fewer methyls are more likely to bind tightly.

BPTI is quite small; if this should cause a pharmacological problem, such as excessively quick elimination from the circulation, two or more BPTI-derived domains may be joined by a linker. This linker is preferably a sequence of one or more amino acids. A preferred linker is one found between repeated domains of a human protein, especially the linkers found in human BPTI homologues, one of which has two domains (BALD85, ALBR83b) and another of which three (WUNT88). Peptide linkers have the advantage that the entire protein may then be expressed by recombinant DNA techniques. It is also possible to use a nonpeptidyl linker, such as one of those commonly used to form immunogenic conjugates. For example, a BPTI-like KuDom to polyethyleneglycol, so called PEGylation (DAVI79).

Another possible pharmacological problem is immunigenicity. BPTI has been used in humans with very few adverse effects. Siekmann *et al.* (SIEK89) have studied immunological characteristics of BPTI and some homologues. Furthermore, one can reduce the probability of immune response by starting with a human protein. Thus, by changing nonessential residues, one may change the protein to more closely resemble a human protein. Other modifications, such as PEGylation, have also been shown to reduce immune responce (DAVI79).

### Reference Example

### Affinity Measurements

The affinity of a protein for another molecule can be measured in many ways. Scatchard (*Ann NY Acad Sci* (1949) **51**:660-669) described a classical method of measuring and analysing binding which has been applied to the binding of proteins. This method requires relatively pure protein and the ability to distinguish bound protein from unbound.

A second method appropriate for measuring the affinity of inhibitors for enzymes is to measure the ability of the inhibitor to slow the action of the enzyme. This method requires, depending on the speed at which the enzyme cleaves substrate and the availability of chromogenic or fluorogenic substrates, tens of micrograms to milligrams of relatively pure inhibitor.

A third method of determing the affinity of a protein for a second material is to have the protein displayed on a genetic package, such as M13, an measure the ability of the protein to adhere to the immobilized "second material". This method is highly sensitive because the genetic packages can be amplified. This approach is not entirely new. Makela, O, H Sarvas, and I Seppala ("Immunological Methods Based on Antigen-Coupled Bacteriophages.", *J Immunol Methods* (1980), 37:213-223) discuss methods of using haptans chemically conjugated to bacteriophage to measure the concentration of antibodies having affinity for the haptans. The present invention uses a novel approach, in that the binding protein is genetically encoded by the phage. Furthermore, we obtain at least semiquantitative values for the binding constants by use of a pH step gradiant. Inhibitors of known affinity for the immobilized protease are used to establish standard profiles against which other phage-displayed inhibitors are judged. Table 203 shows the profile of BPTI-phage and of BPTI(K15L)-phage when these phage are eluted from immobilized hNE. The profiles may vary from one batch of immobilized protease to the next and with the age of the immobilized preparation. Nevertheless, the relative shapes of profiles allow us to identify superior inhibitors.

Ascenzi *et al.* (ASCE90) studied the thermodynamics of binding of BPTI to human and bovine clotting factor Xₐ. They found that K_{A} dropped more than 30-fold as the pH was lowered from 9 to 5. That K_{A} changes with pH is likely to be general to the binding of serine proteases to KuDoms (and other inhibitors) because of the histidine found in the active site. The pH at which these changes occur is characteristic for the particular protease and inhibitor. It can be seen that protonating the active-site histidine when an inhibitor is bound involves burying a charge, usually energetically unfavorable. The reciprocal effect is that an inhibitor that binds very tightly effectively lowers the pKₐ of the imidazole for protonation.

Throughout the present specification, shaken incubations used Labquake shakers.

### Preparation of Immobilized Human Neutrophil Elastase

One ml of Reacti-Gel 6 x CDI activated agarose (Pierce Chemical Co.) in acetone (200 µl packed beads) was introduced into an empty Select-D spin column (5Prime-3Prime). The acetone was drained out and the beads were washed twice rapidly with 1.0 ml of ice cold water and 1.0 ml of ice cold 100 mM boric acid, pH 8.5, 0.9% NaCl. Two hundred µl of 2.0 mg/ml human neutrophil elastase (hNE) (CalBiochem, San Diego, CA) in borate buffer were added to the beads. The column was sealed and mixed end over end on a Labquake Shaker at 4°C for 36 hours. The hNE solution was drained off and the beads were washed with ice cold 2.0 M Tris, pH 8.0 over a 2 hour period at 4°C to block remaining reactive groups. A 50% slurry of the beads in TBS/BSA was prepared. To this was added an equal volume of sterile 100% glycerol and the beads were stored as a 25% slurry at -20°C. Prior to use, the beads were washed 3 times with TBS/BSA and a 50% slurry in TBS/BSA was prepared.

### EXAMPLE I

### CHARACTERIZATION AND FRACTIONATION OF CLONALLY PURE POPULATIONS OF PHAGE, EACH DISPLAYING A SINGLE CHIMERIC APROTININ HOMOLOGUE/M13 GENE III PROTEIN:

This Example demonstrates that chimeric phage proteins displaying a target-binding domain can be eluted from immobilized target by decreasing pH, and the pH at which the protein is eluted indicates the binding affinity of the domain for the target.

### Standard Procedures:

Unless otherwise noted, all manipulations were carried out at room temperature. Unless otherwise noted, all cells are XL1-Blue⁽™⁾ (Stratagene, La Jolla, CA).

### 1) Demonstration of the Binding of BPTI-III MK Phage to Active Trypsin Beads

We demonstrated that BPTI-III display phage bind immobilized active trypsin. Demonstration of the binding of display phage to immobilized active protease and subsequent recovery of infectious phage with a characteristic pH elution profile facilitates evaluation of particular mutants because one need not produce and purify tens of micrograms of each mutant protein.

Phage MK is derived from M13 by inserting a *kan*^{R} gene into the intergenic region. BPTI-III MK phage are derived from MK by inserting into gene *III*, between the codons specifiying the signal sequence and those specifying the mature protein, DNA encodine BPTI. Phage MA is derived from M13 by inserting an *amp*^{R} gene into the intergenic region; phage BPTI-III MA is derived from phage MA by inserting *bpti* into *III* between the signal peptide and mature III encoding regions. BPTI-III MK and BPTI-III MA display BPTI fused to the amino terminus of the gene III protein, about five copies per virion.

Fifty µl of BPTI-III MK phage (3.7·10¹¹ pfu/ml) in either 50 mM Tris, pH 7.5, 150 mM NaCl, 1.0 mg/ml BSA (TBS/BSA) buffer or 50 mM sodium citrate, pH 6.5, 150 mM NaCl, 1.0 mg/ml BSA (CBS/BSA) buffer were added to 10 µl of a 25% slurry of immobilized trypsin (Pierce Chemical Co., Rockford, IL) also in TBS/BSA or CBS/BSA. As a control, 50 µl MK phage (9.3·10¹² pfu/ml) were added to 10 µl of a 25% slurry of immobilized trypsin in either TBS/BSA or CBS/BSA buffer. The infectivity of BPTI-III MK phage is 25-fold lower than that of MK phage; thus the conditions chosen above ensure that an approximately equivalent number of phage particles are added to the trypsin beads. After 3 hours of mixing on a Labquake shaker (Labindustries Inc., Berkeley, CA) 0.5 ml of either TBS/BSA or CBS/BSA was added where appropriate to the samples. Beads were washed for 5 min and recovered by centrifugation for 30 sec. The supernatant was removed and 0.5 ml of TBS/0.1% Tween-20 was added. The beads were mixed for 5 minutes on the shaker and recovered by centrifugation. The supernatant was removed and the beads were washed an additional five times with TBS/0.1% Tween-20 as described above. Finally, the beads were resuspended in 0.5 ml of elution buffer (0.1 M HCl containing 1.0 mg/ml BSA adjusted to pH 2.2 with glycine), mixed for 5 minutes and recovered by centrifugation. The supernatant fraction was removed and neutralized by the addition of 130 µl of 1 M Tris, pH 8.0. Aliquots of the neutralized eluate were diluted in LB broth and titered for plaque-forming units.

A significant percentage of the input BPTI-III MK phage bound to immobilized trypsin and was recovered by washing with elution buffer. The amount of fusion phage which bound to the beads was greater in TBS buffer (pH 7.5) than in CBS buffer (pH 6.5). This is consistent with the observation that the affinity of BPTI for trypsin is greater at pH 7.5 than at pH 6.5 (VINC72, VINC74). A much lower percentage of the MK control phage (no displayed BPTI) bound to immobilized trypsin and this binding was independent of pH. At pH 6.5, 1675 times more of the BPTI-III MK phage than of the MK phage bound to trypsin beads while at pH 7.5, a 2103-fold difference was observed. Hence fusion phage displaying BPTI adhere to active trypsin beads and can be recovered as infectious phage.

### Generation of P1 Mutants of BPTI

To demonstrate the specificity of interaction of BPTI-III fusion phage with immobilized serine proteases, single amino acid substitutions were introduced at the P1 position (residue 15 of BPTI) of the BPTI-III fusion protein. The K15L alteration is desired because BPTI(K15L) is a moderately good inhibitor of human neutrophil elastase (HNE) (K_{d} = 2.9·10⁻⁹ M) (BECK88b) and a poor inhibitor of trypsin. Fusion phage displaying BPTI(K15L) bind to immobilized HNE but not to immobilized trypsin. BPTI-III MK fusion phage display the opposite phenotype (bind to trypsin, fail to bind to HNE). These observations illustrate the binding specificity of BPTI-III fusion phage for immobilized serine proteases.

### Characterization of the Affinity of BPTI-III MK and BPTI(K15L)-III MA Phage for Immobilized Trypsin and Human Neutrophil Elastase

Thirty µl of BPTI-III MK phage in TBS/BSA (1.7·10¹¹ pfu/ml) was added to 5 µl of a 50% slurry of either immobilized human neutrophil elastase or immobilized trypsin (Pierce Chemical Co.) also in TBS/BSA. Similarly, 30 µl of BPTI(K15L)-III MA phage in TBS/BSA (3.2·10¹⁰ pfu/ml) was added to either immobilized HNE or trypsin. Samples were mixed on a Labquake shaker for 3 hours. The beads were washed with 0.5 ml of TBS/BSA for 5 minutes and recovered by centrifugation. The supernatant was removed and the beads were washed 5 times with 0.5 ml of TBS/0.1% Tween-20. Finally, the beads were resuspended in 0.5 ml of elution buffer (0.1 M HCl containing 1.0 mg/ml BSA adjusted to pH 2.2 with glycine), mixed for 5 minutes and recovered by centrifugation. The supernatant fraction was removed, neutralized with 130 µl of 1 M Tris, pH 8.0, diluted in LB broth, and titered for plaque-forming units.

### Effect of pH on the Dissociation of Bound BPTI-III MK and BPTI(K15L)-III MA Phage from Immobilized Neutrophil Elastase

The affinity of a given fusion phage for an immobilized serine protease can be characterized on the basis of the amount of bound fusion phage which elutes from the beads by washing with a step gradient that goes, for example, from about pH 7.0 to about pH 2.2 in steps of 1 or 0.5 pH units. Since the affinity of the above described BPTI variants for HNE is not high (K_{d} > 1·10⁻⁹ M), we anticipated that fusion phage displaying these variants might dissociate from HNE beads at a pH above 2.2. Furthermore fusion phage might dissociate from HNE beads at a specific pH characteristic of the particular BPTI variant displayed. Low pH buffers providing stringent wash conditions might be required to dissociate fusion phage displaying a BPTI variant with a high affinity for HNE whereas neutral pH conditions might be sufficient to dislodge a fusion phage displaying a BPTI variant with a weak affinity for HNE.

Thirty µl of BPTI(K15L)-III MA phage (1.7·10¹⁰ pfu/ml in TBS/BSA) were added to 5 µl of a 50% slurry of HNE beads also in TBS/BSA. Similarly, 30 µl of BPTI-III MA phage (8.6·10¹⁰ pfu/ml in TBS/BSA) were added to 5 µl of HNE beads. Thus, an approximately equivalent number of phage particles were added to the beads. Samples were incubated for 3 hours with shaking. The beads were washed with 0.5 ml of TBS/BSA for 5 min with shaking, recovered by centrifugation, and the supernatant removed. The beads were washed with 0.5 ml of TBS/0.1% Tween-20 for 5 minutes and recovered by centrifugation. Four additional washes with TBS/0.1% Tween-20 were performed. The beads were washed with 0.5 ml of 100 mM sodium citrate, pH 7.0 containing 1.0 mg/ml BSA. The beads were recovered by centrifugation and the supernatant was removed. The HNE beads were washed sequentially with a series of 100 mM sodium citrate, 1.0 mg/ml BSA buffers of pH 6.0, 5.0, 4.0 and 3.0 and finally with the 2.2 elution buffer. The pH washes were neutralized by the addition of 1 M Tris, pH 8.0, diluted in LB broth and titered for plaque-forming units.

Table 203 illustrates that a low percentage of the input BPTI-III MK fusion phage adhered to the HNE beads and was recovered in the pH 7.0 and 6.0 washes predominantly. A significantly higher percentage of the BPTI(K15L)-III MA phage bound to the HNE beads and was recovered predominantly in the pH 5.0 and 4.0 washes. Hence lower pH conditions (*i.e*. more stringent) are required to dissociate BPTI(K15L)-III MA than BPTI-MK phage from immobilized HNE. The affinity of BPTI(K15L) is over 1000 times greater than that of BPTI for HNE (using reported K_{d} values (BECK88b)). Hence this suggests that lower pH conditions are required to dissociate fusion phage displaying a BPTI variant with a higher affinity for HNE.

### Effect of Mutation of Residues 39-42 of BPTI(K15L) on its Affinity for Immobilized HNE

Thirty µl of BPTI(K15L,MGNG)-III MA phage (9.2·10⁹ pfu/ml in TBS/BSA) were added to 5 µl of a 50% slurry of immobilized HNE also in TBS/BSA. Similarly 30 µl of BPTI(K15L)-III MA phage (1.2·10¹⁰ pfu/ml in TBS/BSA) were added to immobilized HNE. The samples were incubated for 3 hours with shaking. Beads were washed for 5 min with 0.5 ml TBS/BSA and spun down. The beads were washed 5 times with 0.5 ml TBS/0.1% Tween-20. Finally, the beads were washed sequentially with a series of 100 mM sodium citrate buffers of pH 7.0, 6.0, 5.5, 5.0, 4.75, 4.5, 4.25, 4.0 and 3.5. pH washes were neutralized, diluted in LB broth and titered for plaque-forming units.

Almost twice as much of BPTI(K15L,MGNG)-III MA as BPTI(K15L)-III MA phage bound to HNE beads. In both cases the pH 4.75 fraction contained the largest proportion of recovered phage confirming that replacement of residues 39-42 of wild type BPTI with M₃₉GNG from BI-8e enhances the binding of the BPTI(K15L) variant to HNE.

### Construction of BPTI(K15V,R17L)-III MA

BPTI(K15V,R17L) demonstrates the highest affinity for HNE of any BPTI variant yet described (K_{d} = 6·10⁻¹¹ M) (AUER89). To test the elution system, a phage displaying this BPTI(K15V,R17L) was generated and used as a reference phage to characterize the affinity for immobilized HNE of fusion phage displaying a BPTI variant with a known affinity for free HNE.

### Affinity of BPTI(K15V,R17L)-III MA Phage for Immobilized HNE

Forty µl of BPTI(K15,R17L)-III MA phage (9.8·10¹⁰ pfu/ml) in TBS/BSA were added to 10 µl of a 50% slurry of immobilized HNE also in TBS/BSA. Similarly, 40 µl of BPTI(K15L,MGNG)-III MA phage (5.13·10⁹ pfu/ml) in TBS/BSA were added to immobilized HNE. The samples were shaken for 1.5 hours. Beads were washed once for 5 min with 0.5 ml of TBS/BSA and then 5 times with 0.5 ml of TBS/1.0% Tween-20. The beads were then washed sequentially with a series of 50 mM sodium citrate buffers containing 150 mM NaCl, 1.0 mg/ml BSA of pH 7.0, 6.0, 5.0, 4.5, 4.0, 3.75, 3.5 and 3.0. For BPTI(R15L,MGNG)-III MA, the pH 3.75 and 3.0 washes were omitted. Two washes were performed at each pH and the supernatants pooled, neutralized with 1 M Tris pH 8.0, diluted in LB broth, and titered for plaque-forming units.

The pH 4.5 and 4.0 fractions contained the largest proportion of the recovered BPTI(K15V,R17L)-III MA phage. BPTI(K15L,MGNG)-III MA phage, like BPTI(K15L)-III MA phage, were recovered predominantly in the pH 5.0 and 4.5 fractions, as above. The affinity of BPTI(K15V,R17L) is 48 times greater than that of BPTI(K15L) for HNE (using K_{d} values, AUER89 for BPTI(K15V,R17L) and BECK88b for BPTI(K15L)). That the pH elution profile for BPTI(K15V,R17L)-III MA phage exhibits a peak at pH 4.0 while the profile for BPTI(K15L)-III MA phage displays a peak at pH 4.5 supports the contention that lower pH conditions are required to dissociate, from immobilized HNE, fusion phage displaying a BPTI variant with a higher affinity for free HNE.

### EXAMPLE II

### BPTI Derivatives having high affinity for hNE

We caused BPTI mutants to appear on the surface of M13-derived display phage as amino-terminal fusions to the gene *III* protein (gIIIp) ; M13 has about five copies of gIIIp per virion. Our phage library theoretically included the 1728 BPTI mutants with PHE, LEU, ILE, VAL or MET at positions 15 and 17, GLY or ALA at position 16, PHE, SER, THR or ILE at position 18 and SER, PRO, THR, LYS or GLN at position 19, as a result of expression of a BPTI gene (coding for the aforementioned MGNG mutation) subjected to controlled random mutagenesis, and screened for hNE-binding activity by incubating phage bearing the mutants with immobilized hNE, and eluting the phage with progressively more acidic buffers. Twenty mutants (see clonal identifiers in Tables 207-208) were selected for sequencing, and exhibited eight unique sequences. Tables 207 and 208 show the sequences of nine (the eight, plus another identified in a pilot study) BPTI derivatives having high affinity for hNE. EpiNE1, EpiNE3, EpiNE5, EpiNE6 and EpiNE7 eluted at pH 3.5; EpiNE2, EpiNE4, and EpiNE8 at pH 3.5-4.

That pH conditions less than 4.0 are required to elute EpiNE1, EpiNE3, and EpiNE7-bearing phage from immobilized HNE suggests that they display BPTI variants having a higher affinity for HNE than BPTI(K15V,R17L).

EpiNE1, EpiNE3 and EpiNE7 were expressed as soluble proteins and analyzed for HNE inhibition activity by the fluorometric assay of Castillo et al. (CAST79) ; the data were analyzed by the method of Green and Work (GREE53). EpiNE1, EpiNE3, and EpiNE7 have been produced as free proteins, both in *E. coli* and in yeast. The ability of these proteins to inhibit hNE was measured by following the cleavage of a fluorogenic substrate. The Kᵢ for these compounds is 1 pM, 3 pM, and 3 pM. Phage that display EpiNE1 are used to establish a reference pH-elution profile to allow quick characterization of other KuDom inhibitors displayed on phage. All of the listed EpiNEs have lower K_{d}s than BPRI(K15V,R17L) (60 pM).

An examination of the sequences of the EpiNE clones is illuminating. A strong preference for either VAL or ILE at the P1 position (residue 15) is indicated with VAL being favored over ILE by 14 to 6. No examples of LEU, PHE, or MET at the P1 position were observed although the screened library theoretically should have included mutants with these amino acids at P1. This is consistent with the observation that BPTI variants with single amino acid substitutions of LEU, PHE, or MET for LYS₁₅ exhibit a significantly lower affinity for HNE than their counterparts containing either VAL or ILE (BECK88b).

PHE is strongly favored at position 17, appearing in 12 of 20 clones. MET is the second most prominent residue at this position but it only appears when VAL is present at position 15. At position 18 PHE was observed in all 20 clones sequenced even though the library should have included other residues at this position. This result is quite surprising and could not be predicted from previous mutational analysis of BPTI, model building, or on any theoretical grounds. We infer that the presence of PHE at position 18 significantly enhances the ability each of the EpiNEs to bind to HNE. Finally at position 19, PRO appears in 10 of 20 codons while SER, the second most prominent residue, appears at 6 of 20 codons. Of the residues targeted for mutagenesis in the present study, residue 19 is the nearest to the edge of the interaction surface of an inhibitor with HNE. Nevertheless, a preponderance of PRO is observed and may indicate that PRO at 19, like PHE at 18, enhances the binding of these proteins to HNE. Interestingly, EpiNE5 appears only once and differs from EpiNE1 only at position 19; similarly, EpiNE6 differs from EpiNE3 only at position 19. These alterations may have only a minor effect on the ability of these proteins to interact with HNE. This is supported by the fact that the pH elution profiles for EpiNE5 and EpiNE6 are very similar to those of EpiNE1 and EpiNE3 respectively.

Only EpiNE2 and EpiNE8 exhibit pH profiles which differ from those of the other selected clones. Both clones contain LYS at position 19 which may restrict the interaction of BPTI with HNE. However, we can not exclude the possibility that other alterations within EpiNE2 and EpiNE8 (R15L and Y21S respectively) influence their affinity for HNE.

Position 18 has not previously been identified as a key position in determining specificity or affinity of aprotinin homologues or derivatives for particular serine proteases. None have reported or suggested that phenylalanine at position 18 will confer specificity and high affinity for HNE.

### EXAMPLE III

### BPTI Derivatives having high affinity for hCG

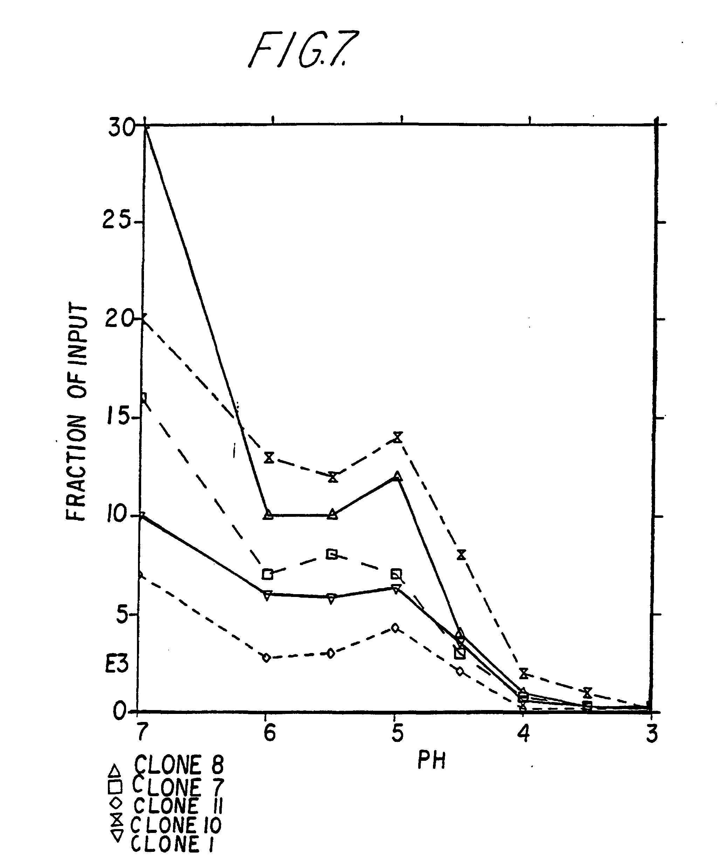

The same library of BPTI mutant-bearing phage was also screened for Cathepsin G binding activity. Figure 7 shows the binding and pH profiles for the individual Cat G binding clones (designated EpiC variants). All clones exhibited minor peaks, superimposed upon a gradual fall in bound phage, at pH elutions of 5 (clones 1, 8, 10 and 11) or pH 4.5 (clone 7). Table 209 reports clones that show binding to Cat G beads.

A comparison of the pH profiles elicited for the Epic variants with Cat G and the EpiNE variants for hNE indicates that the EpiNE variants have a high affinity for hNE while the Epic variants have a moderate affinity for Cat G.

The P1 residue in the Epic mutants is predominantly MET, with one example of PHE, while in BPTI it is LYS and in the EpiNE variants it is either VAL or LEU. In the Epic mutants residue 16 is predominantly ALA with one example of GLY and residue 17 is PHE, ILE or LEU. Interestingly residues 16 and 17 appear to pair off by complementary size, at least in this small sample. The small GLY residue pairs with the bulky PHE while the relatively larger ALA residue pairs with the less bulky LEU and ILE. The majority of the available residues in the MYMUT library for positions 18 and 19 are represented in the Epic variants.

### EXAMPLE IV

### ITI:D1 Derivatives having high affinity for hNE

### Construction of the display vector.

We use the nucleic-acid numbering of the *ITI-light-chain* gene found in TRAB86 and the amino-acid numbering shown for UTI in Fig. 1 of GEBH86. We manipulated DNA according to standard methods as described in SAMB89 and AUSU87.

The protein sequence of human ITI-D1 consists of 56 amino acid residues extending from LYS₂₂ to ARG₇₇ of the complete ITI light chain sequence. This sequence is encoded by the 168 bases between positions 750 and 917 in the cDNA sequence presented in TRAB86. DNA encoding this amino-acid sequence was introduced into M13 gene *iii* by standard means. Phage isolates containing the ITI-D1-III fusion gene are called MA-ITI and carry an *amp*^{R} gene. Expression of the ITI-D1::III fusion protein and its display on the phage surface were demonstrated by Western analysis and phage-titer neutralization experiments with rabbit anti(hITI) serum.

### Fractionation of MA-ITI phage bound to agarose-immobilized protease beads.

To test if phage displaying the ITI-D1-III fusion protein interact strongly with the proteases human neutrophil elastase (hNE) or cathepsin-G, aliquots of display phage were incubated with agarose-immobilized hNE or cathepsin-G beads (hNE beads or Cat-G beads, respectively). The beads were washed and bound phage eluted by pH fractionation. The procession in lowering pH was: pH 7.0, 6.0, 5.5, 5.0, 4.5, 4.0, 3.5, 3.0, 2.5, and 2.0. Following elution and neutralization, the various input, wash, and pH elution fractions were titered.

The results of several fractionations are summarized in Table 212 (EpiNE-7 or MA-ITI phage bound to hNE beads) and Table 213 (Epic-10 or MA-ITI phage bound to Cat-G beads). For the two types of beads (hNE or Cat-G), the pH elution profiles obtained using the control display phage (EpiNE-7 or EpiC-10, respectively) were similar to those seen previously. About 0.3% of the EpiNE-7 display phage applied to the hNE beads were eluted during the fractionation procedure and the elution profile had a maximum for elution at about pH 4.0. A smaller fraction, 0.02%, of the EpiC-10 phage applied to the Cat-G beads were eluted and the elution profile displayed a maximum near pH 5.5.

The MA-ITI phage show no evidence of great affinity for either hNE or cathepsin-G immobilized on agarose beads. The pH elution profiles for MA-ITI phage bound to hNE or Cat-G beads show essentially monotonic decreases in phage recovered with decreasing pH . Further, the total fractions of the phage applied to the beads that were recovered during the fractionation procedures were quite low: 0.002% from hNE beads and 0.003% from Cat-G beads.

Published values of Kᵢ for inhibition neutrophil elastase by the intact, large (Mᵣ=240,000) ITI protein range between 60 and 150 nM and values between 20 and 6000 nM have been reported for the inhibition of Cathepsin G by ITI (SWAI88, ODOM90). Our own measurements of pH fraction of display phage bound to hNE beads show that phage displaying proteins with low affinity (>µM) for hNE are not bound by the beads while phage displaying proteins with greater affinity (nM) bind to the beads and are eluted at about pH 5. If the first KuDom of the ITI light chain is entirely responsible for the inhibitory activity of ITI against hNE, and if this domain is correctly displayed on the MA-ITI phage, then it appears that the minimum affinity of an inhibitor for hNE that allows binding and fractionation of display phage on hNE beads is 50 to 100 nM.

### Alteration of the P1 region of ITI-D1.

If ITI-D1 and EpiNE-7 assume the same configuration in solution as BPTI, then these two polypeptides have identical amino acid sequences in both the primary and secondary binding loops with the exception of four residues about the P1 position and at positions 11 and 34. For ITI-D1 the sequence for positions 15 to 20 is (position 15 in ITI-D1 corresponds to position 36 in the UTI sequence of GEBH86):

| Domain | | BPTI position numbers | | | | | | | | hNE Affinity |
|---|---|---|---|---|---|---|---|---|---|---|
| | 11 | 15 | 16 | 17 | 18 | 19 | 20 | 31 | 34 | |
| EpiNE7 | T | V | A | M | F | P | R | Q | V | very high |
| ITI-D1 | A | M | G | M | T | S | R | E | Q | modest |
| | 32 | 36 | 37 | 38 | 39 | 40 | 41 | 52 | 55 | ← ITI positions |

These two proteins differ greatly in their affinities for hNE. To improve the affinity of ITI-D1 for hNE, the EpiNE-7 sequence was incorporated by cassette mutagenesis into the ITI-D1 sequence at positions 15 through 20. Phage containing the ITI-D1-III fusion gene with the EpiNE-7 changes around the P1 position are called MA-ITI-E7.

### Fractionation of MA-ITI-E7 phage.

To test if the changes at positions 15, 16, 18, and 19 of the ITI-D1-III fusion protein influence binding of display phage to hNE beads, abbreviated pH elution profiles were measured. Aliquots of EpiNE-7, MA-ITI, and MA-ITI-E7 display phage were incubated with hNE beads for three hours at room temperature. The beads were washed and phage were eluted as described above, except that only three pH elutions were performed: pH 7.0, 3.5, and 2.0. The results of these elutions are shown in Table 214.

Binding and elution of the EpiNE-7 and MA-ITI display phage were found to be as described. The total fraction of input phages was high (0.4%) for EpiNE-7 phage and low (0.001%) for MA-ITI phage. Further, the EpiNE-7 phage showed maximum phage elution in the pH 3.5 fraction while the MA-ITI phage showed only a monotonic decrease in phage yields with decreasing pH , as seen above.

MA-ITI-E7 phage show increased levels of binding to hNE beads relative to MA-ITI phage. The total fraction of the input phage eluted from the beads is 10-fold greater for both MA-ITI-E7 phage strains than for MA-ITI phage (although still 40-fold lower that EpiNE-7 phage). Further, the pH elution profiles of the MA-ITI-E7 phage strains show maximum elutions in the pH 3.5 fractions, similar to EpiNE-7 phage.

To further define the binding properties of MA-ITI-E7 phage, the extended pH fractionation procedure described previously was performed using phage bound to hNE beads, as shown in Table 215. The pH elution profile of EpiNE-7 display phage is as previously described. In this more resolved pH elution profile, MA-ITI-E7 phage show a broad elution maximum centered around pH 5. Again, the total fraction of MA-ITI-E7 phage obtained on pH elution from hNE beads was about 40-fold less than that obtained using EpiNE-7 display phage.

The pH elution behavior of MA-ITI-E7 phage bound to hNE beads is qualitatively similar to that seen using BPTI[K15L]-III-MA phage. BPTI with the K15L mutation has an affinity for hNE of ≈3.·10⁻⁹ M. Assuming all else remains the same, the pH elution profile for MA-ITI-E7 suggests that the affinity of the free ITI-D1-E7 domain for hNE is in the nM range. Thus, the substitution of the EpiNE-7 sequence in place of the ITI-D1 sequence around the P1 region has produced an apparent 20- to 50-fold increased affinity for hNE (assuming Kᵢ = 60 to 150 nM for ITI-D1).

If EpiNE-7 and ITI-D1-E7 have the same solution structure, these proteins present the identical amino acid sequences to hNE over the interaction surface. Despite this similarity, EpiNE-7 exhibits a roughly 1000-fold greater affinity for hNE than does ITI-D1-E7. This observation highlights the importance of noncontacting secondary residues in modulating interaction strengths.

ITI light chain is glycosylated at SER10 and ASN45 (GEBH86). Removal of the glycosaminoglycan chains has been shown to decrease the affinity of the inhibitor for hNE about 5-fold (SELL87). Another potentially important difference between EpiNE-7 and ITI-D1-E7 is that of net charge. BPTI has charge +6 while EpiNE7 has charge +1 and ITI-D1 has charge -1. Furthermore, the change in charge between these two molecules arises from differences in the central portions of the molecules which neighbors the binding surface. Position 26 is LYS in EpiNE-7 and is THR in ITI-D1-E7, while at position 31 the residues are GLN and GLU, respectively. These sequence changes not only alter the net molecular charge but also place negative charge close to the interaction surface in ITI-D1-E7. It may be that the occurrence of a negative charge at position 31 (not found in any other hNE inhibitors here described) destabilized the inhibitor-protease interaction.

### Preparation of BITI-E7 Phage

We replaced K₁EDS of ITI-D1 with R₁PDF from EpiNE7 to make phage MA-BITI-E7. Phe₄ of BPTI is part of the hydrophobic core of the protein; replacement with serine may alter the stability or dynamic character of ITI-E7 unfavorably. ITI-E7 has a negatively charged Glu at position 2 while EpiNe7 has Pro.

We made the same changes at the putative amino terminus of the ITI-III fusion protein displayed by the phage MA-ITI. These phage are called MA-BITI.

We compared the properties of the ITI-III fusion proteins displayed by phage MA-ITI and MA-BITI using Western analysis. We found no significant differences in apparent size or relative abundance of the fusion proteins produced by either display phage strain. Thus, there are no large differences in the processed forms of either fusion protein displayed on the phage. By extension, there are also no large differences in the processed forms of the gene III fusion proteins displayed by MA-ITI-E7 and MA-EpiNE7. Large changes in protein conformation due to greatly altered processing are therefore not likely to be responsible for the great differences in binding to hNE-beads shown by MA-ITI-E7 and MA-EpiNE7 display phage.

We characterized the binding properties to hNE-beads of MA-BITI and MA-BITI-E7 display phage using the extended pH fractionation procedure described previously, see Table 216. The pH elution profile of MA-EpiNE7 display phage bound to hNE-beads is similar to that previously described. The pH elution profiles for MA-BITI and MA-BITI-E7 show significant differences from the profiles exhibited by MA-ITI and MA-ITI-E7 (*cf.* Tables 212 and 215). In both cases, the alterations at the putative amino terminus of the displayed fusion protein produce a several-fold increase in the fraction of the input display phage eluted from the hNE-beads.

The binding capacity of hNE-beads for display phage varies among preparations of beads and with age for each individual preparation of beads. Thus, one should compare the relative shapes of profiles obtained on beads of substantially the same age and from the same batch. For example, the fraction of MA-EpiNE7 display phage recovered from hNE-beads varies two-fold among the experiments shown in Tables 212, 215, and 216, and from results given elsewhere in the present specification. However, the shapes of the pH elution profiles are quite similar. It is possible to correct approximately for variations in binding capacity of hNE-beads by normalizing display phage yields to the total yield of MA-EpiNE7 phage recovered from the beads in a concurrent elution. When the data shown in Tables 212, 215, and 216 are so normalized, the recoveries of display phage, relative to recovered MA-EpiNE7, are:

| display phage strain | normalized fraction of input |
|---|---|
| MA-ITI | 0.0067 |
| MA-BITI | 0.027 |
| | |
| MA-ITI-E7 | 0.027 |
| MA-BITI-E7 | 0.13 |

Thus, the alterations in the amino terminal sequence of the displayed fusion protein produce a three- to five-fold increase in the fraction of phage eluted from hNE-beads. While the MA-ITI-E7 elute with a broad pH maximum centered around pH 5.0, the pH elution profile for MA-BITI-E7 phage has a pH maximum at around pH 4.75 to pH 4.5.

The pH elution maximum of the MA-BITI-E7 display phage is located between the maxima exhibited by the BPTI(K15L) and BPTI(K15V, R17L) display phage ( pH 4.75 and pH 4.5 to pH 4.0, respectively) described previously (Example III). From the pH maximum exhibited by the display phage we estimate that the BITI-E7 protein free in solution has an affinity for hNE in the 10⁻¹⁰ M range. This would represent an approximately ten-fold increase in affinity for hNE over that estimated above for ITI-E7.

As described above, Western analysis of phage proteins show that there are no large changes in gene III fusion proteins upon alteration of the amino terminal sequence. Thus, it is unlikely that the changes in affinity of display phage for hNE-beads can be attributed to large-scale alterations in protein folding resulting from altered ("correct") processing of the fusion protein in the amino terminal mutants. The improvements in binding may in part be due to: 1) the decrease in the net negative charge (-1 to 0) on the protein arising from the GLU to PRO change at position 2, or 2) increased protein stability resulting from the SER to PHE substitution at residue 4 in the hydrophobic core of the protein, or 3) the combined effects of both substitutions.

### Production and properties of MA-BITI-E7-1222 and MA-BITI-E7-141

Within the presumed KuDom:hNE interface, BITI-E7 and EpiNE7 differ at only two positions: 11 and 34. In EpiNE7 these residues are THR and VAL, respectively. In BITI-E7 they are ALA and GLN. In addition BITI-E7 has GLU at 31 while EpiNE7 has GLN. This negative charge may influence binding although the residue is not directly in the interface. We used oligonucleotide-directed mutagenesis to investigate the effects of substitutions at positions 11, 31 and 34 on the protease:inhibitor interaction.

Phage MA-BITI-E7-1222 is the same as BITI-E7 with the mutation A11T. Phage MA-BITI-E7-141 is the same as BITI-E7 with the mutations E31Q and Q34V.

We determined the binding properties to hNE-beads of MA-BITI-E7-1222 and MA-BITI-E7-141 display phage using the extended pH fractionation protocol, as shown in Tables 217 (for MA-BITI-E7 and MA-BITI-E7-1222) and 218 (for MA-EpiNE7 and MA-BITI-E7-141).

Thus, the substitution of THR for ALA at position 11 in the displayed ITI derivative has no appreciable effect on the binding of display phage to hNE-beads.

In contrast, the changes at positions 31 and 34 profoundly affect the hNE-binding properties of the display phage (Table 218). The elution profile pH maximum of MA-BITI-E7-141 phage is shifted to lower pH relative to the parental MA-BITI-E7 phage. Further, the position of the maximum (between pH 4.5 and pH 4.0) is identical to that exhibited by MA-EpiNE7 phage in this experiment. Finally, the MA-BITI-E7-141 phage show a ten-fold increase, relative to the parental MA-BITI-E7, in the total fraction of input phage eluted from hNE-beads (0.3% vs 0.03%). Indeed, the total fraction of MA-BITI-E7-141 phage eluted from the hNE-beads is nearly twice that of MA-EpiNE7 phage.

The results discussed above show that binding by MA-BITI-E7-141 display phage to hNE-beads is comparable to that of MA-EpiNE7 phage. Thus, BITI-E7-141 may have K_{D} < 1 pM. Such an affinity is approximately 100-fold greater than that estimated above for the parent (BITI-E7) and is 10⁵ to 10⁶ times as great as the affinity for hNE reported for the intact ITI protein.

### Mutagenesis of BITI-E7-141

BITI-E7-141 differs from ITI-D1 at nine positions (1, 2, 4, 15, 16, 18, 19, 31, and 34). To obtain the protein having the fewest changes from ITI-D1 while retaining high specific affinity for hNE, we have investigated the effects of reversing the changes at positions 1, 2, 4, 16, 19, 31, and 34. The changes we have introduced into the BITI-E7-141 protein are introduced schematically below:

| residue | | | |
|---|---|---|---|
| Displayed | | 1 1 1 1 1 1 | 2 3 3 |
| Protein | 1 2 3 4 .... | 1 .... 5 6 7 8 9 .. | 6 .. 1 .. 4 |
| ITI-D1 | K E D S .... | A .... M G M T S .. | T .. E .. Q |
| 141 | R P D F .... | A .... V A M F P .. | T .. Q .. V |
| | | | |
| MUT1619 | R P D F .... | A .... V G M F S .. | T .. Q .. V |
| MUTP1 | R P D F .... | A .... I G M F S .. | T .. Q .. V |
| | | | |
| AMINO1 | K E D F .... | A .... V A M F P .. | T .. Q .. V |
| AMIN02 | K P D S .... | A .... V A M F P .. | T .. Q .. V |
| | | | |
| MUTQE | R P D F .... | A .... V A K F P .. | T .. E .. V |
| MUTT26A | R P D F .... | A .... V A M F P .. | A .. Q .. V |
| | | | |
| MUT200 | K P D F .... | A .... V G M F S .. | A .. E .. V |

ITI-D1 residues are shown in bold type and residues found in neither ITI-D1 nor in BILTI-E7-141 are shown underlined in bold. MUT1619 restores the ITI-D1 residues at positions 16 and 19. It is likely that MET at 17 and PHE at 18 are optimal for high affinity hNE binding, but F₁₇F₁₈ is also effective. GLY at 16 and SER at 19 occurred frequently in the high affinity hNE-binding BPTI-variants obtained from fractionation of a library of BPTI-variants against hNE (ROBE91). Thus, it seems likely that the ITI-D1 sequence at these positions can be restored while maintaining high specific affinity for hNE. The sequence designated MUT200 is hypothetical, but is very likely to have high affininty for hNE.

The BITI display phage were produced by substituting R₁PDF of EpiNE7 for K₁EDS of ITI phage.

Two changes had been introduced into the sequence for BITI-E7 to produce BITI-E7-141: GLU to GLN at position 31 and GLN to VAL at position 34.

The BITI-E7-141 protein sequence ASN24-GLY25-THR26 matches the general recognition sequence ASN-X-THR/SER for N-linked glycosylation in eukaryotic organisms. In the intact ITI molecule isolated from human serum, the light chain polypeptide is glycosylated at this site (ASN45, ODOM90). It is likely that ASN24 will be glycosylated if the BITI-E7-141 protein is produced via eukaryotic expression. Such glycosylation may render the protein difficult to purify to homogeneity and immunogenic when used for long-term treatment. We changed T₂₆ to A because alanine is found frequently at this locus in KuDoms.

### hNE-binding properties of mutagenized MA-BITI-E7-141 display phage

The binding properties of the individual phage populations to hNE-beads were determined using the abbreviated and extended pH elution protocols described previously. The results of these studies are presented in Table 219.

Table 219 shows pH elution data for the various display phage eluted from hNE-beads. Total pfu applied to the beads are shown in the second column. The fractions of this input pfu recovered in each pH fraction of the abbreviated pH elution protocol (pH 7.0, pH 3.5, and pH 2.0) are listed in the next three columns. For data obtained using the extended pH elution protocol, the pH 3.5 listing represents the sum of the fractions of input recovered in the pH 6.0, pH 5.5, pH 5.0, pH 4.5, pH 4.0, and pH 3.5 elution samples. Likewise, the pH 2.0 listing is the sum of the fractions of input obtained from the pH 3.0, pH 2.5, and pH 2.0 elution samples. The total fraction of the input pfu obtained throughout the pH elution protocol is recorded in the sixth column of Table 219. The final column of the table lists the total fraction of input pfu recovered normalized to the value obtained for MA-BITI-E7-141 display phage.

Two factors must be considered when making comparisons among the data shown in Table 219. The first is that, due to the kinetic nature of phage release from hNE-beads and the longer time involved in the extended pH elution protocol, the fraction of input pfu recovered in the pH 3.5 fraction will be enriched at the expense of the pH 2.0 fraction in the extended protocol relative to those values obtained in the abbreviated protocol. The magnitude of this effect can be seen by comparing the results obtained when MA-BITI-E7-141 display phage were eluted from hNE-beads using the two protocols. The second factor is that, for the range of input pfu listed in Table 219, the input pfu influences recovery. The greater the input pfu, the greater the total fraction of the input recovered in the elution. This effect is apparent when input pfu differ by more than a factor of about 3 to 4. The effect can lead to an overestimate of affinity of display phage for hNE-beads when data from phage applied at higher titers is compared with that from phage applied at lower titers.

Mindful of these caveats, we interpret Table 219. The effects of the mutations introduced into MA-BITI-E7-141 display phage ("parental") on binding of display phage to hNE-beads can be grouped into three categories: those changes that have little or no effects, those that have moderate (2- to 3-fold) effects, and those that have large (>5-fold) effects.

The MUTT26A and MUTQE changes appear to have little effect on the binding of display phage to hNE-beads. In terms of total pfu recovered, the display phage containing these alterations bind as well as the parental to hNE-beads. Indeed, the pH elution profiles obtained for the parental and the MUTT26A display phage from the extended pH elution protocol are indistinguishable. The binding of the MUTQE display phage appears to be slightly reduced relative to the parental and, in light of the applied pfu, it is likely that this binding is somewhat overestimated.

The sequence alterations introduced via the MUTP1 and MUT1619 oligonucleotides appear to reduce display phage binding to hNE-beads about 2- to 3-fold. In light of the input titers and the distributions of pfu recovered among the various elution fractions, it is likely that 1) both of these display phage have lower affinities for hNE-beads than do MA-EpiNE7 display phage, and 2) the MUT1619 display phage have a greater affinity for hNE-beads than do the MUTP1 display phage.

The sequence alterations at the amino terminus of BITI-E7-14 appear to reduce binding by the display phage to hNE-beads at least ten fold. The AMINO2 changes are likely to reduce display phage binding to a substantially greater extent than do the AMINO1 changes.

On the basis of the above interpretations of the data listed in Table 219, we can conclude that:
1.) The substitution of ALA for THR at position 26 in ITI-D1 and its derivatives has no effect on the interaction of the inhibitor with hNE. Thus, the possibility of glycosylation at ASN24 of an inhibitor protein produced in eukaryotic cell culture can be avoided with no reduction in affinity for hNE.
2.) The increase in affinity of display phage for hNE-beads produced by the changes GLU to GLN at position 31 and GLN to VAL at 34 results primarily from the VAL substitution at 34.
3.) All three changes introduced at the amino terminal region of ITI-D1 (positions 1,2, and 4) influence display phage binding to hNE-beads to varying extents. The change at position 4 (SER to PHE) appears to have a much greater effect than does the change at position 2. The change at position 1 may have little or no effect.
4.) The changes in the region around the P1 residue in BITI-E7-141 (position 15) influence display phage binding to hNE. The changes ALA to GLY at 16 and PRO to SER at 19 appear to reduce the affinity of the inhibitor somewhat (perhaps 3-fold). The substitution of ILE for VAL at position 15 further reduces binding.

BITI-E7-141 differs from ITI-D1 at nine positions. On the basis of the discussion above it appears likely that a high affinity hNE-inhibitor based on ITI-D1 could be constructed that would differ from the ITI-D1 sequence at only four or five positions. These differences would be: PHE at position 4, VAL at position 15, PHE at position 18, VAL at position 34, and ALA at position 26. If glycosylation of ASN24 is not a concern THR could be retained at 26.

### 10. Summary: estimated affinities of isolated ITI-D1 derivatives for hNE

On the basis of display phage binding to and elution from hNE beads, it is possible to estimate affinities for hNE that various derivatives of ITI-D1 may display free in solution. These estimates are summarized below and in Table 220.

### CITATIONS

ALBR83a:Albrecht, et al., Hoppe-Seyler's Z Physiol Chem (1983), 364:1697-1702.

ADEY88: Adeyemi and Hodgson, *J Clin Lab Immunol* (1988) **27**(1)1-4.

AFFO88: Afford, et al., *Biol Chem Hoppe-Seyler* (1988) **369**:1065-74.

ALTM91: Altman, et al., *Protein Engineering* (1991) **4**(5)593-600.

ARSE86: Arsenis, et al., *Agents Actions Suppl* (1986) AAS 18(Recent Adv Connect Tissue Res)63-8.

ARSE88: Arsenis, et al., *Current Eye Research* (1988) **7**(2)95-102.

ASCE90: Ascenze, et al., *Biol Chem Hoppe-Seyler* (1990) **371**:389-393.

ALBR83b: Albrecht, et al., Hoppe-Seyler's Z Physiol chem (1983), 364:1703-1708.

ANGE90: Angelastro et al. (1990) J. Med. Chem. **33**:13-16.

ARNA90: Arnaout, in Immunological Reviews (1990), **114**.

AUER87: Auerswald, et al., Biol Chem Hoppe-Seyler (1987), 368:1413-1425.

AUER88:Auerswald, et al., Bio Chem Hoppe-Seyler (1988), 369(Supplement):27-35.

AUER89:Auerswald, et al., UK Patent Application GB 2,208,511 A.

AUER90:Auerswald, et al., US Patent 4,894,436 (16 Jan 1990).

AUSU87:Ausubel, et al., Editors, Current Protocols in Molecular Biology, Greene Publishing Associates and Wiley-Interscience, Publishers: John Wiley & Sons, New York, 1987.

BALD85:Balduyck, et al., Biol Chem Hoppe-Seyler (1985), 366:9-14.

BAND88a:Banda, et al., *J Exp Med* **167**(5)1608-15 (1988)

BAND88b:Banda, et al., *J Biol Chem* **263**(9)4481-4 (1988)

BARR86:Barrett and Salvesen, Editors, Protease Inhibitors, Published by Elsevier, Amsterdam, 1986.

BECK88b:Beckmann, et al., Eur J Biochem (1988), 176:675-82.

BECK89a:Beckmann, et al., J Protein Chem (1989), 8(1)101-113.

BIET86:Bieth, pp. 217-320 in Regulation of Matrix Accumulation, Editor: RP Mecham, Academic Press, Orlando, 1986.

BLOW72:Blow &al., J Mol Biol (1972), 69:137ff.

BARB91 Barbas, et al., *Proc Natl Acad Sci USA* (1991) **88:**7978-82.

BASS90 Bass, et al., *Proteins* (1990) **8:**309-14.

BONN89 Bonney, et al., *J Cell Biochem* (1989) **39**(1)47-53.

BOUD87 Boudier, et al., *Biol Chem Hoppe-Seyler* (1987) **368:**981-990.

BRIN90 Brinkmann and Tschesche, *Biol Chem Hoppe-Seyler* (1990) **371 Suppl**:43-52.

BRIN91 Brinkmann, et al., *Eur J Biochem* (1991) **202**(1)95-9.

BUTT91 Buttle, et al., *Biochem J* (1991) **276**(2)325-31.

CAMP82: Campbell, Senior, McDonald, and Cox, (1982) J Clin Invest **70**:845-52.

CAMP88: Campbell and Campbell (1988) J Cell Biol **106**:667-676.

CAMP90: Campanelli, et al. , J Exp Med (Dec 1990), 172:1709-15.

CANT89:Cantor and Turino, (1989) Chapter 16 in ROBE89.

CHAZ83:Chazin, et al., Eur J Biochem (1985), 152:(2)429-37.

CHAZ85:Chazin, et al., Eur J Biochem (1985), 152:(2)429-37.

COLL90:Collins, et al., (1990) *Biol Chem Hoppe-Seyler* **371 Suppl.** pp.29-36.

CREI74:Creighton (1974) J Mol Biol 87:579-602.

CREI77a:Creighton, J Mol Biol (1977), 113:275-293.

CREI77b:Creighton, J Mol Biol (1977), 113:295-312.

CREI80: Creighton, J Mol Biol (1980), 144:521-550.

CREI84:Creighton, Proteins: Structures and Molecular Principles, W H Freeman & Co, New York, 1984.

### DAVI79:Davis et al, US Patent 4,179,337 (1979)

DIAR90:Diarra-Mehrpour, et al., Eur J Biochem (1990), 191:131-139.

DOHE90 Doherty and Mehdi, *Int J Immunopharmac* (1990) **12**(7)787-795.

DUFT85:Dufton, Eur J Biochem (1985), 153:647-654.

EIGE90:Eigenbrot, Randal, and Kossiakoff, Protein Engineering (1990), 3(7)591-598.

ENGH89:Enghild, Thogersen, Pizzo, and Salvesen, J Biol Biochem (1989), 264:15975-15981.

FERR90:Ferrer-Lopez, et al., American J Physiology (1990) 258:C1100-C1107.

FIOR88:Fioretti, et al., Biol Chem Hoppe-Seyler (1988), 369(Suppl)37-42.

GEBH86:Gebhard, W, and K Hochstrasser, pp.389-401 in BARR86.

GEBH90:Gebhard, et al., Biol Chem Hoppe-Seyler (1990), 371,suppl 13-22.

GIRA89:Girard, et al., Nature (1989), 338:518-20.

GOLD83:Goldenberg, and Creighton, J Mol Biol (1983), 165(2)407-13.

GOLD84:Goldenberg and Creighton (1984) J Mol Biol **179**:527-45.

GOLD86:Goldstein and Doering, (1986) Am Rev Respir Dis **134**:49-56.

GOLD88:Goldenberg, Biochem (1988), 27:2481-89.

GOVH90:Govhardan and Abeles, (1990) Archives Biochem Biophys **280**:137-146.

GUPT90:Gupta, et al., Blood (Nov 15 1990), 76(10)2162.

GREE91 Greenwood, et al., *J Mol Biol* (1991) **220**:821-827.

GROE91 Groeger, et al., *J Protein Chem* (1991) **10**(2)245-251.

HEID86: Heidtmann and Travis, Chapter 14 in ROBE86.

HIEM91:Hiemstra, et al., *Immunobiology* (Stuttgart) **182**(2)117-26 (1991)

HOCH84:Hoschstrasser, and Wachter, US Patent 4,485,100 (27 Nov 1984).

HUBB86:Hubbard and Crystal, Respiration (1986), 50 (Suppl 1)56-73.

HUBE74:Huber, et al., J Mol Biol (1974), 89:73-101.

HUBE75:Huber, et al., Biophys Struct Mechan (1975), 1:189-201.

HUBE77:Huber, et al., Biophys Struct Mech (1975), 1(3)189-201.

HUTC87:Hutchinson, Eur J Respir Dis (1987), 71(Suppl.153)78-85.

HYNE90:Hynes, et al., Biochemistry (1990), 29:10018-10022.

HUBB89a: Hubbard, et al., *Proc Natl Acad Sci USA* (1989) 86:680-4.

HUBB89b: Hubbard, et al., *Annals of Internal Medicine* (1989) 111:206-212.

IMPE86:Imperiali and Abeles, (1986) Biochem **25:**760-67.

IMPE87:Imperiali and Abeles, (1987) Biochem **26:**4474-77.

KAOR88:Kao, et al., J Clin Invest (1988), 82:1963-73.

KAUM86:Kaumerer, et al., Nucleic Acids Res (1986), 14:7839-7850.

KIDO88:Kido, et al., J Biol Chem (1988), 263:18104-7.

KIDO90:Kido, et al., Biochem & Biophys Res Comm (16 Mar 1990), 167(2)716-21.

KITA90 Kitaguchi, et al., *Biochim Biophys Acta* (1990) **1038:**105-113.

LASK80:Laskowski and Kato, Ann Rev Biochem (1980), 49:593-626.

LAZU83:Lazure, et al., Canadian J Biochem Cell Biol (1983), 61:287-92.

MARQ83:Marquart, et al., Acta Cryst, B (1983), 39:480ff.

MCWH89:McWherter, et al., Biochemistry (1989), 28:5708-14.

MEHD90:Mehdi et al. (1990) Biochem Biophys Res Commun **166**:595-600.

NADE87:Nadel, and Borson, Biorheology (1987), 24:541-549.

NADE90:Nadel, 1990 Cystic Fibrosis Meeting, Arlington, Va., p156. and Rubenstein et al, (1990) J Clin Invest **86**:555-9.

NILE89:Niles, et al., Blood (1989), 74(6)1888-93.

NORR89a:Norris and Petersen, European Patent Application 0 339 942 A2.

NORR89b:Norris, et al., PCT patent application WO89/01968.

ODOM90:Odom, Int J Biochem (1990), 22:925-930.

OLTE89:Oltersdorf, et al., Nature (1989), 341:144-7.

PADR89 Padrines, et al., (1989) *Am Rev Respir Dis* **139**(3)783-90 (1989)

PEET90:Peet, et al., (1990) J Med Chem **33**:394-407.

PETE89:Peterson, J Lab Clin Med (1989), 113(3)297-308.

PONT88:Ponte, et al., Nature (1988), 331:525-7.

POWE86:Powers and Harper, pp.55-152 of BARR86.

PADR91:Padrines and Bieth, *Am J Respir Cell Mol Biol* (1991) **4**:187-193.

RITO83:Ritonja, Meloun, and Gubensek, Biochim Biophys Acta (1983), 746:138-145.

ROBE89:Robert and Hornbeck, Editors, Elastin and Elastases, Volume II, CRC Press, Boca Raton, FL. 1989.

RUEH73:Ruehlmann, et al., J Mol Biol (1973), 77:417-436.

SALI90:Salier, TIBS (1990), 15:435-439.

SALV87:Salvesen, et al., Biochem (1987), 26:2289-93.

SAMB89:Sambrook, J, EF Fritsch, and T Maniatis, Molecular Cloning, A Laboratory Manual, Second Edition, Cold Spring Harbor Laboratory, 1989.

SCHE67:Schecter and Berger, *Biochem Biophys Res Common* (1967) **27**:157-162.

SCHN86b:Schnebli and Braun, Chapter 21 in BARR86.

SCHW87:Schwarz, et al., Biochemistry (1987), 26:(12)p3544-51.

SCOT87b:Scott, et al., Blood (1987), 69:1431-6.

SEEM86:Chapter 8 in BARR86.

SELL87:Selloum, et al., Biol Chem Hoppe-Seyler (1987), 368:47-55.

SIEK87:Siekmann, et al., Biol Chem Hoppe-Seyler (1987), 368:1589-96.

SIEK89:Siekmann, et al., Biol Chem Hoppe-Seyler (1989), 370:677-81.

SINH90:Sinha, et al., J Biol Chem (1990), 265(16)8983-5.

SINH91:Sinha et al., (1991) J Biological Chem **266**:21011-13.

SNID91:Snider, et al., (1991) Ann N Y Acad Sci **624:**45-59.

SOMM89:Sommerhoff, et al., J Immunol (1989), 142:2450-56.

SOMM90:Sommerhoff, et al., J Clin Invest (March 1990), 85:682-689.

SOMM91:Sommerhoff *et al.,* (1991) Eur J Pharmacology **193:**153-158.

STAT87:States, et al., J Mol Biol (1987), 195(3)731-9.

STON90:Stone, et al., *Eur Respir J* **3**(6)673-8 (1990)

SWAI88:Swaim and Pizzo, Biochem J (1988), 254:171-178.

TRAB86:Traboni, C, R Cortese, Nucleic Acids Res (1986), 14(15)6340.

TRAV88:Travis, (1988) Am J Med **84**(6A)37-42

TRIB86:Traboni, C, R Cortese, Nucleic Acids Res (1986), 14(15)6340.

TSCH87:Tschesche, et al., Biochimica et Biophysica Acta (1987), 913:97-101.

VINC72:Vincent et al., Biochem (1972), 11:2967ff.

VINC74:Vincent et al., Biochem (1974), 13:4205.

WACH79:Wachter, et al., Hoppe-Seyler Z Physiol Chem (1979), 360:1297-1303.

WACH80: Wachter, et al., FEES Letters (1980), 119:58-62.

WAGN79:Wanger, et al., Eur J Biochem (1979), 95:239-248.

WAGN87:Wagner, et al., J Mol Biol (1987), 196(1)227-31.

WEIS89:Weiss (1989) New Engl J Med **320**:365-76.

WEWE87:Wewers, et al., New Engl J Med (1987), 316(17)1055-62.

WLOD84:Wlodawer, et al., J Mol Biol (1984), 180(2)301-29.

WLOD87a:Wlodawer, et al., J Mol Biol (1987), 198(3)469-80.

WLOD87b:Wlodawer, et al., J Mol Biol (1987), 193(1)145-56.

WUNT88:Wun, et al., J Biol Chem (1988), 263:6001-4.

WELL90 Wells, *Biochem* (1990) **29**(37)8509-17.

WILL91a:Williams, et al., *J Biol Chem* (15 March 1991) **266**(8)5182-90).

WILL91b:Williams, et al., *Experimental Lung-Research* (1991) **17**:725-41.

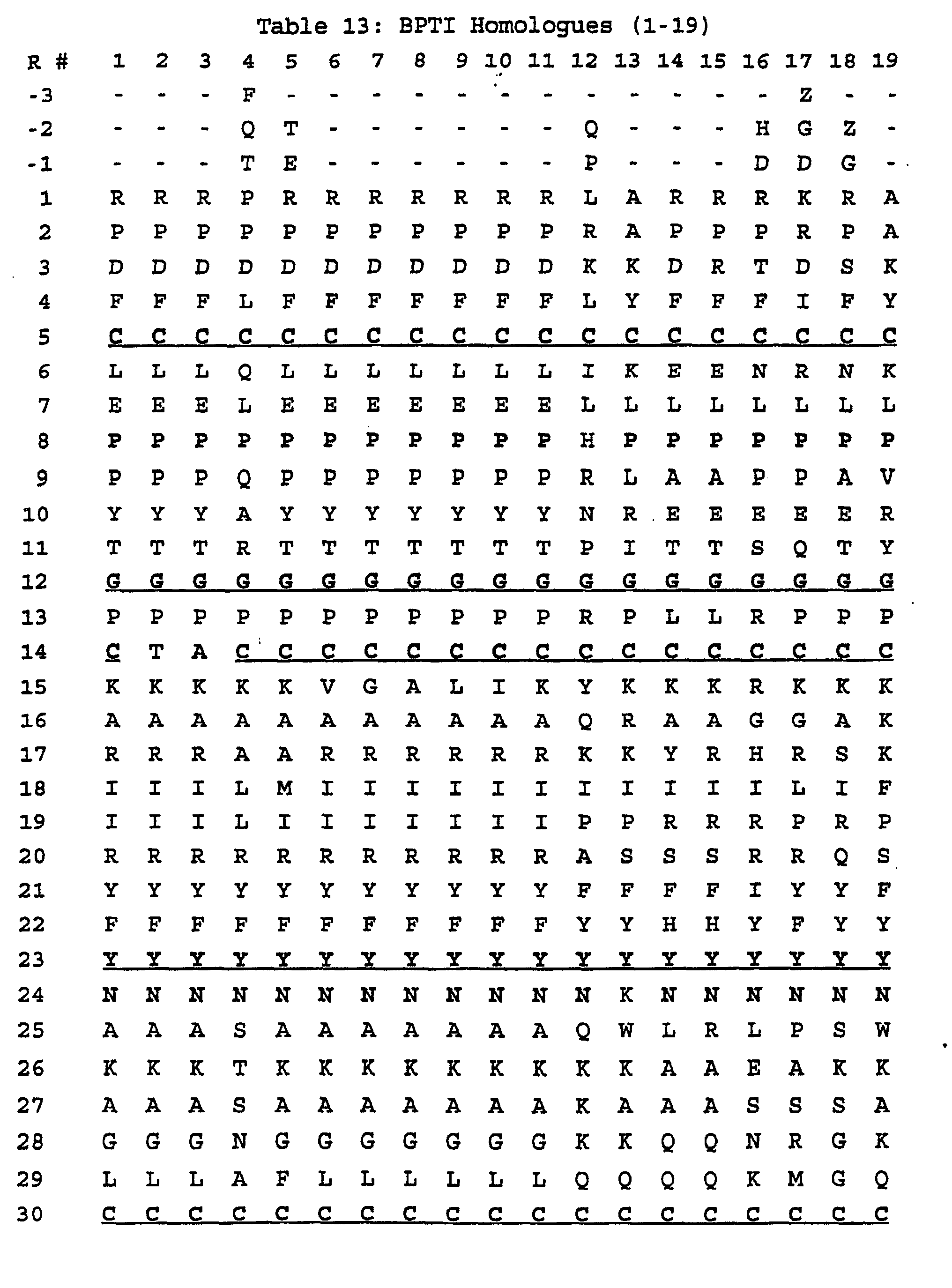

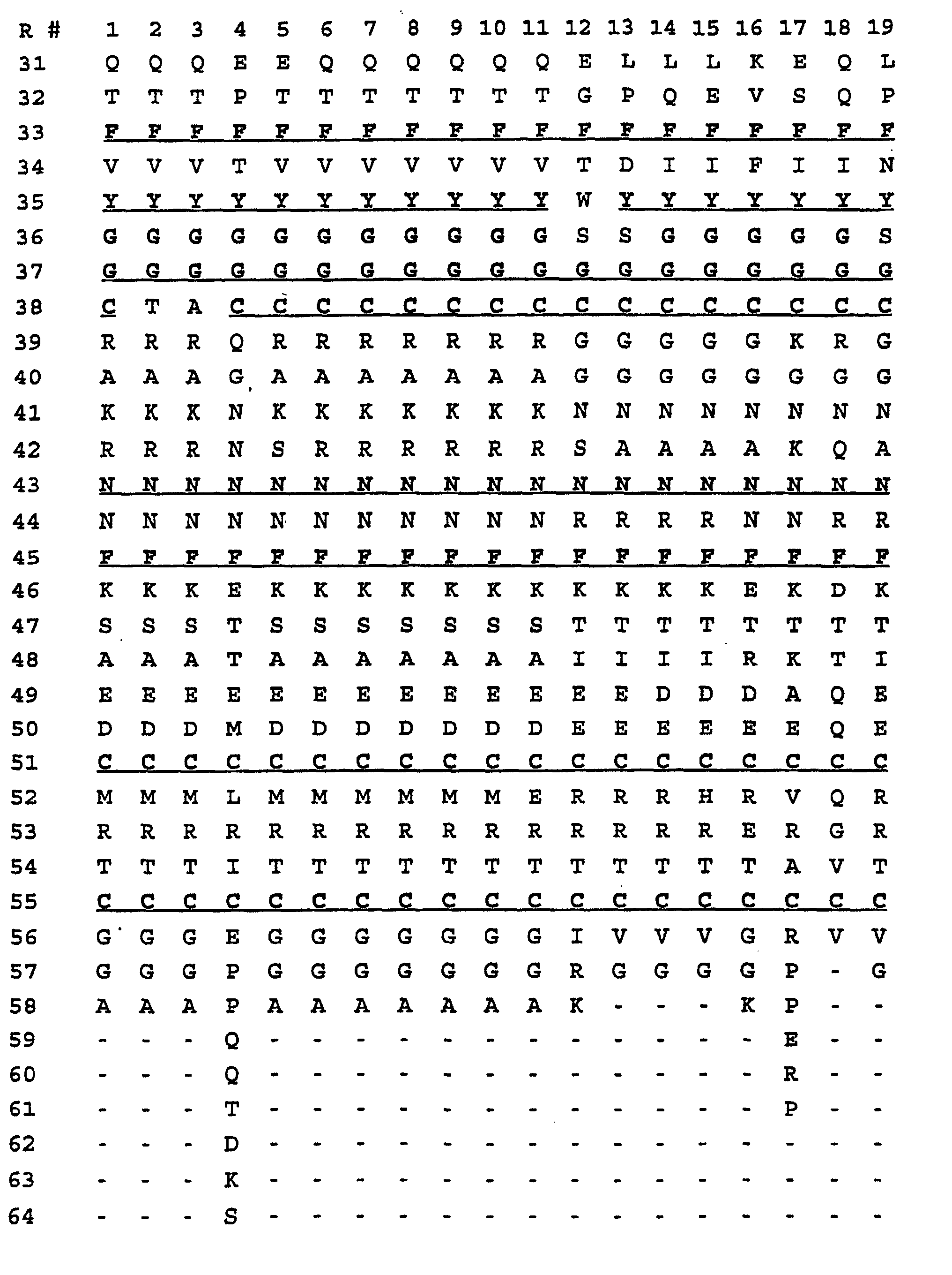

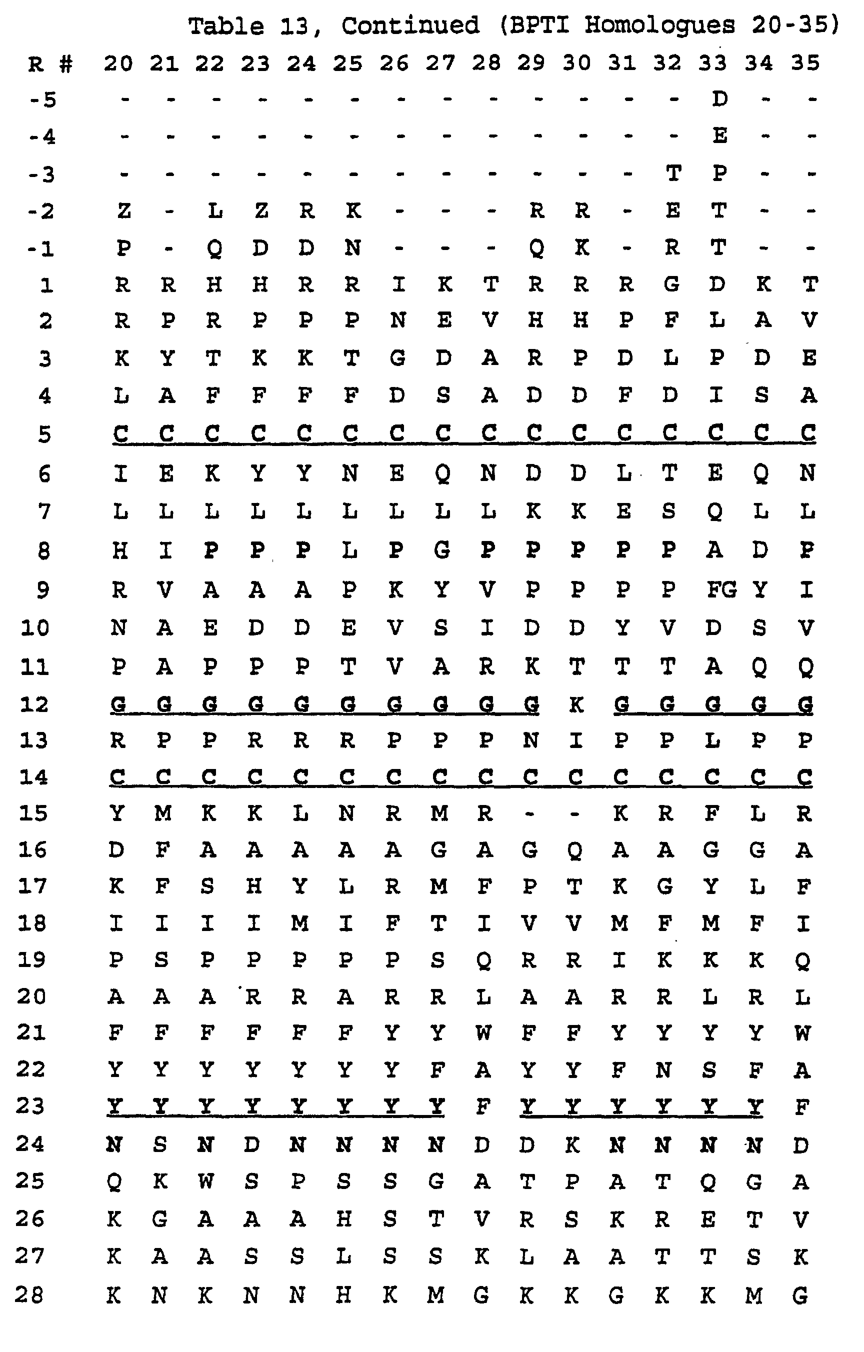

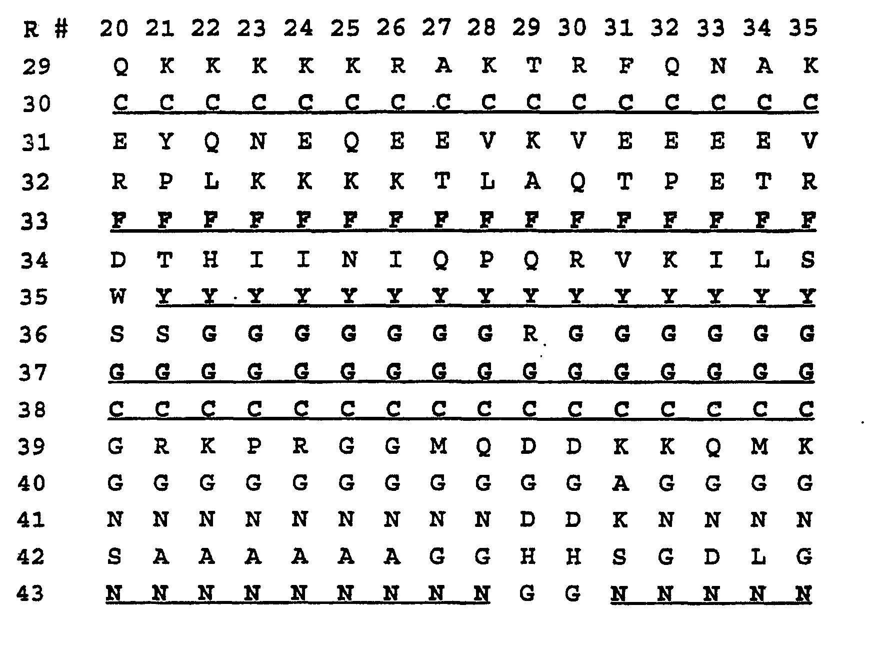

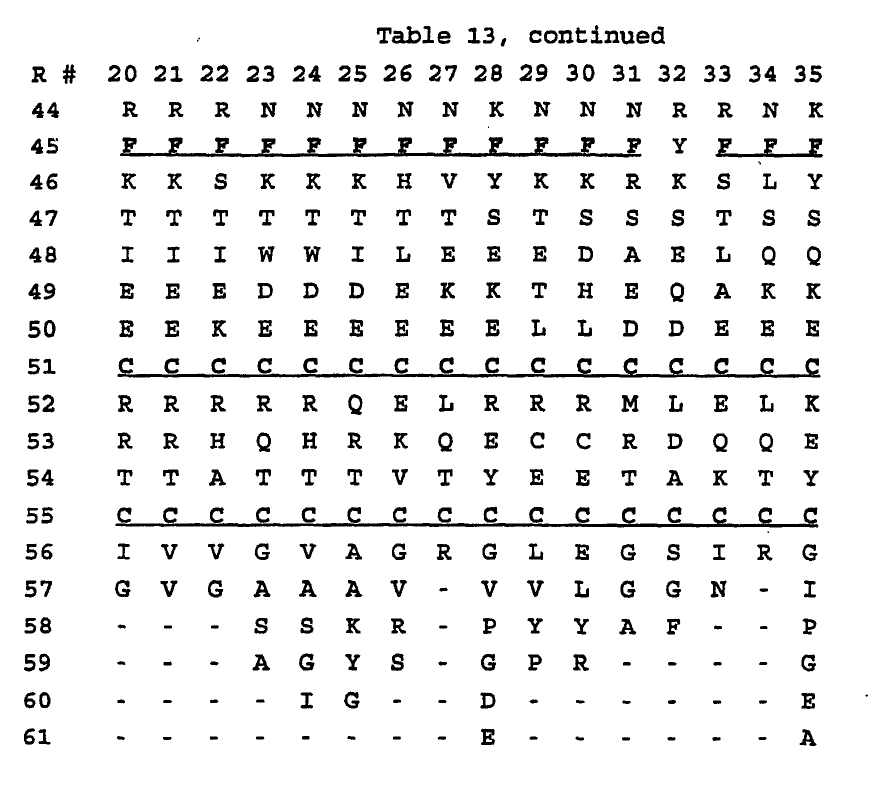

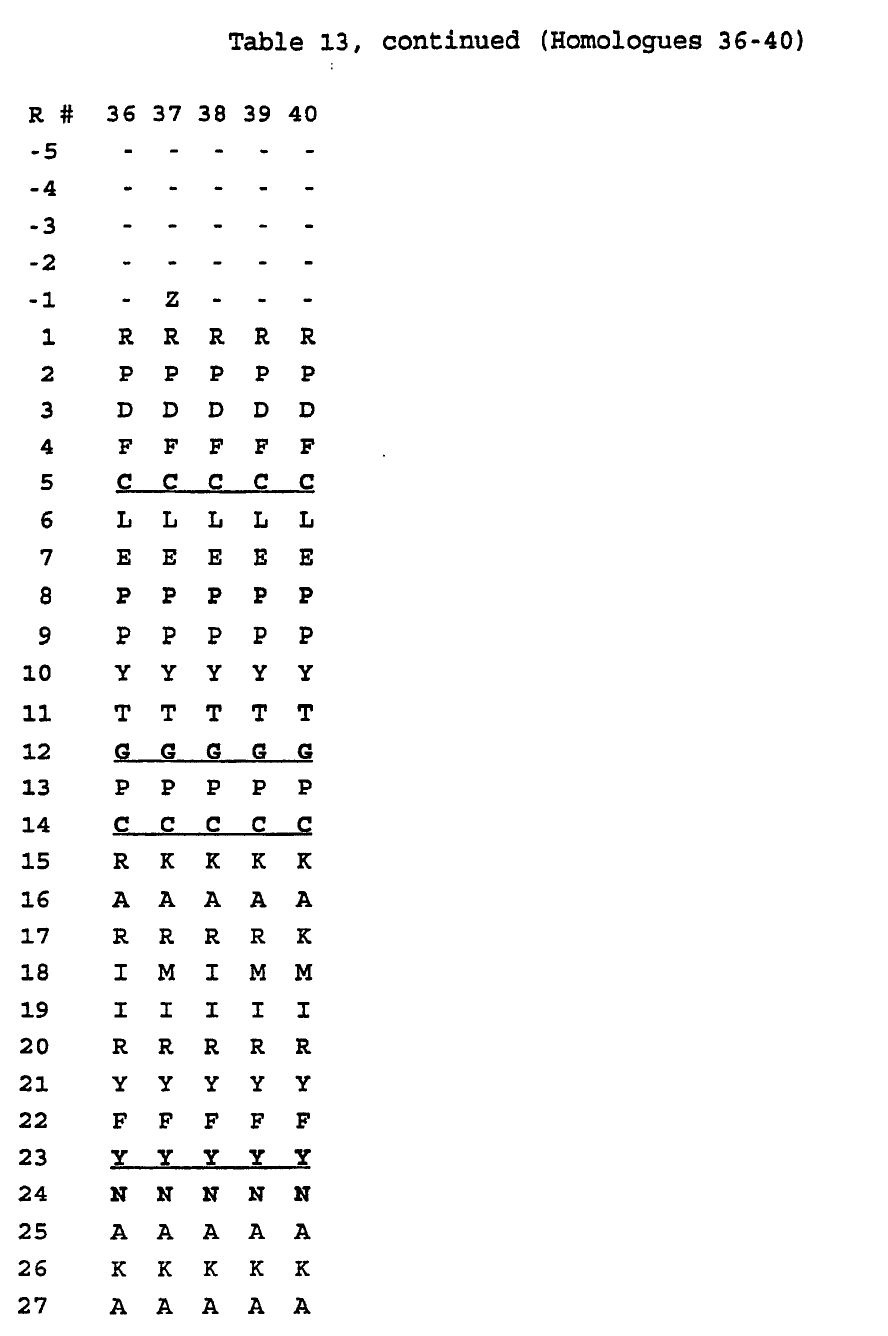

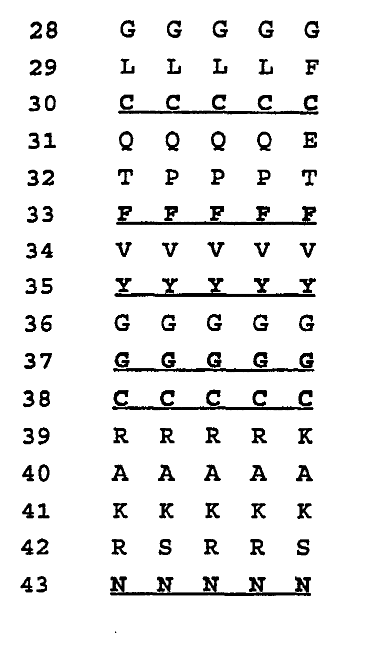

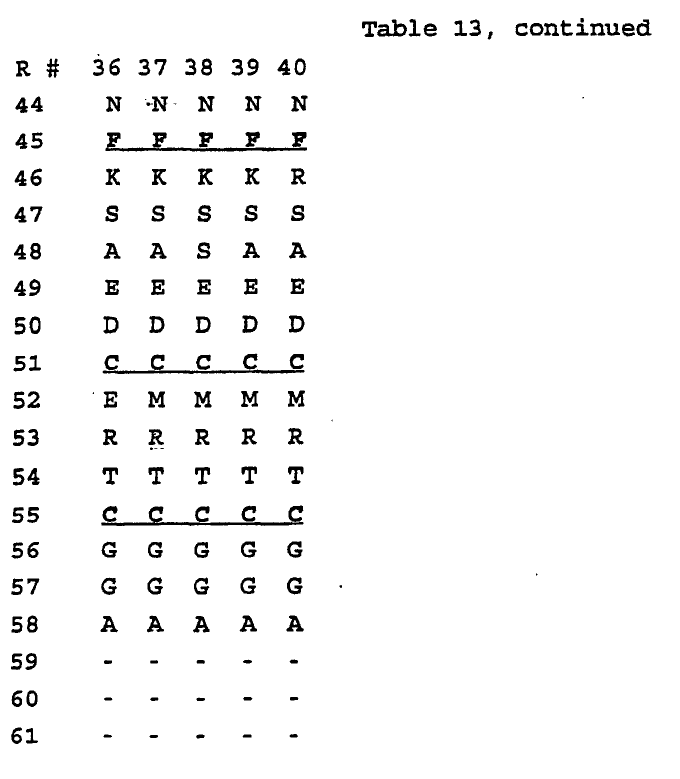

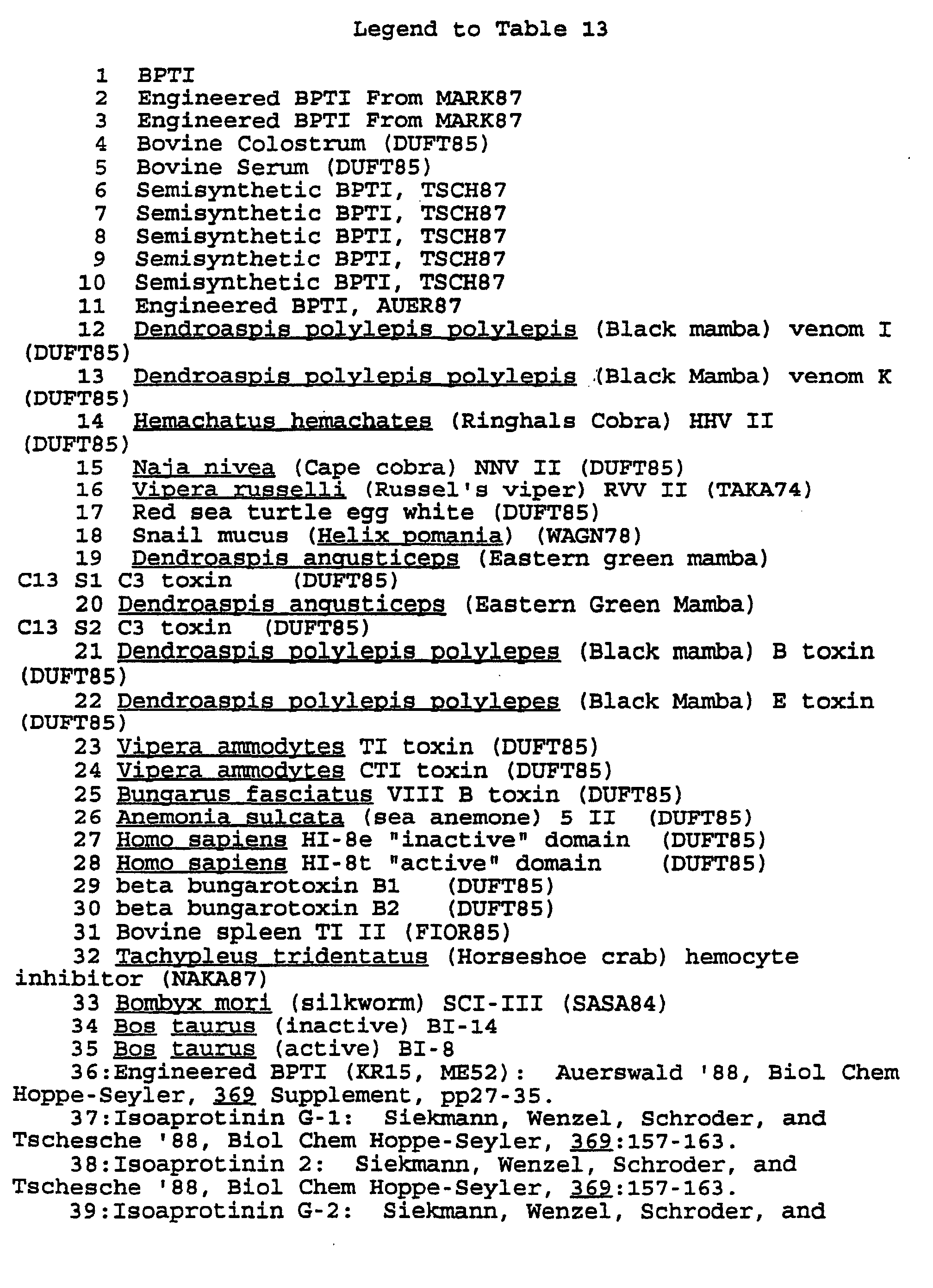

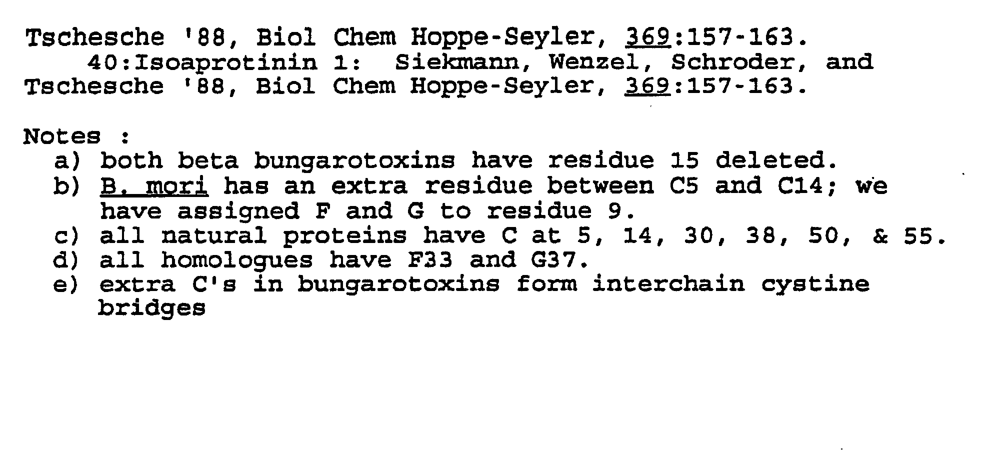

**Table 15:**

| Frequency of Amino Acids at Each Position in BPTI and 58 Homologues | | | |
|---|---|---|---|
| Res. Id. | Different AAs | Contents | First |
| -5 | 2 -58 | D | - |
| -4 | 2 -58 | E | - |
| -3 | 5 -55 | P T Z F | - |
| -2 | 10 -43 | R3 Z3 Q3 T2 E G H K L | - |
| -1 | 11 -41 | D4 P3 R2 T2 Q2 G K N Z E | - |
| 1 | 13 R35 | K6 T4 A3 H2 G2 L M N P I D - | R |
| 2 | 10 P35 | R6 A4 V4 H3 E3 N F I L | P |
| 3 | 11 D32 | K8 S4 A3 T3 R2 E2 P2 G L Y | D |
| 4 | 9 F34 | A6 D4 L4 S4 Y3 I2 W V | F |
| 5 | 1 C59 | | C |
| 6 | 13 L25 | N7 E6 K4 Q4 13 D2 S2 Y2 R F T A | L |
| 7 | 7 L28 | E25 K2 F Q S T | E |
| 8 | 10 P46 | H3 D2 G2 E I K L A Q | P |
| 9 | 12 P30 | A9 I4 V4 R3 Y3 L F Q H E K | P |
| 9a | 2 -58 | G | - |
| 10 | 9 Y24 | E8 D8 V6 R3 S3 A3 N3 I | Y |
| 11 | 11 T31 | Q8 P7 R3 A3 Y2 K S D V I | T |
| 12 | 2 G58 | K | G |
| 13 | 5 P45 | R7 L4 I2 N | P |
| 14 | 3 C57 | A T | C |
| 15 | 12 K22 | R12 L7 V6 Y3 M2 -2 N I A F G | K |
| 16 | 7 A41 | G9 F2 D2 K2 Q2 R | A |
| 17 | 14 R19 | L8 K7 F5 M4 Y4 H2 A2 S2 G2 I N T P | R |
| 18 | 8 I41 | M7 F4 L2 V2 E T A | I |
| 19 | 10 I24 | P12 R8 K5 S4 Q2 L N E T | I |
| 20 | 5 R39 | A8 L6 S5 Q | R |
| 21 | 5 Y35 | F17 W5 I L | Y |
| 22 | 6 F32 | Y18 A5 H2 S N | F |
| 23 | 2 Y52 | F7 | Y |
| 24 | 4 N47 | D8 K3 S | N |
| 25 | 13 A29 | S6 Q4 G4 W4 P3 T2 L2 R N K V I | A |
| 26 | 11 K31 | A9 T5 S3 V3 R2 E2 G H F Q | K |
| 27 | 8 A32 | S11 K5 T4 Q3 L2 I E | A |
| 28 | 7 G32 | K13 N5 M4 Q2 R2 H | G |
| 29 | 10 L22 | K13 Q11 A5 F2 R2 N G M T | L |
| 30 | 2 C58 | A | C |
| 31 | 10 Q25 | E17 L5 V5 K2 N A R I Y | Q |
| 32 | 11 T25 | P11 K4 Q4 L4 R3 E3 G2 S A V | T |
| 33 | 1 F59 | | F |
| 34 | 13 V24 | I10 T5 N3 Q3 D3 K3 F2 H2 R S P L | V |
| 35 | 2 Y56 | W3 | Y |
| 36 | 3 G50 | S8 R | G |
| 37 | 1 G59 | | G |
| 38 | 3 C57 | A T | C |
| 39 | 9 R25 | G13 K6 Q4 E3 M3 L2 D2 P | R |
| 40 | 2 G35 | A24 | A |
| 41 | 3 N33 | K24 D2 | K |
| 42 | 12 R22 | A12 G8 S6 Q2 H2 N2 M D E K L | R |
| 43 | 2 N57 | G2 | N |
| 44 | 3 N40 | R14 K5 | N |
| 45 | 2 F58 | Y | F |
| 46 | 11 K39 | Y5 E4 S2 V2 D2 R H T A L | K |
| 47 | 2 S36 | T23 | S |
| 48 | 11 A23 | I11 E6 Q6 L4 K2 T2 W2 S D R | A |
| 49 | 8 E37 | K8 D6 Q3 A2 P H T | E |
| 50 | 7 E27 | D25 K2 L2 M Q Y | D |
| 51 | 2 C58 | A | C |
| 52 | 9 M17 | R15 E8 L7 K6 Q2 T2 H V | M |
| 53 | 11 R37 | E6 Q5 K2 C2 H2 A N G D W | R |
| 54 | 8 T41 | Y5 A4 V3 I2 E2 M K | T |
| 55 | 1 C59 | | C |
| 56 | 10 G33 | V9 R5 I4 E3 L A S T K | G |
| 57 | 12 G34 | V6 -5 A3 R2 I2 P2 D K S L N | G |
| 58 | 10 A25 | -15 P7 K3 S2 Y2 G2 F D R | A |

### Legend for Table 15

1 BPTI
2 synthetic BPTI, Tan & Kaiser, biochem. 16(8)1531-41
3 Semisynthetic BPTI, TSCH87
4 Semisynthetic BPTI, TSCH87
5 Semisynthetic BPTI, TSCH87
6 Semisynthetic BPTI, TSCH87
7 Semisynthetic BPTI, TSCH87
8 Engineered BPTI, AUER8
9 BPTI Auerswald &al GB 2 208 511A
10 BPTI Auerswald &al GB 2 208 511A
11 Engineered BPTI From MARK87
12 Engineered BPTI From MARK87
13BPTI(KR15,ME52) : Auerswald '88, Biol Chem Hoppe-Seyler, 369 Suppl, pp27-35.
14BPTI CA30/CA51 Eigenbrot &al, Protein Engineering 3(7)591-598 ('90)
15Isoaprotinin 2 Siekmann et al '88, Biol Chem Hoppe-Seyler, 369:157-163.
16Isoaprotinin G-2: Siekmann et al '88, Biol Chem Hoppe-Seyler, 369:157-163.
17 BPTI Engineered, Auerswald &al GB 2 208 511A
18 BPTI Engineered, Auerswald &al GB 2 208 511A
19 BPTI Engineered, Auerswald &al GB 2 208 511A
20Isoaprotinin G-1 Siekmann &al '88, Biol Chem Hoppe-Seyler, 369:157-163.
21 BPTI Engineered, Auerswald &al GB 2 208 511A
22 BPTI Engineered, Auerswald &al GB 2 208 511A
23 Bovine Serum (in Dufton '85)
24 Bovine spleen TI II (FIOR85)
25 Snail mucus (Helix pomatia) (WAGN78)g
26Hemachatus hemachates (Ringhals Cobra) HHV II (in Dufton '85)
27 Red sea turtle egg white (in Dufton '85)
28 Bovine Colostrum (in Dufton '85)
29 Naja nivea (Cape cobra) NNV II (in Dufton '85)
30 Bungarus fasciatus VIII B toxin (in Dufton '85)
31 Vipera ammodytes TI toxin (in Dufton '85)
32 Porcine ITI domain 1, (in CREI87)
33Human Alzheimer's beta APP protease inhibitor, (SHIN90)
34 Equine ITI domain 1, in Creighton & Charles
35 Bos taurus (inactive) BI-8e (ITI domain 1)
36Anemonia sulcata (sea anemone) 5 II (in Dufton '85)
37Dendroaspis polylepis polylepes (Black Mamba) E toxin (in Dufton '85)
38Vipera russelli (Russel's viper) RVV II (TAKA74)
39Tachypleus tridentatus (Horseshoe crab) hemocyte inhibitor (NAKA87)
40 LACI 2 (Factor Xa) (WUNT88)
41 Vipera ammodytes CTI toxin (in Dufton '85) Identification codes for Tables 14 and 15
42Dendroaspis polylepis polylepis (Black Mamba) venom K (in Dufton '85)
43Homo sapiens HI-8e "inactive" domain (in Dufton '85)
44Green Mamba toxin K, (in CREI87)
45Dendroaspis angusticeps (Eastern green mamba) C13 S1 C3 toxin (in Dufton '85)
46 LACI 3
47 Equine ITI domain 2, (CREI87)
48 LACI 1 (VIIa)
49Dendroaspis polylepis polylepes (Black mamba) B toxin (in Dufton '85)
50 Porcine ITI domain 2, Creighton and Charles
51Homo sapiens HI-8t "active" domain (in Dufton '85)
52 Bos taurus (active) BI-8t
53Trypstatin Kito &al ('88) J Biol Chem 263(34)18104-07
54Dendroaspis angusticeps (Eastern Green Mamba) C13 S2 C3 toxin (in Dufton '85)
55Green Mamba I venom Creighton & Charles '87 CSHSQB 52:511-519.
56 beta bungarotoxin B2 (in Dufton '85)
57Dendroaspis polylepis polylepis (Black mamba) venom I (in Dufton '85)
58 beta bungarotoxin B1 (in Dufton '85)
59 Bombyx mori (silkworm) SCI-III (SASA84)

**Table 61:**

| Variability of Naturally-occuring Kunitz domains | | | |
|---|---|---|---|
| Res. Id. | Different AAs | Contents BPTI | |
| 1 | 12 | R16 K6 T4 A3 H2 G2 M N P I L - | R |
| 2 | 9 | P18 R6 A4 V4 E3 N F H I | P |
| 3 | 10 | D14 K8 S4 A3 T3 G2 E2 L R Y | D |
| 4 | 9 | F17 A6 L4 S4 Y3 D2 V W I | F |
| 5 | 1 | C39 | C |
| 6 | 12 | L7 N7 E6 K4 Q4 I3 S2 Y2 R F T A | L |
| 7 | 5 | L29 E7 F S T | E |
| 8 | 9 | P27 H3 D2 G2 I K L E Q | P |
| 9 | 11 | A10 P10 I4 V4 R3 Y3 H Q E K L | P |
| 10 | 9 | E8 V6 Y6 D5 A4 S3 N3 R3 I | Y |
| 11 | 11 | T12 Q8 P7 R3 A2 Y2 K S D V I | T |
| 12 | 1 | G39 | G |
| 13 | 4 | P27 R7 L4 I | P |
| 14 | 1 | C39 | C |
| 15 | 6 | K18 R11 L4 Y3 M2 N | K |
| 16 | 7 | A25 G7 D2 K2 F Q R | A |
| 17 | 12 | K7 R7 F5 M4 H3 Y3 A2 G2 S2 L2 N I | R |
| 18 | 8 | I23 M6 F4 L2 K A E T | I |
| 19 | 10 | P13 I6 R6 K4 S4 Q2 L N E T | I |
| 20 | 5 | R21 A7 L5 S5 Q | R |
| 21 | 5 | F16 Y16 W5 I L | Y |
| 22 | 5 | Y17 F14 A5 H2 N | F |
| 23 | 2 | Y32 F7 | Y |
| 24 | 4 | N29 D7 K2 S | N |
| 25 | 13 | A11 S6 G4 W4 Q3 K2 L2 P2 R I T V N | A |
| 26 | 12 | K13 A9 T5 V3 S2 H D Q R E F G | K |
| 27 | 8 | A13 S12 K5 Q3 T3 I E L | A |
| 28 | 7 | G14 K10 N5 M4 H2 Q2 R2 | G |
| 29 | 8 | K13 Q11 A5 L4 F2 R2 G M | L |
| 30 | 1 | C39 | C |
| 31 | 10 | E16 Q8 L5 V4 A N I R K Y | Q |
| 32 | 10 | P11 T7 K5 L4 R3 Q3 E2 G2 S V | T |
| 33 | 1 | F39 | F |
| 34 | 12 | I10 V6 T5 N3 D3 K3 Q2 H2 F2 S P L | V |
| 35 | 2 | Y36 W3 | Y |
| 36 | 2 | G31 S8 | G |
| 37 | 1 | G39 | G |
| 38 | 1 | C39 | C |
| 39 | 8 | G14 R9 K6 Q3 M3 L2 E P | R |
| 40 | 2 | G33 A6 | A |
| 41 | 2 | N33 K6 | K |
| 42 | 10 | A13 G8 S6 R4 N2 Q2 E K L M | R |
| 43 | 1 | N39 | N |
| 44 | 3 | N20 R14 K5 | N |
| 45 | 2 | F38 Y | F |
| 46 | 11 | K19 Y5 E4 R2 V2 D2 H S T A L | K |
| 47 | 2 | T22 S17 | S |
| 48 | 10 | I12 Q6 A5 E5 L3 K2 T2 W2 R S | A |
| 49 | 6 | E19 K8 D7 Q3 P A | E |
| 50 | 6 | E27 D7 K2 M Q Y | D |
| 51 | 1 | C39 | C |
| 52 | 9 | R13 M7 L7 K6 Q2 N H E V | M |
| 53 | 10 | R20 E6 Q4 H2 K2 A N G D W | R |
| 54 | 6 | T24 Y5 A4 V3 I2 M | T |
| 55 | 1 | C39 | C |
| 56 | 9 | G15 V10 R5 I3 E2 A S T K | G |
| 57 | 10 | G17 V5 -5 A3 R2 I2 P2 S D K | G |
| 58 | 9 | -15 P7 A7 K3 S2 G2 R F D | A |

**Table 62:**

| Kunitz sequences used in compilation of Table 61 |
|---|
| 1 BPTI |
| 2Isoaprotinin 2 (SIEK88) |
| 3Isoaprotinin G-2 (SIEK88) |
| 4Isoaprotinin G-1 (SIEK88) |
| 5Bovine Serum (in DUFT85) |
| 6Bovine spleen TI II (FIOR85) |
| 7Snail mucus *(Helix pomatia*) (WAGN78) |
| 8*Hemachatus hemachates* (Ringhals Cobra) HHV II (in DUFT85) |
| 9Red sea turtle egg white (in DUFT85) |
| 10Bovine Colostrum (in DUFT85) |
| 11*Naja nivea* (Cape cobra) NNV II (in DUFT85) |
| 12*Bungarus fasciatus* VIII B toxin (in DUFT85) |
| 13*Vipera ammodytes* TI toxin (in DUFT85) |
| 14Porcine ITI domain 1, (in CREI87) |
| 15Human Alzheimer's β APP protease inhibitor (SINH90) |
| 16Equine ITI domain 1 (in CREI87) |
| 17*Bos taurus* (inactive) BI-8e (ITI domain 1) (in CREI87) |
| 18*Anemonia sulcata* (sea anemone) 5 II (in DUPT85) |
| 19*Dendroaspis polylepis polylepes* (Black Mamba) E toxin (in DUFT85) |
| 20*Vipera russelli* (Russel's viper) RVV II (TAKA74) |
| 21*Tachypleus tridentatus* (Horseshoe crab) hemocyte inhibitor (NAKA87) |
| 22LACI 2 (Factor Xa) (WUNT88) |
| 23*Vipera ammodytes* CTI toxin (in DUFT85) |
| 24*Naja naja naja* venom (SHAF90) |
| 25*Dendroaspis polylepis polylepis* (Black Mamba) venom K (in DUFT85) |
| 26*Homo sapiens* HI-8e "inactive" domain (in DUFT85) |
| 27Green Mamba toxin K, (in CREI87) |
| 28*Dendroaspis angusticeps* (Eastern green mamba) C13 S1 C3 toxin (in DUFT85) |
| 29LACI 3 (WUNT88) |
| 30Equine ITI domain 2 (in CREI87) |
| 31LACI 1 (VIIa) (GIRA90) |
| 32*Dendroaspis polylepis polylepes* (Black mamba) B toxin (in DUFT85) |
| 33Porcine ITI domain 2 (in CRE1887) |
| 34*Homo sapiens* HI-8t "active" domain (in DUPT85) |
| 35*Bos taurus* (active) BI-8t (in CREI87) |
| 36Trypstatin (KITO88) |
| 37*Dendroaspis angusticeps* (Eastern Green Mamba) C13 S2 C3 toxin (in DUFT85) |
| 38Green Mamba I venom (in CREI87) |
| 39*Dendroaspis polylepis polylepis* (Black mamba) venom I (in DUFT85) |

**Table 63:**

| Histogram of (Number of residues having given variability) *vs.* Variability | | |
|---|---|---|
| N different | 58 locations | Core 51 sites |
| 1 | 10 | 10 |
| 2 | 7 | 7 |
| 3 | 1 | 1 |
| 4 | 2 | 2 |
| 5 | 4 | 4 |
| 6 | 4 | 4 |
| 7 | 2 | 2 |
| 8 | 4 | 4 |
| 9 | 7 | 5 |
| 10 | 8 | 7 |
| 11 | 3 | 3 |
| 12 | 5 | 4 |
| 13 | 1 | 1 |

**Table 64:**

| Citations for Table of Naturally-Occurring Kunitz Domains (Table 62) |
|---|
| CREI87Creighton & Charles (1987) *Cold Spring Harbor Symp Quant Biol* **52**:511-519. |
| DUFT85Dufton (1985) *Eur J Biochem* **153**:647-654. |
| FIOR85Fioretti *et al*. (1985) *J Biol Chem* **260**:11451-11455. |
| GIRA90 Guard *et al*. (1990) *Science* **248**:1421-24. |
| KITO88 Kito *et al*. (1988) *J Biol Chem* **263**(34)18104-07 |
| NAKA8 7Nakamura *et al*. (1987) *J Biochem* **101**:1297-1306. |
| SHAF90 Shafqat *et al*. (1990) *Eur J Biochem* **194**:337-341. |
| SIEK88 Siekmann *et al*. (1988) *Biol Chem Hoppe-Seyler*, **369**:157-163. |
| TAKA74Takahashi *et al*. (1974) J *Biochem* **76**:721-733. |
| WAGN78Wagner *et al*. (1978) *Eur J Biochem* **89**:367-377. |
| WUNT88 Wun *et al*. (1988) *J Biol Chem* **263**:6001-4. |

**Table 65:**

| Effects of mutations to Kunitz domains on binding to serine proteases. | | | |
|---|---|---|---|
| Classes: ANo major effect expected if molecular charge stays in range -1 to +1. B Major effects not expected, but are more likely than in "A". C Residue in the binding interface; any change must be tested. X No substitution allowed. | | | |

| Res. Id. | EpiNE1 | Substitutions | Class |
|---|---|---|---|
| 1 | R | any | A |
| 2 | P | any | A |
| 3 | D | any | A |
| 4 | F | Y, W, L | B |
| 5 | C | C | X |
| 6 | L | non-proline | A |
| 7 | E | L, S, T, D, N, K, R | A |
| 8 | P | any | A |
| 9 | P | any | A |
| 10 | Y | non-proline prefr'd | B |
| 11 | T | any | C |
| 12 | G | must be G | X |
| 13 | P | any | C |
| 14 | C | C strongly preferred, any non-proline | C |
| 15 | I | V, A | C |
| 16 | A | | C |
| 17 | F | L, I, M, Y, W, H, V | C |
| 18 | F | Y, W, H | C |
| 19 | P | any | C |
| 20 | R | non-proline prefr'd | C |
| 21 | Y | F & Y most prefr'd; W, I, L prefr'd; M, V allowed | C |
| 22 | F | Y & F most prefr'd; non-proline prefr'd | Y, F B |
| 23 | Y | Y & F strongly prefr'd | F ,Y B |
| 24 | N | non-proline prefr'd | A |
| 25 | A | any | A |
| 26 | K | any | A |
| 27 | A | any | A |
| 28 | G | non-proline prefr'd | A |
| 29 | L | non-proline prefr'd | A |
| 30 | C | must be C | X |
| 31 | Q | non-proline prefr'd | B |
| 32 | T | non-proline prefr'd | B |
| 33 | F | F very strongly prefr'd; Y possible | X |
| 34 | V | any | C |
| 35 | Y | Y most prefr'd; W prefr'd; F allowed | B |
| 36 | G | G strongly prefr'd; S, A prefr'd; | C |
| 37 | G | must be G so long as 38 is C | X |
| 38 | C | C strongly prefr'd | X |
| 39 | M | any | C |
| 40 | G | A,S,N,D,T,P | C |
| 41 | N | K,Q,S,D,R,T,A,E | C |
| 42 | G | any | C |
| 43 | N | must be N | X |
| 44 | N | S,K,R,T,Q,D,E | B |
| 45 | F | Y | B |
| 46 | K | any non-proline | B |
| 47 | ST, | N, A, G | B |
| 48 | A | any B | |
| 49 | E | any | A |
| 50 | D | any | A |
| 51 | C | must be C | X |
| 52 | M | any | A |
| 53 | R | any | A |
| 54 | T | any | A |
| 55 | C | must be C | X |
| 56 | G | any | A |
| 57 | G | any | A |
| 58 | A | any | A |

prefr'd stands for preferred.

**TABLE 203**

| Effect of pH on the Disociation of Bound BPTI-III MK and BPTI(K15L)-III MA Phage from Immobilized HNE | | | | |
|---|---|---|---|---|
| pH | BPTI-III MK | | BPTI(K15L)-III MA | |
| | Total Plaque-Forming Units in Fraction | % of Input Phage | Total Plaque-Forming Units in Fraction | % of Input Phage |
| 7.0 | 5.0·10⁴ | 2·10⁻³ | 1.7·10⁵ | 3.2·10⁻² |
| 6.0 | 3.8·10⁴ | 2·10⁻³ | 4.5·10⁵ | 8.6·10⁻² |
| 5.0 | 3.5·10⁴ | 1·10⁻³ | 2.1·10⁶ | 4.0·10⁻¹ |
| 4.0 | 3.0·10⁴ | 1·10⁻³ | 4.3·10⁶ | 8.2·10⁻¹ |
| 3.0 | 1.4·10⁴ | 1·10⁻³ | 1.1·10⁶ | 2.1·10⁻¹ |
| 2.2 | 2.9·10⁴ | 1·10⁻³ | 5.9·10⁴ | 1.1·10⁻² |
| Percentage of Input Phage = 8.0·10⁻³ Recovered | | | Percentage ofInput Phage = 1.56 Recovered | |
| The total input of BPTI-III MK phage was 0.030 ml x (8.6·10¹⁰ pfu/ml) = 2.6·10⁹. | | | | |
| The total input of BPTI(K15L)-III MA phage was 0.030 ml x (1.7·10¹⁰ pfu/ml) = 5.2·10⁸. | | | | |
| Given that the infectivity of BPTI(K15L)-III MA phage is 5 fold lower than that of BPTI-III MK phage, the phage inputs utilized above ensure that an equivalent number of phage particles are added to the immobilized HNE. | | | | |

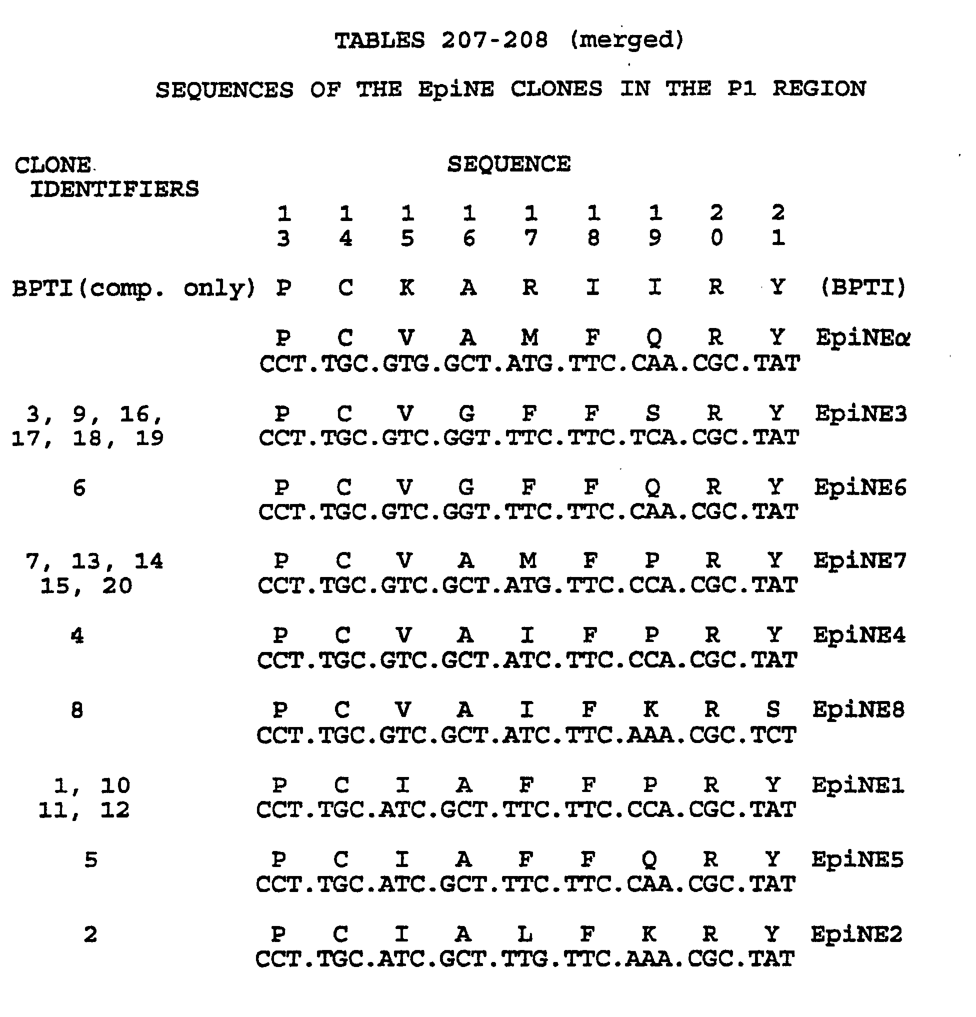

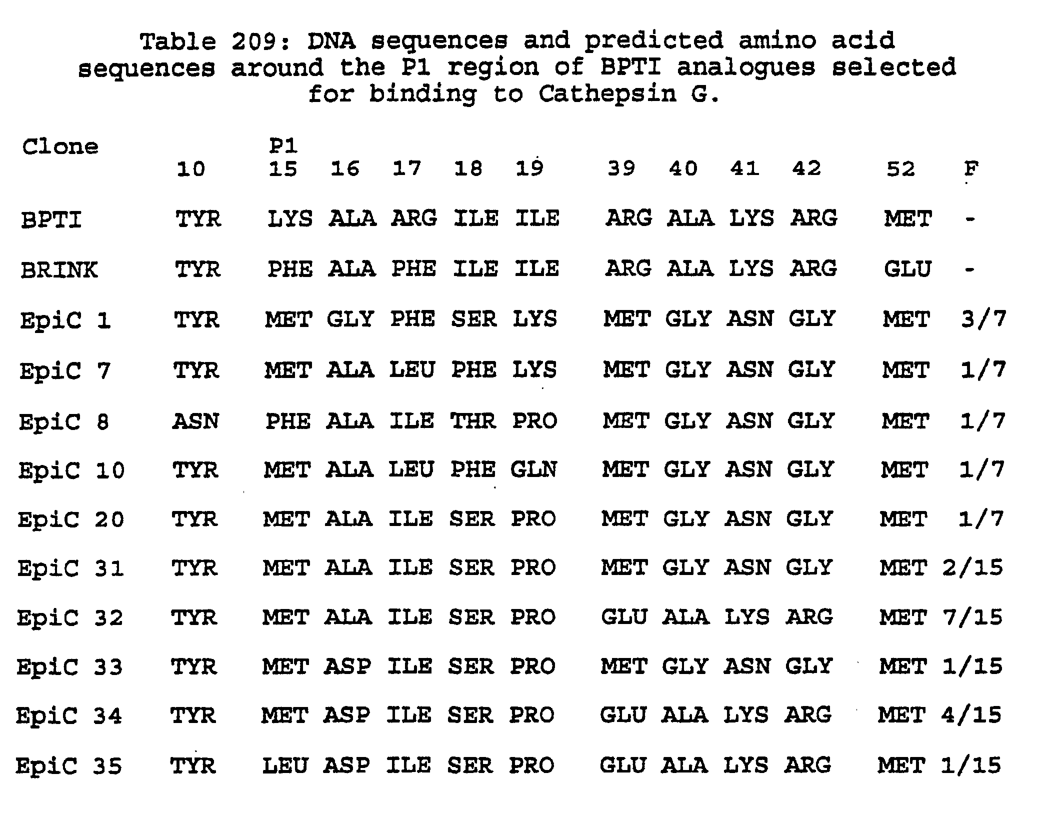

**TABLE 211**

| Effects of antisera on phage infectivity | | | |
|---|---|---|---|
| Phage (dilution of stock) | Incubation Conditions | pfu/ml | Relative Titer |
| MA-ITI | PBS | | |
| 1.2·10¹¹ | 1.00 | | |
| (10⁻¹) | NRS | 6.8·10¹⁰ | 0.57 |
| | anti-ITI | 1.1·10¹⁰ | 0.09 |
| | | | |
| MA-ITI | PBS | 7.7·10⁸ | 1.00 |
| (10⁻³) | NRS | 6.7·10⁸ | 0.87 |
| | anti-ITI | 8.0·10⁶ | 0.01 |
| | | | |
| MA | PBS | 1.3·10¹² | 1.00 |
| (10⁻¹) | NRS | 1.4·10¹² | 1.10 |
| | anti-ITI | 1.6·10¹² | 1.20 |
| | | | |
| MA | PBS | 1.3·10¹⁰ | 1.00 |
| (10⁻³) | NRS | 1.2·10¹⁰ | 0.92 |
| | anti-ITI | 1.5·10¹⁰ | 1.20 |

**TABLE 212**

| Fractionation of EpiNE-7 and MA-ITI phage on hNE beads | | | | |
|---|---|---|---|---|
| | EpiNE-7 | | MA-ITI | |
| Sample | Total pfu in sample | Fraction of input | Total pfu in sample | Fraction of input |
| INPUT | 3.3·10⁹ | 1.00 | 3.4·10¹¹ | 1.00 |
| Final TBS-TWEEN Wash | 3.8·10⁵ | 1.2·10⁻⁴ | 1.8·10⁶ | 5.3·10⁻⁶ |
| | | | | |
| pH 7.0 | 6.2·10⁵ | 1.8·10⁻⁴ | 1.6·10⁶ | 4.7·10⁻⁶ |
| pH 6.0 | 1.4·10⁶ | 4.1·10⁻⁴ | 1.0·10⁶ | 2.9·10⁻⁶ |
| pH 5.5 | 9.4·10⁵ | 2.8·10⁻⁴ | 1.6·10⁶ | 4.7·10⁻⁶ |
| pH 5.0 | 9.5·10⁵ | 2.9·10⁻⁴ | 3.1·10⁵ | 9.1·10⁻⁷ |
| pH 4.5 | 1.2·10⁶ | 3.5·10⁻⁴ | 1.2·10⁵ | 3.5·10⁻⁷ |
| pH 4.0 | 1.6·10⁶ | 4.8·10⁻⁴ | 7.2·10⁴ | 2.1·10⁻⁷ |
| pH 3.5 | 9.5·10⁵ | 2.9·10⁻⁴ | 4.9·10⁴ | 1.4·10⁻⁷ |
| pH 3.0 | 6.6·10⁵ | 2.0·10⁻⁴ | 2.9·10⁴ | 8.5·10⁻⁸ |
| pH 2.5 | 1.6·10⁵ | 4.8·10⁻⁵ | 1.4·10⁴ | 4.1·10⁻⁸ |
| pH 2.0 | 3.0·10⁵ | 9.1·10⁻⁵ | 1.7·10⁴ | 5.0·10⁻⁸ |
| SUM* | 6.4·10⁶ | 3·10⁻³ | 5.7·10⁶ | 2·10⁻⁵ |

| | | | | |
|---|---|---|---|---|
| * SUM is the total pfu (or fraction of input) obtained from all pH elution fractions | | | | |

**TABLE 213**

| Fractionation of EpiC-10 and MA-ITI phage on Cat-G beads | | | | |
|---|---|---|---|---|
| | Epic-10 | | MA-ITI | |
| Sample | Total pfu in sample | Fraction of input | Total pfu in sample | Fraction of input |
| INPUT | 5.0·10¹¹ | 1.00 | 4.6·10¹¹ | 1.00 |
| Final TBS-TWEEN Wash | 1.8·10⁷ | 3.6·10⁻⁵ | 7.1·10⁶ | 1.5·10⁻⁵ |
| | | | | |
| pH 7.0 | 1.5·10⁷ | 3.0·10⁻⁵ | 6.1·10⁶ | 1.3·10⁻⁵ |
| pH 6.0 | 2.3·10⁷ | 4.6·10⁻⁵ | 2.3·10⁶ | 5.0·10⁻⁶ |
| pH 5.5 | 2.5·10⁷ | 5.0·10⁻⁵ | 1.2·10⁶ | 2.6·10⁻⁶ |
| pH 5.0 | 2.1·10⁷ | 4.2·10⁻⁵ | 1.1·10⁶ | 2.4·10⁻⁶ |
| pH 4.5 | 1.1·10⁷ | 2.2·10⁻⁵ | 6.7·10⁵ | 1.5·10⁻⁶ |
| pH 4.0 | 1.9·10⁶ | 3.8·10⁻⁶ | 4.4·10⁵ | 9.6·10⁻⁷ |
| pH 3.5 | 1.1·10⁶ | 2.2·10⁻⁶ | 4.4·10⁵ | 9.6·10⁻⁷ |
| pH 3.0 | 4.8·10⁵ | 9.6·10⁻⁷ | 3.6·10⁵ | 7.8·10⁻⁷ |
| pH 2.5 | 2.0·10⁵ | 4.0·10⁻⁷ | 2.7·10⁵ | 5.9·10⁻⁷ |
| pH 2.0 | 2.4·10⁵ | 4.8·10⁻⁷ | 3.2·10⁵ | 7.0·10⁻⁷ |
| SUM* | 9.9·10⁷ | 2·10⁻⁴ | 1.4·10⁷ | 3·10⁻⁵ |

| | | | | |
|---|---|---|---|---|
| * SUM is the total pfu (or fraction of input) obtained from all pH elution fractions | | | | |

**TABLE 214**

| Abbreviated fractionation of display phage on hNE beads | | | | |
|---|---|---|---|---|
| | | DISPLAY PHAGE | | |
| | EpiNE-7 | MA-ITI 2 | MA-ITI-E7 1 | MA-ITI-E7 2 |
| INPUT (pfu) | 1.00 (1.8·10⁹) | 1.00 (1.2·10¹⁰) | 1.00 (3.3·10⁹) | 1.00 (1.1·10⁹) |
| WASH | 6·10⁻⁵ | 1·10⁻⁵ | 2·10⁻⁵ | 2·10⁻⁵ |
| pH 7.0 | 3·10⁻⁴ | 1·10⁻⁵ | 2·10⁻⁵ | 4·10⁻⁵ |
| pH 3.5 | 3·10⁻³ | 3·10⁻⁶ | 8·10⁻⁵ | 8·10⁻⁵ |
| pH 2.0 | 1·10⁻³ | 1·10⁻⁶ | 6·10⁻⁶ | 2·10⁻⁵ |
| SUM* | 4.3·10⁻³ | 1.4·10⁻⁵ | 1.1·10⁻⁴ | 1.4·10⁻⁴ |

| | | | | |
|---|---|---|---|---|
| * SUM is the total fraction of input pfu obtained from all pH elution fractions | | | | |

**TABLE 215**

| Fractionation of EpiNE-7 and MA-ITI-E7 phage on hNE beads | | | | |
|---|---|---|---|---|
| | EpiNE-7 | | MA-ITI-E7 | |
| Sample | Total pfu in sample | Fraction of input | Total pfu in sample | Fraction of input |
| INPUT | 1.8·10⁹ | 1.00 | 3.0·10⁹ | 1.00 |
| | | | | |
| ^{p}H 7.0 | 5.2·10⁵ | 2.9·10⁻⁴ | 6.4·10⁴ | 2.1·10⁻⁵ |
| pH 6.0 | 6.4·10⁵ | 3.6·10⁻⁴ | 4.5·10⁴ | 1.5·10⁻³ |
| pH 5.5 | 7.8·10⁵ | 4.3·10⁻⁴ | 5.0·10⁴ | 1.7·10⁻⁵ |
| pH 5.0 | 8.4·10⁵ | 4.7·10⁻⁴ | 5.2·10⁴ | 1.7·10⁻⁵ |
| pH 4.5 | 1.1·10⁶ | 6.1·10⁻⁴ | 4.4·10⁴ | 1.5·10⁻⁵ |
| pH 4.0 | 1.7·10⁶ | 9.4·10⁻⁴ | 2.6·10⁴ | 8.7·10⁻⁶ |
| ^{p}H 3.5 | 1.1·10⁶ | 6.1·10⁻⁴ | 1.3·10⁴ | 4.3·10⁻⁶ |
| pH 3.0 | 3.8·10⁵ | 2.1·10⁻⁴ | 5.6·10³ | 1.9·10⁻⁶ |
| pH 2.5 | 2.8·10⁵ | 1.6·10⁻⁴ | 4.9·10³ | 1.6·10⁻⁶ |
| pH 2.0 | 2.9·10⁵ | 1.6·10⁻⁴ | 2.2·10³ | 7.3·10⁻⁷ |
| SUM* | 7.6·10⁶ | 4.1·10⁻³ | 3.1·10⁵ | 1.1·10⁻⁴ |

| | | | | |
|---|---|---|---|---|
| * SUM is the total pfu (or fraction of input) obtained from all pH elution fractions | | | | |

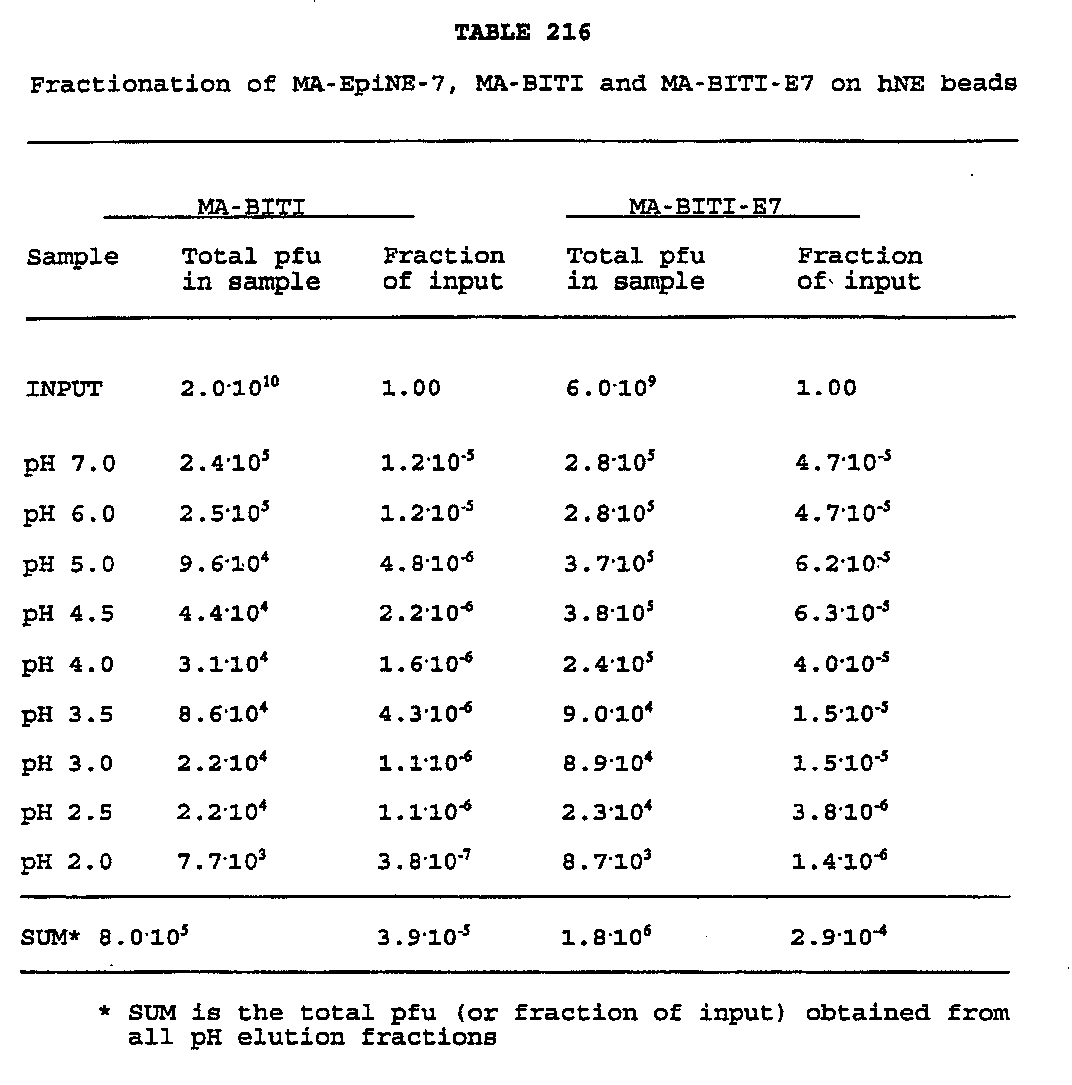

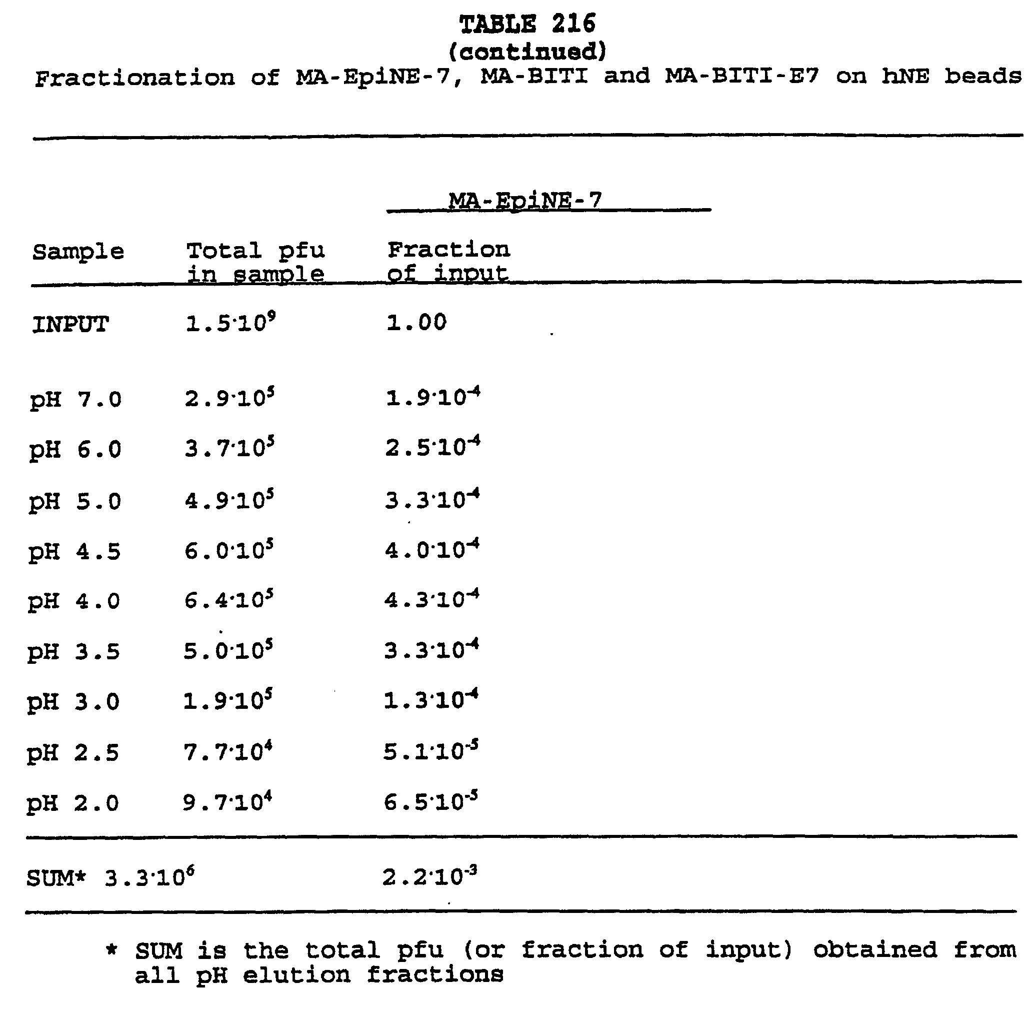

**TABLE 217**

| Fractionation of MA-BITI-E7 and MA-BITI-E7-1222 on hNE beads | | | | |
|---|---|---|---|---|
| | MA-BITI-E7 | | MA-BITI-E7-1222 | |
| Sample | Total pfu in sample | Fraction of input | Total pfu in sample | Fraction of input |
| INPUT | 1.3·10⁹ | 1.00 | 1.2·10⁹ | 1.00 |
| | | | | |
| pH 7.0 | 4.7·10⁴ | 3.6·10⁻⁵ | 4.0·10⁴ | 3.3·10⁻⁵ |
| pH 6.0 | 5.3·10⁴ | 4.1·10⁻⁵ | 5.5·10⁴ | 4.6·10⁻⁵ |
| pH 5.5 | 7.1·10⁴ | 5.5·10⁻⁵ | 5.4·10⁴ | 4.5·10⁻⁵ |
| pH 5.0 | 9.0·10⁴ | 6.9·10⁻⁵ | 6.7·10⁴ | 5.6·10⁻⁵ |
| pH 4.5 | 6.2·10⁴ | 4.8·10⁻⁵ | 6.7·10⁴ | 5.6·10⁻⁵ |
| pH 4.0 | 3.4·10⁴ | 2.6·10⁻⁵ | 2.7·10⁴ | 2.2·10⁻⁵ |
| pH 3.5 | 1.8·10⁴ | 1.4·10⁻⁵ | 2.3·10⁴ | 1.9·10⁻⁵ |
| pH 3.0 | 2.5·10³ | 1.9·10⁻⁶ | 6.3·10³ | 5.2·10⁻⁶ |
| pH 2.5 | <1.3·10³ | <1.0·10⁻⁶ | <1.3·10³ | <1.0·10⁻⁶ |
| pH 2.0 | 1.3·10³ | 1.0·10⁻⁶ | 1.3·10³ | 1.0·10⁻⁶ |
| SUM* | 3.8·10⁵ | 2.9·10⁴ | 3.4·10⁵ | 2.8·10⁻⁴ |

| | | | | |
|---|---|---|---|---|
| * SUM is the total pfu (or fraction of input) obtained from all pH elution fractions | | | | |

**TABLE 218**

| Fractionation of MA-EpiNE7 and MA-BITI-E7-141 on hNE beads | | | | |
|---|---|---|---|---|
| | MA-EpiNE7 | | MA-BITI-E7-141 | |
| Sample | Total pfu in sample | Fraction of input | Total pfu in sample | Fraction of input |
| INPUT | 6.1·10⁸ | 1.00 | 2.0·10⁹ | 1.00 |
| | | | | |
| pH 7.0 | 5.3·10⁴ | 8.7·10⁻⁵ | 4.5·10⁵ | 2.2·10⁻⁴ |
| pH 6.0 | 9.7·10⁴ | 1.6·10⁻⁴ | 4.4·10⁵ | 2.2·10⁻⁴ |
| pH 5.5 | 1.1·10⁵ | 1.8·10⁻⁴ | 4.4·10⁵ | 2.2·10⁻⁴ |
| pH 5.0 | 1.4·10⁵ | 2.3·10⁻⁴ | 7.2·10⁵ | 3.6·10⁻⁴ |
| pH 4.5 | 1.0·10⁵ | 1.6·10⁻⁴ | 1.3·10⁶ | 6.5·10⁻⁴ |
| pH 4.0 | 2.0·10⁵ | 3.3·10⁻⁴ | 1.1·10⁶ | 5.5·10⁻⁴ |
| pH 3.5 | 9.7·10⁴ | 1.6·10⁻⁴ | 5.9·10⁵ | 3.0·10⁻⁴ |
| pH 3.0 | 3.8·10⁴ | 6.2·10⁻⁵ | 2.3·10⁵ | 1.2·10⁻⁴ |
| pH 2.5 | 1.3·10⁴ | 2.1·10⁻⁵ | 1.2·10⁵ | 6.0·10⁻⁵ |
| pH 2.0 | 1.6·10⁴ | 2.6·10⁻⁵ | 1.0·10⁵ | 5.0·10⁻⁵ |
| SUM* | 8.6·10⁵ | 1.4·10⁻³ | 5.5·10⁶ | 2.8·10⁻³ |

| | | | | |
|---|---|---|---|---|
| * SUM is the total pfu (or fraction of input) obtained from all pH elution fractions | | | | |

**TABLE 219**

| pH Elution Analysis of hNE Bilnding by BITI-E7-141 Varient Display Phage | | | | | | |
|---|---|---|---|---|---|---|
| | | FRACTION OF INPUT RECOVERED AT pH: | | | RECOVERY | |
| DISPLAYED PROTEIN | INPUT PFU^{c} | 7.0^{d} | 3.5^{d} | 2.0^{d} | TOTAL^{e} | RELATIVE^{f} |
| AMINO1^{b} | 0.96 | 0.24 | 2.3 | 0.35 | 2.9 | 0.11 |
| AMINO2^{a} | 6.1 | 0.57 | 2.1 | 0.45 | 3.1 | 0.12 |
| BITI-E7-1222^{b} | 1.2 | 0.72 | 4.0 | 0.64 | 5.4 | 0.21 |
| | | | | | | |
| EpiNE7^{b} | 0.72 | 0.44 | 6.4 | 2.2 | 9.0 | 0.35 |
| MUTP1^{a} | 3.9 | 1.8 | 9.2 | 1.2 | 12 | 0.46 |
| MUT1619^{b} | 0.78 | 0.82 | 9.9 | 0.84 | 12 | 0.46 |
| | | | | | | |
| MUTQE^{a} | 4.7 | 1.2 | 16 | 5.3 | 22 | 0.85 |
| MUTT26A^{b} | 0.51 | 2.5 | 19 | 3.3 | 25 | 0.96 |
| BITI-E7-141^{a} | 1.7 | 2.2 | 18 | 5.4 | 26 | 1.00 |
| BITI-E7-141^{b} | 0.75 | 2.1 | 21 | 3.2 | 26 | 1.00 |

| | | | | | | |
|---|---|---|---|---|---|---|
| ^{a} results from abbreviated pH elution protocol | | | | | | |
| ^{b} results from extended pH elution protocol | | | | | | |
| ^{c} units are 10⁹ pfu | | | | | | |
| ^{d} units are 10⁻⁴ | | | | | | |
| ^{e} sum of pH 7.0, pH 3.5, and pH 2.0 recoveries, units are 10⁻⁴ | | | | | | |
| ^{f} total fraction of input recovered divided by total fraction of input recovered for BITI-E7-141 | | | | | | |

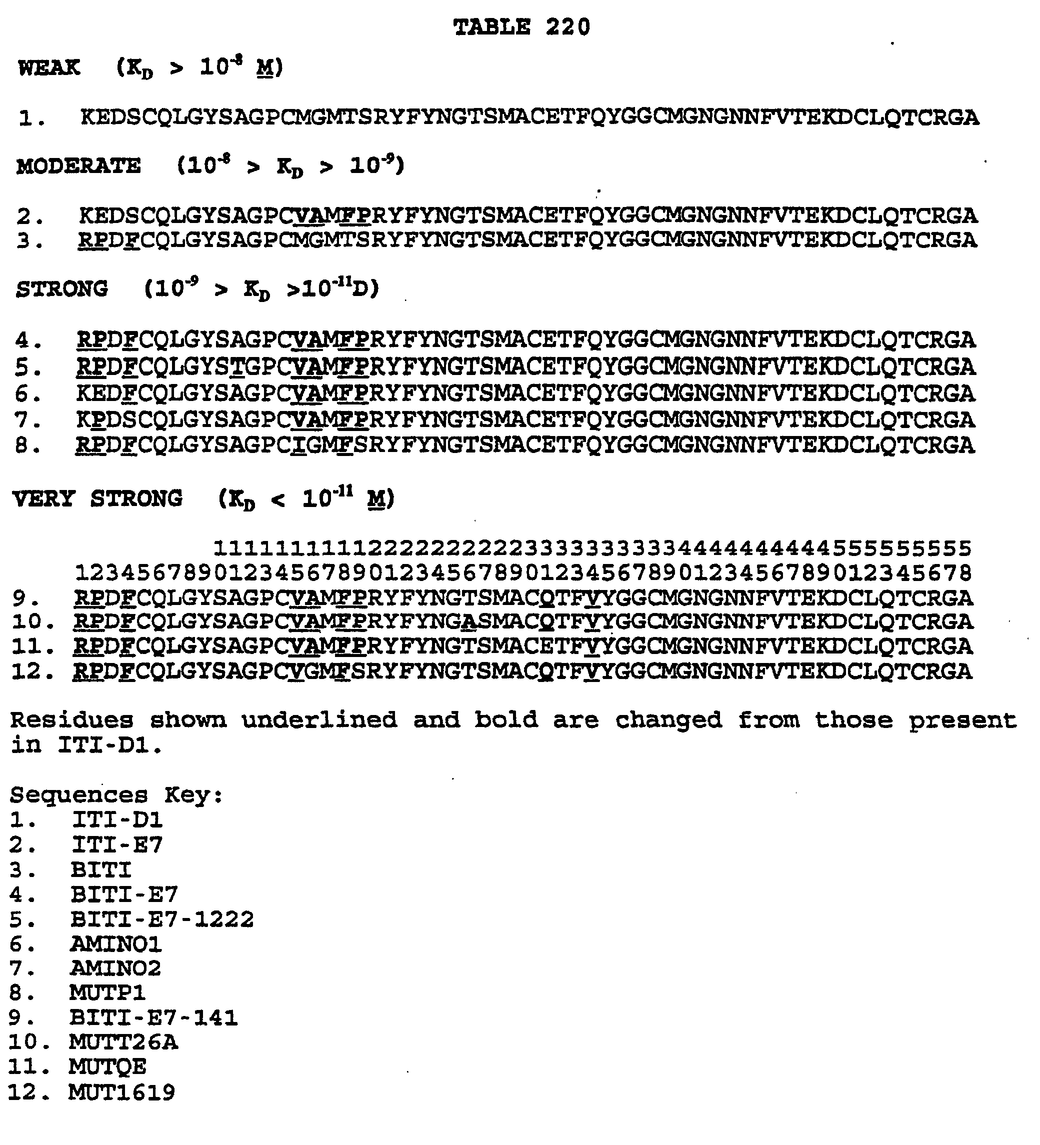

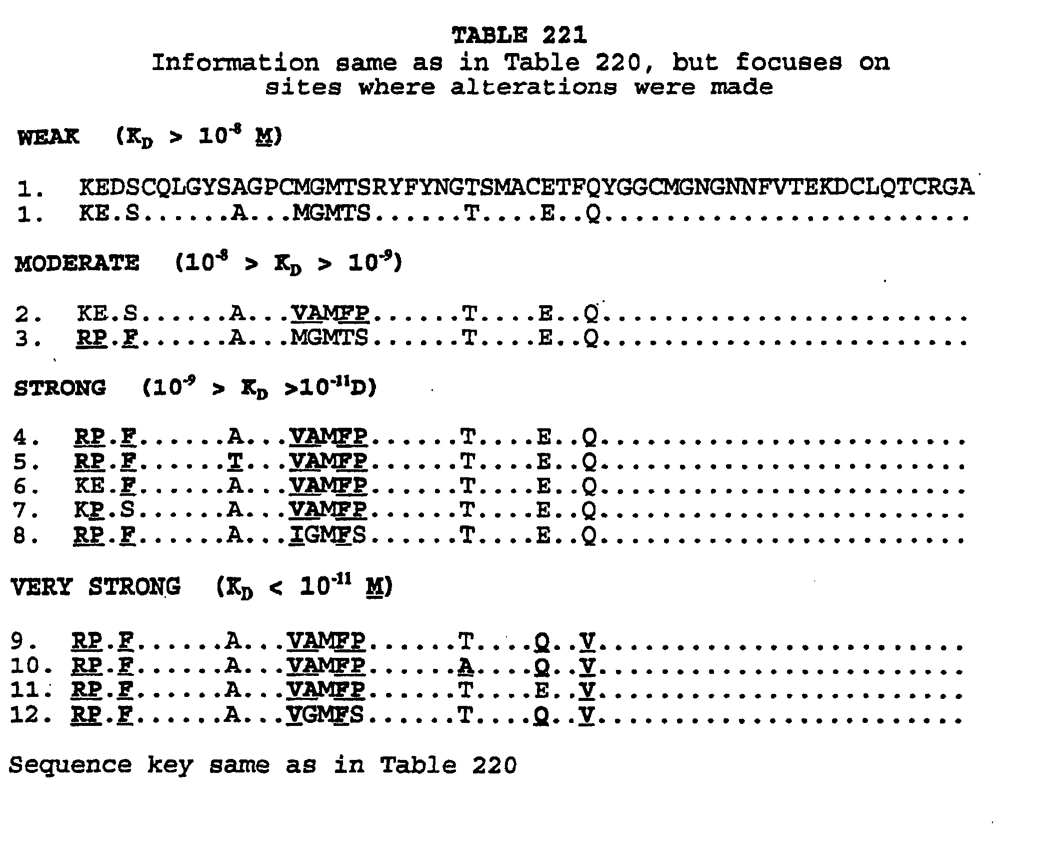

**TABLE 222**

| AFFINITY CLASS | ESTIMATED K_{D} | FRACTION OF INPUT BOUND | pH ELUTION MAXIMUM | PROTEIN |
|---|---|---|---|---|
| WEAK K_{D} > | 10⁻⁸ M | <0.005%> | pH 6.0 | ITI-D1 |
| | | | | |
| MODERATE | 10⁻⁸ M to | 0.01% to | pH 5.5 to | BITI |
| | 10⁻⁹ M | 0.03% | pH 5.0 | ITI-E7 |
| | | | | |
| STRONG | 10⁻⁹ M to | 0.03% to | pH 5.0 to | BITI-E7 |
| | 10⁻¹¹ M | 0.06% | pH4.5 | BITI-E7-1222 |
| | | | | AMINO1 |
| | | | | AMINO2 |
| | | | | MUTP1 |
| | | | | |
| VERY | | | | |
| STRONG | K_{D} < 10⁻¹¹ M | >0.1% | ≤ pH 4.0 | BITI-E7-141 |
| | | | | MUTT26A |
| | | | | MUTQE |
| | | | | MUT1619 |

## Claims

1. A non-naturally occurring protein which inhibits human neutrophil elastase, and which is a protein comprising at least the core sequence of a non-naturally occurring Kunltz domain, a Kunitz domain being **characterized by** cysteines at positions corresponding to bovine pancreatic trypsin Inhibitor (BPTI) positions 5, 30, 51, and 55, glycine at a position corresponding to BPTI position 12, Asn at a position corresponding to BPTI 43 and Phe at a position corresponding to BPTI 33, with the proviso that when the positions corresponding to BPTI 14 and 38 are cysteine, the position corresponding to BPTI 37 is glycine; the core sequence being the residues corresponding to BPTI positions 5-55;
where, in said non-naturally occurring Kunitz domain, the residue corresponding to BPTI position 18 is Phe, and
where the residues corresponding to BPTI positions 39-42 are all uncharged amino acids.

2. The protein of claim 1 which comprises residues corresponding to BPTI positions 1-58.

3. The protein of any one of claims 1-2 wherein the residues corresponding to BPTI positions 14 and 38 are Cys, and the residue corresponding to BPTI position 37 is Gly.

4. The protein of any one of claims 1-3 wherein the residue corresponding to BPTI position 45 is Phe and the residue corresponding to BPTI position 43 is Asn.

5. The protein of any one of claims 1-4 wherein the residue corresponding to BPTI position 15 is Val, Ile or Leu.

6. The protein of claim 5 wherein the residue corresponding to BPTI position 15 is Val.

7. The protein of claim 5 wherein the residue corresponding to BPTI position 15 is Ile.

8. The protein of claim 5 wherein the residue corresponding to BPTI position 15 is Leu.

9. The protein of any one of claims 1-8 wherein the residue corresponding to BPTI position 16 is Ala or Gly.

10. The protein of any one of claims 1-9 wherein the residue corresponding to BPTI position 17 is Met, Phe, Ile or Leu.

11. The protein of any one of claims 1-10 wherein the residue corresponding to BPTI position 19 is Pro, Ser, Lys or Gln.

12. The protein of any one of claims 1-11 wherein the residue corresponding to BPTI position 16 is Ala.

13. The protein of any one of claims 1-12 wherein the residue corresponding to BPTI position 17 is Phe.

14. The protein of any one of claims 1-13 wherein the residue corresponding to BPTI position 19 is Pro.

15. The protein of any one of claims 1-14 wherein the residue corresponding to BPTI position 39 is Met.

16. The protein of any one of claims 1-15 wherein the residue corresponding to BPTI position 40 is Gly, Ala, Ser, Asn, Thr or Pro.

17. The protein of any one of claims 1-16 wherein the residue corresponding to BPTI position 40 is Gly or Ala.

18. The protein of any one of claims 1-17 wherein the residue corresponding to BPTI position 40 is Gly.

19. The protein of any one of claims 1-18 wherein the residue corresponding to BPTI position 41 is Asn, Gln, Ser, Thr, or Ala.

20. The protein of any one of claims 1-19 wherein the residue corresponding to BPTI position 41 is Asn.

21. The protein of any one of claims 1-20 wherein the residue corresponding to BPTI position 42 is Gly.

22. The protein of any one of claims 1-21 where the residue corresponding to BPTI position 40 is Gly and the residue corresponding to BPTI position 42 is Gly.

23. The protein of any of claims 1-22 where the residues corresponding to BPTI positions 40, 41 and 42 are Gly, Asn and Gly, respectively.

24. The protein of any one of claims 1-23 wherein the residues corresponding to BPTI positions 39, 40, 41, and 42 are Met, Gly, Asn, and Gly respectively.

25. The protein of any one of claims 1-24 wherein the residue corresponding to BPTI position 31 is Gln.

26. The protein of any one of claims 1-25 wherein the residue corresponding to BPTI position 34 is Pro.

27. The protein of any one of claims 1-26 wherein the residue corresponding to BPTI position 34 is Val.

28. The protein of any one of claims 1-27 wherein the residue corresponding to BPTI position 1 is Arg.

29. The protein of any one of claims 1-28 wherein the residue corresponding to BPTI position 2 is Pro.

30. The protein of any one of claims 1-29 wherein the residue corresponding to BPTI position 3 is Asp.

31. The protein of any one of claims 1-30 wherein the residue corresponding to BPTI position 4 is Phe.

32. The protein of any one of claims 1-31 where for each residue corresponding to a previously unspecified position, the reference Kunitz domain is a Kunitz domain of the light chain of a human ITI.

33. The protein of any one of claims 1-31 where for each residue corresponding to a previously unspecified position, the reference Kunitz domain is BPTI or the first Kunitz domain of the light chain of a human ITI (human ITI-D1).

34. The protein of any one of claims 1-33 wherein said domain has a higher percentage identity to a naturally-occurring human Kunitz domain of Tables 13, 15 or 62 than to any naturally-occurring non-human Kunitz domain of Table 13, 15 or 62.

35. The protein of any one of claims 1-34 wherein the core amino acid sequence of said non-naturally occurring Kunitz domain is of higher percentage identity to that of a human ITI-D1 than to that of BPTI.

36. The protein of any one of claims 1-35 where the core sequence of said domain otherwise differs from the core sequence of a reference Kunitz domain,
selected from the group consisting of EpiNeα, EpiNe1, EpiNe2, EpiNe3, EpiNe4, EpiNe5, EpiNe6, EpiNe7, and EpiNe8 of Tables 207-208,
solely by one or more class A substitutions and/or one or more class B substitutions as defined in Table 65.

37. The protein of any one of claims 1-35 wherein the core sequence of said domain otherwise differs from the core sequence of a reference Kunitz domain,
selected from the group consisting of EpiNeα, EpiNe1, EpiNe2, EpiNe3, EpiNe4, EpiNe5,
EpiNe6, EpiNe7, and EpiNe8, of Tables 207-208,
solely by one or more dass A substitutions as defined in Table 65.

38. The protein of Balm 1 or 2 wherein the core sequence of said domain consists of an amino add sequence identical to that of the core sequence of a protein selected from the group consisting of EpiNeα, EpiNe1, EpiNe2, EpiNe3, EpiNe4, EpiNe5, EpiNe6, EpiNe7, and EpiNe8 of Tables 207-208.

39. The protein of claim 1 which is selected from the group consisting of EpiNeα, EpiNe1, EpiNe2, EpiNe3, EpiNe4, EpiNe5, EpiNe6, EpiNe7, and EpiNe8 of Tables 207-208.

40. The protein of claims 1 or 2 wherein the core sequence of said domain consists of an amino acid sequence identical to that of the core sequence of a protein selected from the group consisting of ITI-E7, BITI-E7, BITI-E7-1222, AMINO1, AMINO2, MUTP1, BITI-E7-141, MUTT26A, MUTQE, and MUT1619 of Table 220.

41. The protein of claim 1 which is selected from the group consisting of ITI-E7, BITI-E7, BITI-E7-1222, AMINO1, AMINO2, MUTP1, BITI-E7-141, MUTT26A, MUTQE, and MUT1619 of Table 220.

42. The protein of any one of claims 1-41 which comprises an amino acid sequence otherwise differing from the core sequence of a reference inhibitor solely by one or more substitutions of class A according to Table 65, the reference inhibitor being selected from the group consisting of ITI-E7, BITI-E7, BITI-E7-1222, AMINO1, AMINO2, MUTP1, BITI-E7-141, MUTT26A, MUTQE, and MUT1619 in Table 220.

43. The protein of any one of claims 1-41 which comprises an amino acid sequence otherwise differing from the core sequence of a reference inhibitor solely by one or more substitutions of class A and/or B according to Table 65, the reference inhibitor being selected from the group consisting of ITI-E7, BITI-E7, BITI-E7-1222, AMINO1, AMINO2, MUTP1, BIT1-E7-141, MUTT26A, MUTQE, and MUT1619 in Table 220.

44. The protein of claim 1 where (a) the residue corresponding to BPTI position 15 is Val or Ile and/or (b) the residue corresponding to BPTI position 17 is Phe.

45. The protein of any one of claims 1-44, where said Kunitz domain is not identical in amino acid sequence to any one of the amino acid sequences set forth in Table 13.

46. The protein of any one of claims 1-38, 40, or 42-45, which comprises at least two of said mutant Kuriltz domains.

47. The protein of any one of claims 1-46 which has a strong (10⁻⁹>K_{D}>10⁻¹¹M) or very strong (K_{D}<10⁻¹¹M) binding affinity for human neutrophll elastase.

48. The protein of any one of claims 1-47 which has a very strong (K_{D}<10⁻¹¹M) binding affinity for human neutrophil elastase.

49. The protein of any one of claims 1-48 which is a chimeric phage protein.

50. The protein of any one of claims 1-49 in PEGylated form.

51. The protein of any one of claims 1-50 in purified form.

52. A protein according to any one of claims 1-50 for use in binding human neutrophil elastase.

53. A protein according to any of claims 1-50 for use in inhibiting human neutrophll elastase activity.

54. A protein according to any one of claims 1-50 for use in inhibiting harmful human neutrophil elastase activity.

55. A protein according to any one of claims 1-50 for use in inhibiting excessive human neutrophil elastase activity.

56. Use of an therapeutically effective amount of a protein of any one of claims 1-50 in the preparation of a pharmaceutical composition for use in inhibiting harmful human neutrophil elastase activity.

57. Use of an inhibitorlly effective amount of a protein of any one of claims 1-50 in the preparation of a pharmaceutical composition for inhibiting excessive human neutrophil elastase activity.

## Patentansprüche

1. Nicht natürlich vorkommendes Protein, das menschliche Neutrophilen-Elastase hemmt und das ein Protein ist, das mindestens die Kernsequenz einer nicht natürlich vorkommenden Kunitz-Domäne umfasst, wobei die Kunitz-Domäne durch Cysteine in Positionen, die den Positionen 5, 30, 51 und 55 des Rinderpankreas-Trypsin-Inhibitors (BPTI) entsprechen, Glycin in einer Position, die der BPTI-Position 12 entspricht, Asn in einer Position, die der BPTI-Position 43 entspricht, und Phe in einer Position, die der BPTI-Position 33 entspricht, gekennzeichnet ist, mit der Maßgabe, dass, wenn die Positionen, die BPTI 14 und 38 entsprechen, Cystein sind, die Position, die BPTI 37 entspricht, Glycin ist; wobei die Kernsequenz die Reste sind, die den BPTI-Positionen 5-55 entsprechen;
wobei in der nicht natürlich vorkommenden Kunitz-Domäne, der Rest, der der BPTI-Position 18 entspricht, Phe ist, und
wobei die Reste, die den BPTI-Positionen 39-42 entsprechen, alle ungeladene Aminosäuren sind.

2. Protein nach Anspruch 1, das Reste umfasst, die den BPTI-Positionen 1-58 entsprechen.

3. Protein nach einem der Ansprüche 1 bis 2, wobei die Reste, die den BPTI-Positionen 14 und 38 entsprechen, Cys sind, und der Rest, der der BPTI-Position 37 entspricht, Gly ist.

4. Protein nach einem der Anprüche 1 bis 3, wobei der Rest, der der BPTI-Position 45 entspricht, Phe ist und der Rest, der der BPTI-Position 43 entspricht, Asn ist.

5. Protein nach einem der Ansprüche 1 bis 4, wobei der Rest, der der BPTI-Position 15 entspricht, Val, Ile oder Leu ist.

6. Protein nach Anspruch 5, wobei der Rest, der der BPTI-Position 15 entspricht, Val ist.

7. Protein nach Anspruch 5, wobei der Rest, der der BPTI-Position 15 entspricht, Ile ist.

8. Protein nach Anspruch 5, wobei der Rest, der der BPTI-Position 15 entspricht, Leu ist.

9. Protein nach einem der Ansprüche 1 bis 8, wobei der Rest, der der BPTI-Position 16 entspricht, Ala oder Gly ist.

10. Protein nach einem der Ansprüche 1 bis 9, wobei der Rest, der der BPTI-Position 17 entspricht, Met, Phe, Ile oder Leu ist.

11. Protein nach einem der Ansprüche 1 bis 10, wobei der Rest, der der BPTI-Position 19 entspricht, Pro, Ser, Lys oder Gln ist.

12. Protein nach einem der Ansprüche 1 bis 11, wobei der Rest, der der BPTI-Position 16 entspricht, Ala ist.

13. Protein nach einem der Ansprüche 1 bis 12, wobei der Rest, der der BPTI-Position 17 entspricht, Phe ist.

14. Protein nach einem der Ansprüche 1 bis 13, wobei der Rest, der der BPTI-Position 19 entspricht, Pro ist.

15. Protein nach einem der Ansprüche 1 bis 14, wobei der Rest, der der BPTI-Position 39 entspricht, Met ist.

16. Protein nach einem der Ansprüche 1 bis 15, wobei der Rest, der der BPTI-Position 40 entspricht, Gly, Ala, Ser, Asn, Thr oder Pro ist.

17. Protein nach einem der Ansprüche 1 bis 16, wobei der Rest, der der BPTI-Position 40 entspricht, Gly oder Ala ist.

18. Protein nach einem der Ansprüche 1 bis 17, wobei der Rest, der der BPTI-Position 40 entspricht, Gly ist.

19. Protein nach einem der Ansprüche 1 bis 18, wobei der Rest, der der BPTI-Position 41 entspricht, Asn, Gln, Ser, Thr oder Ala ist.

20. Protein nach einem der Ansprüche 1 bis 19, wobei der Rest, der der BPTI-Position 41 entspricht, Asn ist.

21. Protein nach einem der Ansprüche 1 bis 20, wobei der Rest, der der BPTI-Position 42 entspricht, Gly ist.

22. Protein nach einem der Ansprüche 1 bis 21, wobei der Rest, der der BPTI-Position 40 entspricht, Gly ist und der Rest, der der BPTI-Position 42 entspricht, Gly ist.

23. Protein nach einem der Ansprüche 1 bis 22, wobei die Reste, die den BPTI-Positionen 40, 41 und 42 entsprechen, Gly, Asn beziehungsweise Gly sind.

24. Protein nach einem der Ansprüche 1 bis 23, wobei die Reste, die den BPTI-Positionen 39, 40, 41 und 42 entsprechen, Met, Gly, Asn beziehungsweise Gly sind.

25. Protein nach einem der Ansprüche 1 bis 24, wobei der Rest, der der BPTI-Position 31 entspricht, Gln ist.

26. Protein nach einem der Ansprüche 1 bis 25, wobei der Rest, der der BPTI-Position 34 entspricht, Pro ist.

27. Protein nach einem der Ansprüche 1 bis 26, wobei der Rest, der der BPTI-Position 34 entspricht, Val ist.

28. Protein nach einem der Ansprüche 1 bis 27, wobei der Rest, der der BPTI-Position 1 entspricht, Arg ist.

29. Protein nach einem der Ansprüche 1 bis 28, wobei der Rest, der der BPTI-Position 2 entspricht, Pro ist.

30. Protein nach einem der Ansprüche 1 bis 29, wobei der Rest, der der BPTI-Position 3 entspricht, Asp ist.

31. Protein nach einem der Ansprüche 1 bis 30, wobei der Rest, der der BPTI-Position 4 entspricht, Phe ist.

32. Protein nach einem der Ansprüche 1 bis 31, wobei für jeden Rest, der einer vorher nicht spezifizierten Position entspricht, die Referenz-Kunitz-Domäne eine Kunitz-Domäne der leichten Kette eines menschlichen ITI ist.

33. Protein nach einem der Ansprüche 1 bis 31, wobei für jeden Rest, der einer vorher nicht spezifizierten Position entspricht, die Referenz-Kunitz-Domäne BPTI oder die erste Kunitz-Domäne der leichten Kette eines menschlichen ITI (menschlicher ITI-D1) ist.

34. Protein nach einem der Ansprüche 1 bis 33, wobei die Domäne eine höhere prozentuale Identität zu einer natürlich vorkommenden menschlichen Kunitz-Domäne der Tabellen 13, 15 oder 62 besitzt als zu jeglichen anderen natürlich vorkommenden Kunitz-Domänen der Tabellen 13, 15 oder 62.

35. Protein nach einem der Ansprüche 1 bis 34, wobei die Kernaminosäuresequenz der nicht natürlich vorkommenden Kunitz-Domäne eine prozentual höhere Identität zu der von menschlichem ITI-D1 besitzt als zu der von BPTI.

36. Protein nach einem der Ansprüche 1 bis 35, wobei sich die Kernsequenz der Domäne ansonsten von der Kernsequenz einer Referenz-Kunitz-Domäne,
ausgewählt aus der Gruppe bestehend aus EpiNeα, EpiNe1, EpiNe2, EpiNe3, EpiNe4, EpiNe5, EpiNe6, EpiNe7 und EpiNe8 der Tabellen 207 bis 208,
nur durch eine oder mehrere Substitutionen der Klasse A und/oder eine oder mehrere Substitutionen der Klasse B wie in Tabelle 65 definiert unterscheidet.

37. Protein nach einem der Ansprüche 1 bis 35, wobei sich die Kernsequenz der Domäne ansonsten von der Kernsequenz einer Referenz-Kunitz-Domäne,
ausgewählt aus der Gruppe bestehend aus EpiNeα, EpiNe1, EpiNe2, EpiNe3, EpiNe4, EpiNe5, EpiNe6, EpiNe7 und EpiNe8 der Tabellen 207 bis 208,
nur durch eine oder mehrere Substitutionen der Klasse A wie in Tabelle 65 definiert unterscheidet.

38. Protein nach Anspruch 1 oder 2, wobei die Kernsequenz der Domäne aus einer Aminosäuresequenz besteht, die zu der der Kernsequenz eines Proteins ausgewählt aus der Gruppe bestehend aus EpiNeα, EpiNe1, EpiNe2, EpiNe3, EpiNe4, EpiNe5, EpiNe6, EpiNe7 und EpiNe8 der Tabellen 207 bis 208 identisch ist.

39. Protein nach Anspruch 1, das ausgewählt ist aus der Gruppe bestehend aus EpiNeα, EpiNe1, EpiNe2, EpiNe3, EpiNe4, EpiNeS, EpiNe6, EpiNe7 und EpiNe8 der Tabellen 207 bis 208.

40. Protein nach Anspruch 1 oder 2, wobei die Kernsequenz der Domäne aus einer Aminosäuresequenz besteht, die zu der der Kernsequenz eines Proteins ausgewählt aus der Gruppe bestehend aus ITI-E7, BITI-E7, BITI-E7-1222, AMINO1, AMINO2, MUTP1, BITI-E7-141, MUTT26A, MUTQE und MUT1619 von Tabelle 220 identisch ist.

41. Protein nach Anspruch 1, das ausgewählt ist aus der Gruppe bestehend aus ITI-E7, BITI-E7, BITI-E7-1222, AMINO1, AMINO2, MUTP1, BITI-E7-141, MUTT26A, MUTQE und MUT1619 von Tabelle 220.

42. Protein nach einem der Ansprüche 1 bis 41, umfassend eine Aminosäuresequenz, die sich ansonsten von der Kernsequenz eines Referenzinhibitors nur durch eine oder mehrere Substitutionen der Klasse A nach Tabelle 65 unterscheidet, wobei der Referenzinhibitor ausgewählt ist aus der Gruppe bestehend aus ITI-E7, BITI-E7, BITI-E7-1222, AMINO1, AMINO2, MUTP1, BITI-E7-141, MUTT26A, MUTQE und MUT1619 von Tabelle 220.

43. Protein nach einem der Ansprüche 1 bis 41, das eine Aminosäuresequenz umfasst, die sich ansonsten von der Kernsequenz eines Referenzinhibitors nur durch eine oder mehrere Substitutionen der Klasse A und/oder B nach Tabelle 65 unterscheidet, wobei der Referenzinhibitor ausgewählt ist aus der Gruppe bestehend aus ITI-E7, BITI-E7, BITI-E7-1222, AMINO1, AMINO2, MUTP1, BITI-E7-141, MUTT26A, MUTQE und MUT1619 von Tabelle 220.

44. Protein nach Anspruch 1, wobei (a) der Rest, der der BPTI-Position 15 entspricht, Val oder Ile ist und/oder (b) der Rest, der der BPTI-Position 17 entspricht, Phe ist.

45. Protein nach einem der Ansprüche 1 bis 44, wobei die Kunitz-Domäne in ihrer Aminosäuresequenz zu keiner der in Tabelle 13 dargestellten Aminosäuresequenzen identisch ist.

46. Protein nach einem der Ansprüche 1 bis 38, 40 oder 42 bis 45, das mindestens zwei der mutanten Kunitz-Domänen umfasst.

47. Protein nach einem der Ansprüche 1 bis 46, das eine starke (10⁻⁹>K_{D}>10⁻¹¹M) oder sehr starke (K_{D}<10⁻¹¹M) Bindungsaffinität für menschliche Neutrophilen-Elastase besitzt.

48. Protein nach einem der Ansprüche 1 bis 47, das eine sehr starke (k_{D}<10⁻¹¹M) Bindungsaffinität für menschliche Neutrophilen-Elastase besitzt.

49. Protein nach einem der Ansprüche 1 bis 48, das ein chimeres Phagenprotein ist.

50. Protein nach einem der Ansprüche 1 bis 49, das in Polyethlylenglycolysierter Form vorliegt.

51. Protein nach einem der Ansprüche 1 bis 50, das in gereinigter Form vorliegt.

52. Protein nach einem der Ansprüche 1 bis 50 zur Verwendung in der Bindung menschlicher Neutrophilen-Elastase.

53. Protein nach einem der Ansprüche 1 bis 50 zur Verwendung in der Hemmung menschlicher Neutrophllen-Elastaseaktivität.

54. Protein nach einem der Ansprüche 1 bis 50 zur Verwendung in der Hemmung von schädlicher menschlicher Neutrophilen-Elastaseaktivität.

55. Protein nach einem der Ansprüche 1 bis 50 zur Verwendung in der Hemmung von übermäßiger Neutrophilen-Elastaseaktivität.

56. Verwendung einer therapeutisch wirksamen Menge eines Proteins nach einem der Ansprüche 1 bis 50 für die Herstellung eines Arzneimittels zur Hemmung schädlicher menschlicher Neutrophilen-Elastaseaktivtät.

57. Verwendung einer für die Hemmung wirksamen Menge eines Proteins nach einem der Ansprüche 1 bis 50 für die Herstellung eines Arzneimittels zur Hemmung von übermäßiger menschlicher Neutrophilen-Elastaseaktivität.

## Revendications

1. Protéine d'origine non naturelle qui inhibe l'élastase neutrophile humaine, et qui est une protéine comprenant au moins la séquence principale d'un domaine Kunitz d'origine non naturelle, un domaine Kunitz étant **caractérisé par** des cystéines sur les positions correspondant aux positions d'inhibiteur de trypsine pancréatique bovine (BPTI) 5, 30, 51 et 55, la glycine sur une position correspondant à la position BPTI 12, l'Asn sur une position correspondant au BPTI 43 et la Phe sur une position correspondant au BPTI 33, à condition que quand les positions correspondant aux BPTI 14 et 38 sont la cystéine, la position correspondant au BPTI 37 est la glycine ; la séquence principale étant les résidus correspondant aux positions BPTI 5 à 55 ;
où, dans ledit domaine Kunitz d'origine non naturelle, le résidu correspondant à la position BPTI 18 est la Phe, et
où les résidus correspondant aux positions BPTI 39 à 42 sont tous des acides aminés non chargés.

2. Protéine selon la revendication 1 qui comprend les résidus correspondant aux positions BPTI 1 à 58.

3. Protéine selon l'une quelconque des revendications 1 à 2, dans laquelle les résidus correspondant aux positions BPTI 14 et 38 sont la Cys, et le résidu correspondant à la position BPTI 37 est la Gly.

4. Protéine selon l'une quelconque des revendications 1 à 3, dans laquelle le résidu correspondant à la position BPTI 45 est la Phe et le résidu correspondant à la position BPTI 43 est l'Asn.

5. Protéine selon l'une quelconque des revendications 1 à 4, dans laquelle le résidu correspondant à la position BPTI 15 est la Val, Ile ou Leu.

6. Protéine selon la revendication 5, dans laquelle le résidu correspondant à la position BPTI 15 est la Val.

7. Protéine selon la revendication 5, dans laquelle le résidu correspondant à la position BPTI 15 est l'Ile.

8. Protéine selon la revendication 5, dans laquelle le résidu correspondant à la position BPTI 15 est la Leu.

9. Protéine selon l'une quelconque des revendications 1 à 8, dans laquelle le résidu correspondant à la position BPTI 16 est l'Ala ou Gly.

10. Protéine selon l'une quelconque des revendications 1 à 9, dans laquelle le résidu correspondant à la position BPTI 17 est la Met, Phe, Ile ou Leu.

11. Protéine selon l'une quelconque des revendications 1 à 10, dans laquelle le résidu correspondant à la position BPTI 19 est la Pro, Ser, Lys ou Gln.

12. Protéine selon l'une quelconque des revendications 1 à 11, dans laquelle le résidu correspondant à la position BPTI 16 est l'Ala.

13. Protéine selon l'une quelconque des revendications 1 à 12, dans laquelle le résidu correspondant à la position BPTI 17 est la Phe.

14. Protéine selon l'une quelconque des revendications 1 à 13, dans laquelle le résidu correspondant à la position BPTI 19 est la Pro.

15. Protéine selon l'une quelconque des revendications 1 à 14, dans laquelle le résidu correspondant à la position BPTI 39 est la Met.

16. Protéine selon l'une quelconque des revendications 1 à 15, dans laquelle le résidu correspondant à la position BPTI 40 est la Gly, Ala, Ser, Asn, Thr ou Pro.

17. Protéine selon l'une quelconque des revendications 1 à 16, dans laquelle le résidu correspondant à la position BPTI 40 est la Gly ou Ala.

18. Protéine selon l'une quelconque des revendications 1 à 17, dans laquelle le résidu correspondant à la position BPTI 40 est la Gly.

19. Protéine selon l'une quelconque des revendications 1 à 18, dans laquelle le résidu correspondant à la position BPTI 41 est l'Asn, Gln, Ser, Thr ou Ala.

20. Protéine selon l'une quelconque des revendications 1 à 19, dans laquelle le résidu correspondant à la position BPTI 41 est l'Asn.

21. Protéine selon l'une quelconque des revendications 1 à 20, dans laquelle le résidu correspondant à la position BPTI 42 est la Gly.

22. Protéine selon l'une quelconque des revendications 1 à 21, où le résidu correspondant à la position BPTI 40 est la Gly et le résidu correspondant à la position BPTI 42 est la Gly.

23. Protéine selon l'une quelconque des revendications 1 à 22, où les résidus correspondant aux positions BPTI 40, 41 et 42 sont la Gly, Asn et Gly, respectivement.

24. Protéine selon l'une quelconque des revendications 1 à 23, dans laquelle les résidus correspondant aux positions BPTI 39, 40, 41 et 42 sont la Met, Gly, Asn et Gly, respectivement.

25. Protéine selon l'une quelconque des revendications 1 à 24, dans laquelle le résidu correspondant à la position BPTI 31 est la Gln.

26. Protéine selon l'une quelconque des revendications 1 à 25, dans laquelle le résidu correspondant à la position BPTI 34 est la Pro.

27. Protéine selon l'une quelconque des revendications 1 à 26, dans laquelle le résidu correspondant à la position BPTI 34 est la Val.

28. Protéine selon l'une quelconque des revendications 1 à 27, dans laquelle le résidu correspondant à la position BPTI 1 est l'Arg.

29. Protéine selon l'une quelconque des revendications 1 à 28, dans laquelle le résidu correspondant à la position BPTI 2 est la Pro.

30. Protéine selon l'une quelconque des revendications 1 à 29, dans laquelle le résidu correspondant à la position BPTI 3 est l'Asp.

31. Protéine selon l'une quelconque des revendications 1 à 30, dans laquelle le résidu correspondant à la position BPTI 4 est la Phe.

32. Protéine selon l'une quelconque des revendications 1 à 31, où, pour chaque résidu correspondant à une position précédemment non spécifiée, le domaine Kunitz de référence est un domaine Kunitz de la chaîne légère d'un ITI humain.

33. Protéine selon l'une quelconque des revendications 1 à 31, où, pour chaque résidu correspondant à une position précédemment non spécifiée, le domaine Kunitz de référence est le BPTI
ou le premier domaine Kunitz de la chaîne légère d'un ITI humain (ITI-D1 humain).

34. Protéine selon l'une quelconque des revendications 1 à 33, dans laquelle ledit domaine a un pourcentage d'identité à un domaine Kunitz humain d'origine naturelle des Tableaux 13, 15 ou 62 supérieur à celui à un domaine Kunitz non humain d'origine naturelle quelconque des Tableaux 13, 15 ou 62.

35. Protéine selon l'une quelconque des revendications 1 à 34, dans laquelle la séquence principale d'acides aminés dudit domaine Kunitz d'origine non naturelle présente un pourcentage d'identité à celui d'un ITI-D1 humain supérieur à celle de BPTI.

36. Protéine selon l'une quelconque des revendications 1 à 35, dans laquelle la séquence principale dudit domaine diffère normalement de la séquence principale d'un domaine Kunitz de référence,
choisie dans le groupe constitué des EpiNeα, EpiNe1, EpiNe2, EpiNe3, EpiNe4, EpiNe5, EpiNe6, EpiNe7 et EpiNe8 des Tableaux 207-208,
uniquement par une ou plusieurs substitutions de classe A et/ou une ou plusieurs substitutions de classe B comme défini dans le Tableau 65.

37. Protéine selon l'une quelconque des revendications 1 à 35, dans laquelle la séquence principale dudit domaine diffère normalement de la séquence principale d'un domaine Kunitz de référence,
choisie dans le groupe constitué des EpiNeα, EpiNe1, EpiNe2, EpiNe3, EpiNe4, EpiNe5, EpiNe6, EpiNe7 et EpiNe8 des Tableaux 207-208,
uniquement par une ou plusieurs substitutions de classe A comme défini dans le Tableau 65.

38. Protéine selon la revendication 1 ou la revendication 2, dans laquelle la séquence principale dudit domaine consiste en une séquence d'acides aminés identiques à ceux de la séquence principale d'une protéine choisie dans le groupe constitué des EpiNeα, EpiNe1, EpiNe2, EpiNe3, EpiNe4, EpiNe5, EpiNe6, EpiNe7 et EpiNe8 des Tableaux 207-208.

39. Protéine selon la revendications 1 qui est choisie dans le groupe constitué des EpiNeα, EpiNe1, EpiNe2, EpiNe3, EpiNe4, EpiNe5, EpiNe6, EpiNe7 et EpiMe8 des Tableaux 207-208.

40. Protéine selon la revendication 1 ou la revendication 2, dans laquelle la séquence principale dudit domaine consiste en une séquence d'acides aminés identiques à ceux de la séquence principale d'une protéine choisie dans le groupe constitué des ITI-E7, BITI-E7, BITI-E7-1222, AMINO1, AMINO2, MUTP1, BITI-E7-141, MUTT26A, MUTQE et MUT1619 du Tableau 220.

41. Protéine selon la revendication 1 qui est choisie dans le groupe constitué des ITI-E7, BITI-E7, BITI-E7-1222, AMINO1, AMINO2, MUTP1, BITI-E7-141, MUTT26A, MUTQE et MUT1619 du Tableau 220.

42. Protéine selon l'une quelconque des revendications 1 à 41 qui comprend une séquence d'acides aminés différant normalement de la séquence principale d'un inhibiteur de référence uniquement par une ou plusieurs substitutions de classe A conformément au Tableau 65, l'inhibiteur de référence étant choisi dans le groupe constitué des ITI-E7, BITI-E7, BITI-E7-1222, AMINO1, AMINO2, MUTP1, BITI-E7-141, MUTT26A, MUTQE et MUT1619 dans le Tableau 220.

43. Protéine selon l'une quelconque des revendications 1 à 41 qui comprend une séquence d'acides aminés différant normalement de la séquence principale d'un inhibiteur de référence uniquement par une ou plusieurs substitutions de classe A et/ou B conformément au Tableau 65, l'inhibiteur de référence étant choisi dans le groupe constitué des ITI-E7, BITI-E7, BITI-E7-1222, AMINO1, AMINO2, MUTP1, BITI-E7-141, MUTT26A, MUTQE et MUT1619 dans le Tableau 220.

44. Protéine selon la revendication 1, où (a) le résidu correspondant à la position BPTI 15 est la Val ou Ile et/ou (b) le résidu correspondant à la position BPTI 17 est la Phe.

45. Protéine selon l'une quelconque des revendications 1 à 44, où ledit domaine Kunitz n'est pas identique par la séquence d'acides aminés à l'une quelconque des séquences d'acides aminés répertoriées dans le Tableau 13.

46. Protéine selon l'une quelconque des revendications 1 à 38, 40 ou 42 à 45 qui comprend au moins deux desdits domaines Kunitz mutants.

47. Protéine selon l'une quelconque des revendications 1 à 46 qui possède une affinité de liaison puissante (10⁻⁹ > Ko >10⁻¹¹M) ou très puissante (Ko < 10⁻¹²M) vers l'élastase neutrophile humaine.

48. Protéine selon l'une quelconque des revendications 1 à 47 qui possède une affinité de liaison très puissante (Ko < 10⁻¹¹M) vers l'élastase neutrophile humaine.

49. Protéine selon l'une quelconque des revendications 1 à 48 qui est une protéine phagique chimérique.

50. Protéine selon l'une quelconque des revendications 1 à 49 sous forme pégylée.

51. Protéine selon l'une quelconque des revendications 1 à 50 sous forme purifiée.

52. Protéine suivant l'une quelconque des revendications 1 à 50 à utiliser pour la liaison de l'élastase neutrophile humaine.

53. Protéine suivant l'une quelconque des revendications 1 à 50 à utiliser pour l'inhibition de l'activité de l'élastase neutrophile humaine.

54. Protéine suivant l'une quelconque des revendications 1 à 50 à utiliser pour l'inhibition de l'activité nocive de l'élastase neutrophile humaine.

55. Protéine suivant l'une quelconque des revendications 1 à 50 à utiliser pour l'inhibition de l'activité excessive de l'élastase neutrophile humaine.

56. Utilisation d'une quantité thérapeutiquement efficace de la protéine selon l'une quelconque des revendications 1 à 50 dans la préparation d'une composition pharmaceutique à utiliser pour l'inhibition de l'activité nocive de l'élastase neutrophile humaine.

57. Utilisation d'une quantité inhibitrice efficace de la protéine selon l'une quelconque des revendications 1 à 50 dans la préparation d'une composition pharmaceutique à utiliser pour l'inhibition de l'activité excessive de l'élastase neutrophile humaine.
